(19) Europäisches Patentamt
European Patent Office
Office européen des brevets

(11) Publication number: 0 207 609 B1

(12) EUROPEAN PATENT SPECIFICATION

(45) Date of publication of patent specification: 08.01.92

(51) Int. Cl.5: C07D 409/12, C07D 491/048, C07D 491/052, C07D 333/34, C07D 333/38, C07D 333/42, C07D 333/36, C07F 9/38, A01N 47/36, A01N 57/08, A01N 47/34, //(C07D491/048, 307:00,239:00),(C07D491/052, 311:00,239:00)

(21) Application number: 86303788.3

(22) Date of filing: 19.05.86

The file contains technical information submitted after the application was filed and not included in this specification

# (54) Herbicidal thiophenesulfonamides.

(30) Priority: 29.05.85 US 739074
21.04.86 US 852739
20.05.85 US 735529
10.04.86 US 849332

(43) Date of publication of application:
07.01.87 Bulletin 87/02

(45) Publication of the grant of the patent:
08.01.92 Bulletin 92/02

(84) Designated Contracting States:
BE CH DE FR GB IT LI LU NL SE

(56) References cited:
EP-A- 0 030 140
EP-A- 0 041 404
EP-A- 0 057 546
EP-A- 0 073 562
EP-A- 0 097 122
EP-A- 0 108 708
EP-A- 0 164 269
EP-A- 0 171 286
EP-A- 0 208 708
DE-A- 2 534 689

(73) Proprietor: E.I. DU PONT DE NEMOURS AND COMPANY
1007 Market Street
Wilmington Delaware 19898(US)

(72) Inventor: Levitt, George
3218 Romilly Road
Wilmington Delaware 19810(US)

(74) Representative: Hildyard, Edward Martin et al
Frank B. Dehn & Co. Imperial House 15-19 Kingsway
London WC2B 6UZ(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 207 609 B1

GB-A- 2 007 227
US-A- 4 293 330
US-A- 4 425 155

BEILSTEINS: "Handbuch der Organischen Chemie", vol. 18, part 7, 4th edition, 3rd, 4th supplement, pages 6704-6718,Springer-Verlag Berlin, 1976; Chapter VI A "Monosulfonsäuren"

CHEMICAL ABSTRACTS, vol. 70, no. 25, 23rd June 1969, page 317, right-hand column, abstract no. 114909d, Columbus, Ohio, US; A. CHRZASZCZEWSKA et al.: "Thiophenesulfonamides and their derivatives. IV. Reactions of 3,5-thiophenedisulfonamides"

CHEMICAL ABSTRACTS, vol. 55, no. 6, 20th March 1961, columns 5455a-5456c, Columbus, Ohio, US; A. BUZAS et al.: "Chlorosulfonation of thiophenes"

CHEMICAL ABSTRACTS, vol. 95, no. 7, 17th August 1981, page 17, right-hand column, abstract no. 54622j, Columbus, Ohio, US; I.T. BARNISH et al.: "Cerebrovasodilatation through selective inhibition of the enzyme carbonic anhydrase. 3. 5-(Arylthio)-, 5-(arylsulfinyl)-, and 5-(arylsulfonyl)thiophene-2-sulfonamides", & J. MED. CHEM. 1981, 24(8), 959-964 in connection with CHEMICAL SUBSTANCE INDEX, vol. 95, July-December 1981, page 6969CS, column 2, lines 93, 120 "5-bromo-3-chloro-2-thiophenesulfonamide"; column 2, line 98; column 3, line 59 "3,5-dibromo-2-thiophenesulfonamide"

## Description

Background of the Invention

U.S. 4,127,405 and U.S. 4,169,719 disclose herbicidal thiophenesulfonamides, wherein the thiophene ring may be optionally substituted with $CH_3$, Cl or Br.

U.S. 4,398,939 discloses herbicidal thiophenesulfonamides, wherein the thiophene ring is substituted with substituent groups selected from $C_1$-$C_4$ alkyl, $C_3$ alkenyl, $OCH_3$, $NO_2$, Cl, Br, $SO_2N(C_1$-$C_3$ alkyl$)_2$ or $SO_2N(OCH_3)CH_3$.

U.S. 4,481,029 discloses herbicidal thiophenesulfonamides, wherein the thiophene ring is substituted with carboxylic acid, carboxylic ester and alkylcarbonyl groups or derivatives thereof.

U.S. 4,441,910 discloses herbicidal thiophenesulfonamides, wherein the thiophene ring is substituted with the group represented by $R_6S(O)_n$ wherein $R_6$ is $C_1$-$C_4$ alkyl, $C_3$-$C_4$ alkenyl, cyclopentyl or cyclopropylmethyl.

U.S. 4,518,776 (Swiss priority 7/19/82) discloses, in part, a process for the preparation of compounds of formula

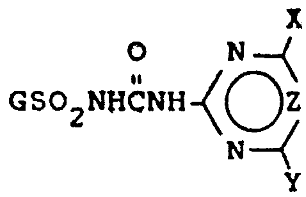

wherein

G is 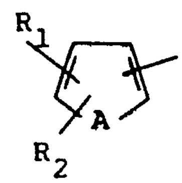 or 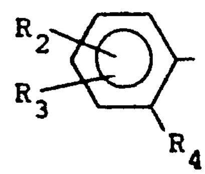 ;

| | |
|---|---|
| $R_1$ | is H, $C_1$-$C_4$ alkyl, halogen, $NO_2$, CN, $NH_2$, $S(O)_nC_1$-$C_4$ alkyl, $SO_2C_1$-$C_4$ alkoxy, $SO_2$-di-$C_1$-$C_4$ alkylamino, CHO, $CONH_2$, $DC_3$-$C_5$ alkynyl, $CODC_3$-$C_5$ alkynyl, $DC_1$-$C_4$ alkyl, $DC_3$-$C_5$ alkenyl, $COC_1$-$C_4$ alkyl, $CODC_1$-$C_4$ alkyl or $CODC_3$-$C_5$ alkenyl; |
| n | is 1 or 2; |
| D | is O, S, NH or $NC_1$-$C_4$ alkyl; |
| $R_2$ | is H, halogen, $CF_3$, $NO_2$, $C_1$-$C_4$ alkyl or $C_1$-$C_4$ alkoxy; |
| A | is O, S, $NR_5$ or -C=N-; |
| X | is $C_1$-$C_4$ alkyl, $C_1$-$C_4$ haloalkyl, $C_1$-$C_4$ alkoxy, $C_1$-$C_4$ alkylthio, $C_1$-$C_4$ haloalkoxy, $C_1$-$C_4$ alkylamino or di-$C_1$-$C_4$ alkylamino; |
| Y | is $C_1$-$C_4$ alkyl, $C_1$-$C_4$ alkoxy or $C_1$-$C_4$ haloalkoxy; and |
| Z | is CH or N. |

EP-A-101,670 (Swiss priority 8/23/82) discloses, in part, a process for the preparation of compounds of formula

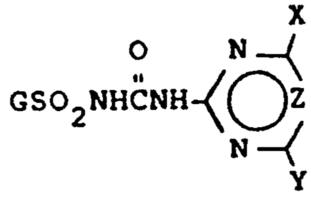

wherein

G is 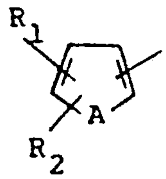 or 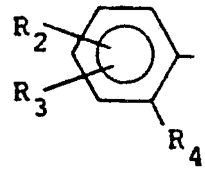 ;

$R_1$ is H, halogen, $NO_2$, $QC_1$-$C_4$ alkyl, $C_1$-$C_4$ alkyl, $CF_3$, $SO_2$-di-$C_1$-$C_4$ alkylamino, $COQC_3$-$C_4$ alkynyl, $COQC_1$-$C_4$ alkyl or $COQC_3$-$C_5$ alkenyl optionally substituted by halogen, CN, $C_1$-$C_4$ alkoxy or $C_1$-$C_4$ alkylthio;
Q is O, $S(O)_n$, NH or N($C_1$-$C_4$ alkyl);
n is 0, 1 or 2;
$R_2$ is H, halogen, $CF_3$, $NO_2$, $C_1$-$C_4$ alkyl or $C_1$-$C_4$ alkoxy;
A is O, S, $NR_5$ or -C=N-;
X is $C_1$-$C_4$ alkyl, $C_1$-$C_4$ haloalkyl, $C_1$-$C_4$ alkoxy, $C_1$-$C_4$ alkylthio, $C_1$-$C_4$ haloalkoxy, $C_1$-$C_4$ alkylamino or di-$C_1$-$C_4$ alkylamino;
Y is $C_1$-$C_4$ alkyl, $C_1$-$C_4$ alkoxy or $C_1$-$C_4$ haloalkoxy; and
Z is CH or N.

U.S. 4,521,597 discloses, in part, a process for the preparation of compounds of formula

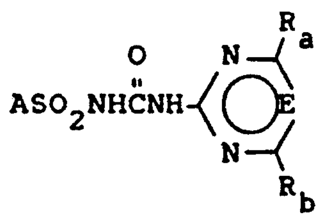

wherein

A is 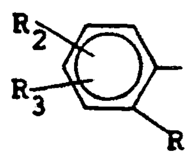 , 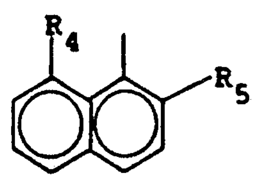

or

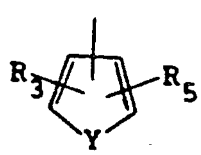 ;

$R_3$ is H, halogen, $NO_2$, $OCH_3$ or $CF_3$;
$R_5$ is H, F, Cl, Br, $NO_2$, $C_1$-$C_5$ alkyl, $C_1$-$C_5$ alkoxy, $CF_3$, $S(O)_mC_1$-$C_5$ alkyl, $COR_7$ or $SO_2NR_8R_9$;
Y is O, S or $C(R_6)=N$;
$R_a$ is H, halogen, $C_1$-$C_5$ alkyl, $C_1$-$C_5$ haloalkyl, $C_1$-$C_5$ alkoxy, $C_1$-$C_5$ haloalkoxy, $C_1$-$C_5$ alkylthio, $C_2$-$C_{10}$ alkoxyalkyl or $C_2$-$C_{10}$ alkoxyalkoxy;
$R_b$ is the same as $R_a$ or $NR_cR_d$; and
E is CH or N.

U.S. 4,549,898 discloses herbicidal sulfonylureas of formula

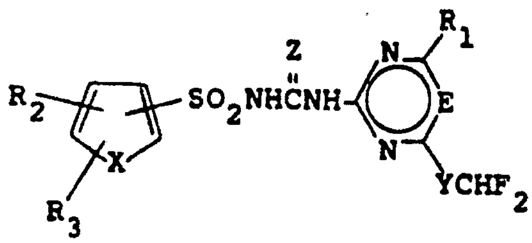

wherein

X is O, S, $NR_4$ or $C(R_5)=N$;
Y is O or S;
Z is O or S;
E is N or CH;
$R_1$ is H, $C_1$-$C_4$ alkyl, $C_1$-$C_4$ haloalkyl, $C_1$-$C_4$ haloalkoxy, $C_1$-$C_4$ alkoxy, halogen, $C_1$-$C_4$ alkylthio, $NR_6R_7$ or alkoxyalkyl containing not more than 4 carbon atoms;
$R_2$ is H, $C_1$-$C_3$ alkyl, $C_1$-$C_3$ haloalkyl, halogen, $NO_2$, $C_1$-$C_3$ alkoxy, $C(W)R_8$, $SO_2NR_6R_7$, $S(O)_n$-$C_1$-$C_3$ alkyl or $COR_9$;
$R_3$ is H, halogen, $C_1$-$C_3$ alkyl, $OCH_3$ or $CF_3$;
$R_5$ is H, $NO_2$, F, Cl, Br, $CH_3$, $CF_3$, $S(O)_nC_1$-$C_3$ alkyl, $COC_1$-$C_4$ alkoxy or $C_1$-$C_3$ alkoxy;
$R_6$ is H, $C_1$-$C_6$ alkyl, $C_1$-$C_4$ cyanoalkyl, methoxy or ethoxy; and
$R_7$ is H, $C_1$-$C_6$ alkyl or $C_3$-$C_6$ alkenyl.

U.S. 4,127,405 discloses herbicidal benzene- and thiophenesulfonamides, wherein the substituents on thiophene are selected from H, $CH_3$, Cl or Br.

U.S. 4,398,939 discloses herbicidal thiophenesulfonamides, wherein the substituents on thiophene are selected from $C_1$-$C_4$ alkyl, $C_3$ alkenyl, $OCH_3$, $NO_2$, Cl, Br, $SO_2NR_1R_2$ or $SO_2N(OCH_3)CH_3$.

U.S. 4,441,910 discloses herbicidal thiophenesulfonamides substituted with $R_6S(O)_n$ wherein $R_6$ is $C_1$-$C_4$ alkyl, $C_3$-$C_4$ alkenyl, cyclopentyl or cyclopropylmethyl; and n is O, 1 or 2.

U.S. 4,481,029 discloses herbicidal thiophenesulfonamides, wherein the substituents on thiophene are selected from various carboxylic acids, esters, amides and alkylcarbonyl groups and derivatives thereof.

South African Patent Application 83/7434 (Swiss priority 10/6/82) discloses herbicidal sulfonamides of formula

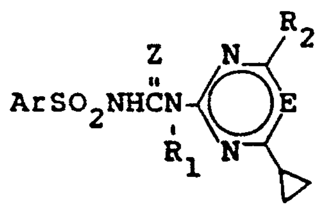

wherein Ar is

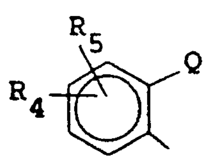

or

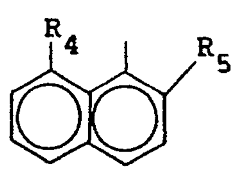

.

Other Specifications disclosing herbicidal sulfonylureas include EP-A-30,140; EP-A-41,404; EP-A-57,546; EP-A-97,122; EP-A-164,269 and EP-A-171,286.

## Summary of the Invention

This application pertains to novel compounds of Formula I, agriculturally suitable compositions containing them and their method-of-use as preemergent and/or post- emergent herbicides or plant growth

regulants.

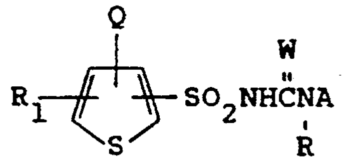

I

wherein

| | |
|---|---|
| R | is H or $CH_3$; |
| $R_1$ | is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, CN, $CO_2R_c$, $C_1$-$C_3$ haloalkoxy on $C_1$-$C_3$ haloalkylthio; |
| $R_a$ | is H, $C_1$-$C_4$ alkyl, $C_2$-$C_3$ cyanoalkyl, methoxy, ethoxy or $C_3$-$C_4$ alkenyl; |
| $R_b$ | is H or $C_1$-$C_3$ alkyl; or |
| $R_a$ | and $R_b$ may be taken together to form $(CH_2)_3$, $(CH_2)_4$, $(CH_2)_5$ or $CH_2CH_2OCH_2CH_2$; |
| $R_c$ | is $C_1$-$C_4$ alkyl, $C_3$-$C_4$ alkenyl, $C_3$-$C_4$ alkynyl, $C_2$-$C_4$ haloalkyl, $C_2$-$C_3$ cyanoalkyl, cyclopropylmethyl or $C_2$-$C_4$ alkoxyalkyl; |
| Q | is $ER_2$, $NR_3R_4$, 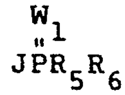 $OSO_2R_7$, $C_1$-$C_4$ haloalkyl, CN, $SO_2NHR_{21}$, 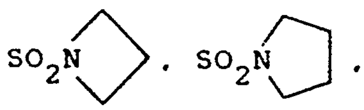 $SO_2NR_{22}NR_{23}R_{24}$ or $C_1$-$C_4$ alkyl substituted with $R_8$; |
| E | is O, S, SO or $SO_2$; |
| W and $W_1$ | are independently O or S; |
| J | is O, S, NH, $NCH_3$, $CH_2$ or a single bond; |
| $R_2$ | is $C_1$-$C_6$ alkyl substituted with $R_8$, $C_2$-$C_6$ alkenyl substituted with $R_8$, $C_3$-$C_6$ alkynyl, $C_3$-$C_6$ alkynyl substituted with $R_8$, $C_1$-$C_6$ haloalkyl, $C_2$-$C_6$ haloalkenyl or $C_3$-$C_6$ haloalkynyl; |
| $R_3$ | is $C_1$-$C_4$ alkyl; |
| $R_4$ | is H or $C_1$-$C_4$ alkyl; or |
| $R_3$ and $R_4$ | may be taken together to form $(CH_2)_4$, $(CH_2)_5$ or $CH_2CH_2OCH_2CH_2$; |
| $R_5$ and $R_6$ | are independently $C_1$-$C_2$ alkyl, $C_1$-$C_2$ alkoxy, $C_1$-$C_2$ alkylthio, $C_1$-$C_2$ alkylamino or di($C_1$-$C_2$ alkyl)amino; |
| $R_7$ | is $C_1$-$C_4$ alkyl, $C_1$-$C_4$ haloalkyl, $C_3$-$C_4$ alkenyl, $C_3$-$C_4$ haloalkenyl, $C_3$-$C_4$ alkynyl, $C_3$-$C_4$ haloalkynyl or $NR_{19}R_{20}$; |
| $R_8$ | is $OR_9$, $S(O)_nR_{10}$, $CO_2R_{10}$, $SO_2NR_{11}R_{12}$, $NR_{11}R_{12}$, $CONR_{11}R_{12}$, $C(O)R_{13}$, 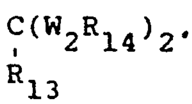, 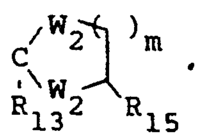, |

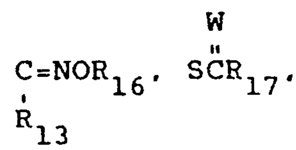

| | |
|---|---|
| | CN, SCN, SH, $NO_2$ or $N_3$; |
| $R_9$ | is H, $C_1$-$C_4$ alkyl, $C_3$-$C_4$ alkenyl, $C_3$-$C_4$ alkynyl, $C_1$-$C_4$ haloalkyl, $C_3$-$C_4$ haloalkenyl, $C_3$-$C_4$ haloalkynyl, $C_2$-$C_4$ alkylcarbonyl, $C_1$-$C_4$ alkylsulfonyl, $C_2$-$C_4$ alkoxyalkyl, $C_2$-$C_4$ alkylthioalkyl or $C_2$-$C_4$ cyanoalkyl; |
| $R_{10}$ | is H, $C_1$-$C_4$ alkyl, $C_3$-$C_4$ alkenyl, $C_3$-$C_4$ alkynyl, $C_1$-$C_4$ haloalkyl, $C_3$-$C_4$ haloalkenyl, $C_3$-$C_4$ haloalkynyl, $C_2$-$C_4$ alkoxyalkyl, $C_2$-$C_4$ alkylthioalkyl or $C_2$-$C_4$ cyanoalkyl; |
| $R_{11}$ | is H or $C_1$-$C_3$ alkyl; |
| $R_{12}$ | is H, $C_1$-$C_4$ alkyl, $C_1$-$C_4$ haloalkyl, $C_3$-$C_6$ cycloalkyl, $C_2$-$C_4$ alkoxyalkyl, $C_2$-$C_4$ alkylthioalkyl, $C_2$-$C_4$ cyanoalkyl, $C_1$-$C_3$ alkoxy, $C_3$-$C_4$ alkenyl or $C_3$-$C_4$ alkynyl; or |
| $R_{11}$ and $R_{12}$ | may be taken together to form $(CH_2)_3$, $(CH_2)_4$, $(CH_2)_5$ or $CH_2CH_2OCH_2CH_2$; |
| $R_{13}$ | is H, $C_1$-$C_4$ alkyl or $C_1$-$C_4$ haloalkyl; |
| $R_{14}$ | is $C_1$-$C_2$ alkyl; |
| $R_{15}$ | is H or $CH_3$; |
| $R_{16}$ | is H, $C_1$-$C_4$ alkyl, $C_3$-$C_4$ alkenyl or $C_3$-$C_4$ alkynyl; |
| $R_{17}$ | is $C_1$-$C_4$ alkyl, $C_1$-$C_4$ alkoxy, $C_1$-$C_4$ alkylthio, $C_1$-$C_4$ alkyl amino or di($C_1$-$C_4$ alkyl)amino; |
| $R_{18}$ | is F, Cl, Br, CN, $CH_3$, $OCH_3$, $SCH_3$ or $NO_2$; |
| $R_{19}$ | is H, $C_1$-$C_3$ alkyl, $C_3$-$C_4$ alkenyl or $C_3$-$C_4$ alkynyl; or |
| $R_{20}$ | is H or $C_1$-$C_3$ alkyl; or |
| $R_{19}$ and $R_{20}$ | may be taken together to form $(CH_2)_4$, $(CH_2)_5$ or $CH_2CH_2OCH_2CH_2$; |
| $R_{21}$ | is $C_1$-$C_4$ alkyl, $C_1$-$C_3$ haloalkyl, $C_2$-$C_3$ cyanoalkyl, cyclopropyl, $C_3$-$C_4$ alkenyl, $C_3$-$C_4$ alkynyl, $C_2$-$C_4$ alkoxyalkyl or $C_1$-$C_2$ alkoxy; |
| $R_{22}$ | is H or $C_1$-$C_4$ alkyl; |
| $R_{23}$ | is H or $C_1$-$C_4$ alkyl; |
| $R_{24}$ | is H, $C_1$-$C_4$ alkyl, $C_3$-$C_4$ alkenyl, $C_3$-$C_4$ alkynyl, $C_1$-$C_4$ haloalkyl or phenyl which may be optionally substituted with $R_{25}$; or |
| $R_{23}$ and $R_{24}$ | may be taken together to form $(CH_2)_4$, $(CH_2)_5$ or $CH_2CH_2OCH_2CH_2$; |
| $R_{25}$ | is H, $CH_3$, Cl, F, Br, $NO_2$, $CF_3$, CN or $OCH_3$; |
| m | is 1 or 2; |
| n | is 0, 1 or 2; |
| $W_2$ and $W_3$ | are independently O or S; |

A is 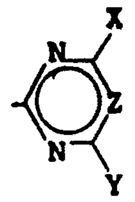 , 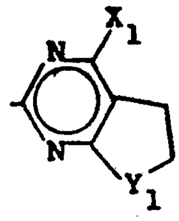 , 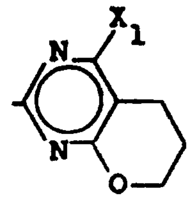 ,

A-1 A-2 A-3

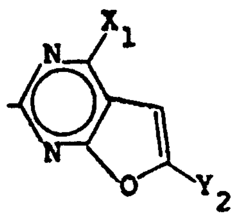 , 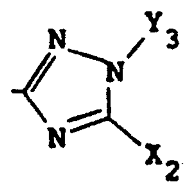 , 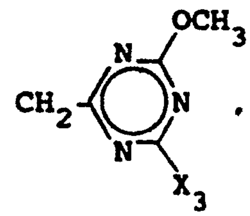 ,

A-4 A-5 A-6

or 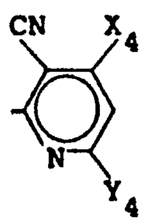 ;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino or di($C_1$-$C_3$ alkyl)amino;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_2$-$C_5$ alkylsulfinylalkyl, $C_2$-$C_5$ alkylsulfonylalkyl, $C_1$-$C_4$ haloalkyl, $C_4$-$C_5$ cycloalkyl, $C_2$-$C_4$ alkynyl, cyano,

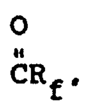 , 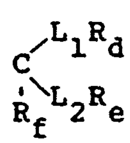 , 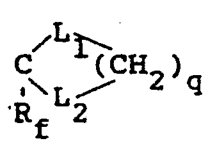 ,

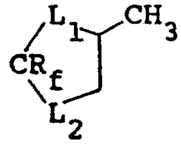

or $N(OCH_3)CH_3$;

q is 2 or 3;

$L_1$ and $L_2$ are independently O or S;

$R_d$ and $R_e$ are independently $C_1$-$C_2$ alkyl;

$R_f$ is H or $CH_3$;
Z is CH or N;
$Y_1$ is O or $CH_2$; $X_1$ is $CH_3$, $OCH_3$, $OC_2H_5$ or $OCF_2H$;
$Y_2$ is H or $CH_3$;
$X_2$ is $CH_3$, $OCH_3$ or $SCH_3$;
$Y_3$ is $CH_3$, $CH_2CH_3$ or $CH_2CF_3$;
$X_3$ is $CH_3$ or $OCH_3$;
$X_4$ is $CH_3$, $OCH_3$, $OC_2H_5$, $CH_2OCH_3$ or Cl: and
$Y_4$ is $CH_3$, $OCH_3$, $OC_2H_5$ or Cl:

and their agriculturally suitable salts: provided that

a) when X is Cl, F, Br or I, then Z is CH and Y is $OCH_3$, $OC_2H_5$, $N(OCH_3)CH_3$, $NHCH_3$, $N(CH_3)_2$ or $OCF_2H$;

b) when X or Y is $C_1$ haloalkoxy, then Z is CH;

c) when Q is $CF_3$, then A is A-4, A-5, A-6 or A-7;

d) when E is O or S and $R_9$ is H, then $R_2$ is other than $CH_2O_9$;

e) when W is S, then A is A-1, R is H and Y is $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$

or 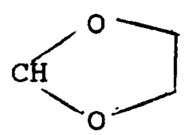 ;

f) the total number of carbon atoms in $R_1$ and Q is less than or equal to 10;

g) when Q is $C_1$-$C_4$ haloalkyl or

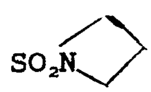 ,

then X and Y are other than $OCF_2H$ or $SCF_2H$;

h) $X_4$ and $Y_4$ cannot simultaneously be Cl;

i) the substituent Q and the sulfonylurea bridge are on adjacent carbon atoms;

j) the total number of carbon atoms in $R_{22}$, $R_{23}$ and $R_{24}$ is less than or equal to 10;

k) when $R_{21}$ is $C_1$-$C_4$ alkyl, $C_2$-$C_3$ cyanoalkyl, $C_1$-$C_2$ alkoxy or $C_3$-$C_4$ alkenyl, then X and Y are other than $OCF_2H$ or $SCF_2H$;

l) when Y is CN and $R_1$ is H, F, Cl or $CH_3$ then $R_{21}$ is other than $C_1$-$C_3$ alkyl;

m) when $R_{21}$ is $C_1$-$C_4$ alkyl, then Y is other than $C_1$-$C_3$ alkylamino, di($C_1$-$C_3$ alkyl)amino, $N(OCH_3)CH_3$ or $C_2$-$C_4$ alkynyl and X is other than halogen, $C_1$-$C_3$ alkylamino or di($C_1$-$C_3$ alkyl)amino.

In the above definitions, the term "alkyl", used either alone or in compound words such as "alkylthio" or "haloalkyl", denotes straight chain or branched alkyl, e.g. methyl, ethyl, n-propyl, isopropyl or the different butyl, pentyl or hexyl isomers.

Alkoxy denotes methoxy, ethoxy, n-propyloxy, isopropyloxy and the different butyl isomers.

Alkenyl denotes straight chain or branched alkenes, e.g. vinyl, 1-propenyl, 2-propenyl, 3-propenyl, isopropenyl and the different butenyl, pentenyl or hexenyl isomers.

Alkynyl denotes straight chain or branched alkynes, e.g. ethynyl, 1-propynyl, 2-propynyl and the different butynyl, pentynyl or hexynyl isomers.

Alkylcarbonyl denotes. e.g., acetyl, propionyl and the different butyryl isomers.

Alkylsulfonyl denotes methylsulfonyl, ethylsulfonyl or the different propylsulfonyl and butylsulfonyl isomers.

Cycloalkyl denotes cyclopropyl, cyclobutyl, cyclopentyl and cyclohexyl.

The term "halogen", either alone or in compound words such as "haloalkyl", denotes fluorine, chlorine, bromine or iodine. Further, when used in compound words such as "haloalkyl" said alkyl may be partially halogenated or fully substituted with halogen atoms which may be the same or different. Examples of haloalkyl include $CH_2CH_2F$, $CF_2CF_3$ and $CH_2CHFCl$.

The total number of carbon atoms in a substituent group is indicated by the $C_i$-$C_j$ prefix where i and j

are numbers from 1 to 6. For example, $C_1$-$C_3$ alkylsulfonylwould designate methylsulfonyl through propylsulfonyl, $C_2$ alkoxyalkoxy would designate $OCH_2OCH_3$, $C_2$ cyanoalkyl would designate $CH_2CN$ and $C_3$ cyanoalkyl would designate $CH_2CH_2CN$ and $CH(CN)CH_3$.

Groups of compounds within the scope of our invention include those disclosed in our copending U.S. Patent Applications Serial Nos. 735,529; 739,074; 849,332 and 852,739, Copies of which are available for inspection on the file of the present Application.

Preferred for reasons of their higher herbicidal activity, greater plant growth regulant activity or more favorable ease of synthesis are:

1. Compounds of Formula I wherein

R is H;
W is O;
$R_1$ is H, $CH_3$ or Cl;
X is $CH_3$, $OCH_3$, $OCH_2CH_3$, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
Y is H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C{\equiv}CH$, $CH_2OCH_2CH_3$, $OCH_2CH_2OCH_3$, $CH_2SCH_3$, $CH_2SCH_2CH_3$,

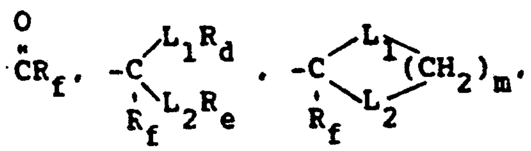

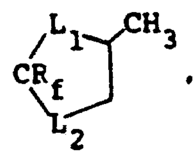

$OCF_2H$, $SCF_2H$, $C{\equiv}CH$ or $C{\equiv}CCH_3$;

2. Compounds of Preferred 1 where A is A-1.
3. Compounds of Preferred 2 where the sulfonylurea bridge is attached to the 2-position of the thiophene ring.
4. Compounds of Preferred 3 where

Q is $ER_2$,

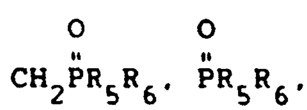

$C_1$-$C_2$ alkyl substituted with $R_8$, $C_1$-$C_2$ haloalkyl, CN, $SO_2NHC_1$-$C_2$ alkyl, $SO_2NH$cyclopropyl or

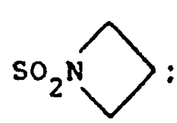

$R_2$ is $C_1$-$C_3$ alkyl substituted with $R_8$, $C_1$-$C_3$ haloalkyl, $C_2$-$C_3$ haloalkenyl or $C_3$-$C_4$ alkynyl;
$R_5$ and $R_6$ are independently $CH_3$, $OCH_3$ or $SCH_3$;
$R_8$ is $OR_9$, $C_1$-$C_2$ alkylthio, $C_1$-$C_2$ alkylsulfinyl, $C_1$-$C_2$ alkylsulfonyl, $CO_2(C_1$-$C_2$ alkyl), $SO_2N(C_1$-$C_2$ alkyl$)_2$, $C(O)CH_3$, CN or $C(O)N(CH_3)_2$;
$R_9$ is H, $C_1$-$C_2$ alkyl or $C_2$-$C_3$ cyanoalkyl.

5. Compounds of Preferred 4 where

X is $CH_3$, $OCH_3$, $OCH_2CH_3$, Cl, $OCF_2H$ or $OCH_2CF_3$;
Y is $CH_3$, $OCH_3$, $C_2H_5$, $CH_2OCH_3$, $NHCH_3$ or $CH(OCH_3)_2$;
Q is $ER_2$, $C_1$-$C_2$ alkyl substituted with $R_8$ or $C_1$-$C_2$ haloalkyl, CN or $SO_2NHC_1$-$C_2$ alkyl;
E is S or $SO_2$;
$R_2$ is $C_1$-$C_2$ alkyl substituted with $R_8$ or $C_1$-$C_2$ haloalkyl;
$R_8$ is $CH_3O$, $CH_3CH_2O$, $CH_3S$, $CH_3SO_2$, $SO_2N(CH_3)_2$ or $CO_2CH_3$;

6. Compounds of Preferred 2 where the sulfonylurea bridge is attached to the 3-position of the thiophene ring.

7. Compounds of Preferred 6 where
Q is $ER_2$,

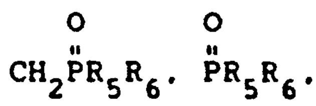

$C_1$-$C_2$ alkyl substituted with $R_8$, $C_1$-$C_2$ haloalkyl, CN, $SO_2NHC_1$-$C_2$ alkyl, $SO_2NH$cyclopropyl or

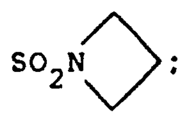

$R_2$ is $C_1$-$C_3$ alkyl substituted with $R_8$, $C_1$-$C_3$ haloalkyl, $C_2$-$C_3$ haloalkenyl or $C_3$-$C_4$ alkynyl:
$R_5$ and $R_6$ are independently $CH_3$, $OCH_3$ or $SCH_3$;
$R_8$ is $OR_9$, $C_1$-$C_2$ alkylthio, $C_1$-$C_2$ alkylsulfinyl, $C_1$-$C_2$ alkylsulfonyl, $CO_2(C_1$-$C_2$ alkyl), $SO_2N(C_1$-$C_2$ alkyl$)_2$, $C(O)CH_3$, CN or $C(O)N(CH_3)_2$;
$R_9$ is H, $C_1$-$C_2$ alkyl or $C_2$-$C_3$ cyanoalkyl.

8. Compounds of Preferred 7 where
X is $CH_3$, $OCH_3$, $OCH_2CH_3$, Cl, $OCF_2H$ or $OCH_2CF_3$;
Y is $CH_3$, $OCH_3$, $C_2H_5$, $CH_2OCH_3$, $NHCH_3$ or $CH(OCH_3)_2$;
Q is $ER_2$, $C_1$-$C_2$ alkyl substituted with $R_8$ or $C_1$-$C_2$ haloalkyl, CN or $SO_2NHC_1$-$C_2$ alkyl;
E is S or $SO_2$;
$R_2$ is $C_1$-$C_2$ alkyl substituted with $R_8$ or $C_1$-$C_2$ haloalkyl;
$R_8$ is $CH_3O$, $CH_3CH_2O$, $CH_3S$, $CH_3SO_2$, $SO_2N(CH_3)_2$ or $CO_2CH_3$.

Specifically preferred for reasons of greatest ease of synthesis and/or greatest herbicidal efficacy are:

N-[(4,6-dimethoxypyrimidin-2-yl)aminocarbonyl]-2-(methoxymethyl)-3-thiophenesulfonamide. m.p. 171-173° C;

N-[(4-methoxy-6-methyl-1,3,5-triazin-2-yl)aminocarbonyl]-2-(methoxymethyl)-3-thiophenesulfonamide, m.p. 155-156° C;

N-[(4-methoxy-6-methylpyrimidin-2-yl)aminocarbonyl]-4-cyano-3-thiophenesulfonamide, m.p. 189-192° C; and

N-[(4,6-dimethoxypyrimidin-2-yl)aminocarbonyl]-3-(2-methoxyethyl)-2-thiophenesulfonamide, m.p. 173-176° C.

## Detailed Description of the Invention

### Synthesis

The compounds of Formula I can be prepared by one or more of the methods described below in Equations 1 through 4. Reagents and reaction conditions are given by way of illustration only.

Many of the compounds of Formula I can be prepared by reaction of a sulfonamide of Formula 2 with an appropriate methyl carbamate of Formula 3 in the presence of at least an equimolar amount of trimethylaluminum, according to Equation 1.

Equation 1

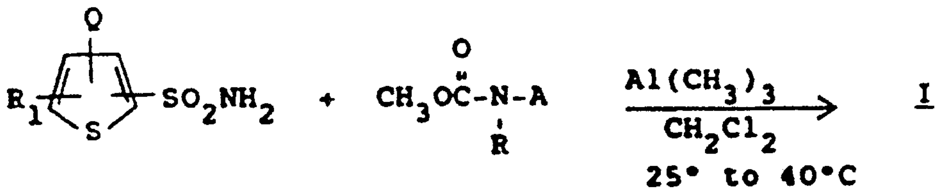


2 3

This reaction is carried out at 25° to 40° C under an inert atmosphere and in an inert, dipolar aprotic solvent such as methylene chloride for 10 to 96 hours. Details of this reaction as well as the preparation of the carbamates of Formula 3 can be found in EPO Publication No. 13,480.

Many of the compounds of Formula I also can be prepared by reacting a sulfonylcarbamate of Formula 4 with an appropriate heterocyclic amine of Formula 5. according to Equation 1a.

Equation 1a

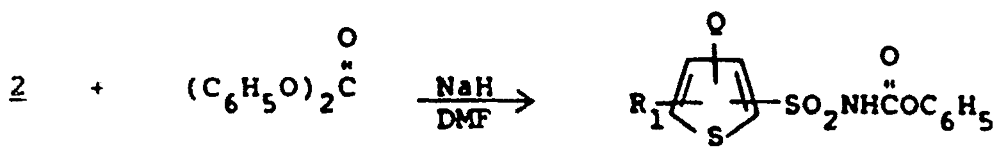


4

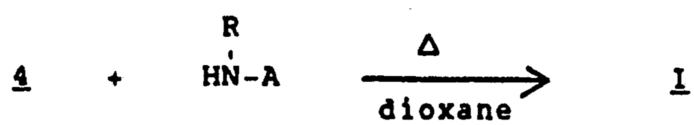


5

The reaction is carried out at 50° to 100° C in a solvent such as 1,4-dioxane for 0.5 to 24 hours according to EPO Publication No. 44807. The required carbamates of Formula 4 are prepared by reacting the appropriate sulfonamide, 2, with diphenylcarbonate in the presence of equimolar quantities of a strong base, such as sodium or potassium hydride.

Some of the compounds of Formula I also can be prepared as shown in Equation 2.

Equation 2

a)

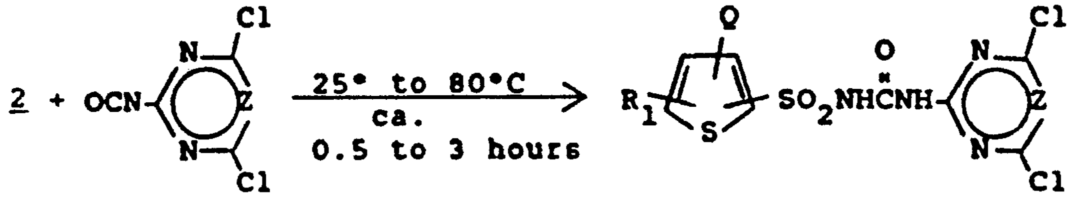


6 7

b)

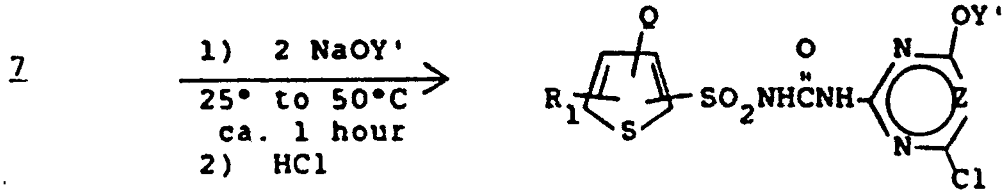


I'

c)

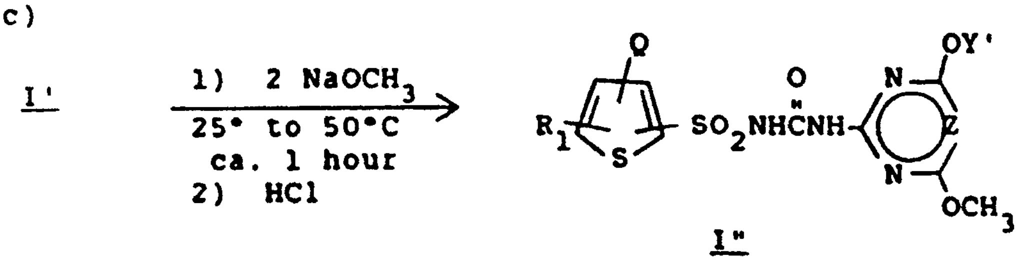


I"

wherein Y' is $CH_3$, $C_2H_5$ or $CH_2CF_3$. This reaction series is performed according to the procedures disclosed by EPO Publication No. 30,140 and the requisite heterocyclic isocyanates of Formula 6 can be prepared according to methods described in Swiss 579,062, U.S. 3,919,228, U.S. 3,732,223 and U. von Gizycki, Agnew. Chem. Int. Ed. Engl. 1976. 10. 402 and 403.

Compounds of Formula I also may be prepared by reaction of a thienylsulfonylisocyanate of Formula 8 with the appropriate heterocyclic amine, as shown in Equation 3.

Equation 3

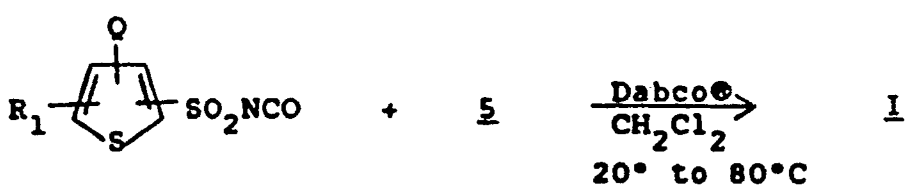


8

wherein Q, $R_1$ and A are as previously defined provided $R_a$, $R_b$, $R_4$, $R_9$, $R_{11}$, $R_{12}$, $R_{19}$ and $R_{20}$ are not H; J is not NH and $R_5$, $R_6$ and $R_7$ are not alkylamino.

The reaction of Equation 3 is most successful when performed in an inert dipolar aprotic solvent such as methylene chloride, tetrahydrofuran or acetonitrile at temperatures between 20° and 80°C. A catalytic amount of 1,4-diazabicyclo[2.2.2]octane (Dabco®) may be used to accelerate the reaction. In cases where the products are insoluble in the reaction solvent, isolation may be performed by simple filtration; when the

products are soluble, isolation may be performed by evaporation of the solvent, trituration with a solvent such as 1-chlorobutane, diethyl ether or methanol and filtration.

Thienylsulfonylisocyanates cf Formula 8 can be prepared from sulfonamides of Formula 2 by methods described in U.S. 4,238,621, as indicated in Equation 3a. Alternatively, these sulfonylisocyanates can be synthesized via a two-step procedure, consisting of (1) reacting sulfonamide 2 with n-butylisocyanate in the presence of one molar equivalent of a base such as potassium carbonate in a solvent such as 2-butanone or acetonitrile to form n-butylsulfonylureas of Formula 9. and (2) reaction of 9 with phosgene using Dabco® as a catalyst in refluxing xylene as solvent. This method is similar to the preparation found in "Newer Methods of Preparative Organic Chemistry." Forest, W., Ed., Vol. VI. Academic Press, NY, 1967, pp. 223-241. Equation 3b illustrates the procedure,

Equation 3a

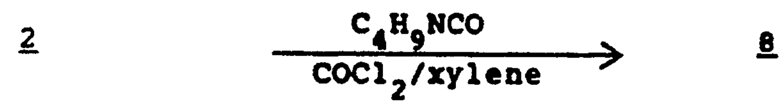


Equation 3b

(1)

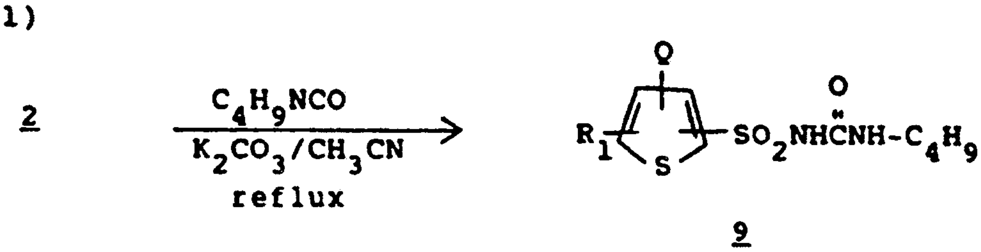


(2)

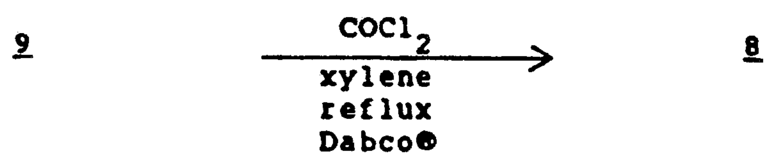


wherein the substituents are as defined for Equation 3 above.

The compounds of Formula I also are available by the methodology described in South African Application No. 830441 and illustrated by Equation 4. Thienylsulfonamides of Formula 2 react with heterocyclic carbamates of Formula 10 in 1,4-dioxane at 20° to 80° C for periods of 1 to 24 hours when 1 equivalent of 1.8-diazabicyclo[5.4.0]undec-7-ene (DBU) is added to the reaction mixture. The resultant products are isolated by dilution of the reaction mixture with water, acidification and subsequent filtration. Heterocyclic carbamates of Formula 10 in turn are synthesized by reaction of heterocyclic amines of Formula 5 with diphenyl carbonate or phenyl chloroformate in pyridine at temperatures ranging from 20° to 80° C, as indicated in Equation 4a.

Equation 4

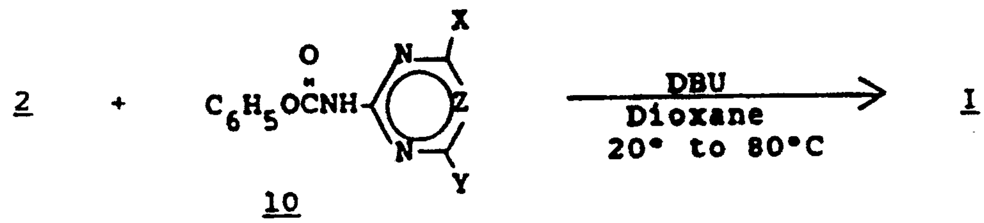


10

Equation 4a

$(C_6H_5O)_2\overset{\overset{O}{\|}}{C}$ + 5

pyridine
20° to 80°C

10

The synthesis of thienylsulfonylureas of Formula I relies upon the requisite intermediate thiophenesulfonamides of Formula 2.

Some of the intermediate sulfonamides of Formula 2 described above can be prepared from amines of Formula 11 by a two-step procedure, as shown in Equation 5. This consists of (5a) diazotizing 11 and coupling the diazonium salt with sulfur dioxide to form a sulfonyl chloride of Formula 12; and (5b) aminating 12 with ammonium hydroxide or anhydrous ammonia to form 2.

Equation 5

a)

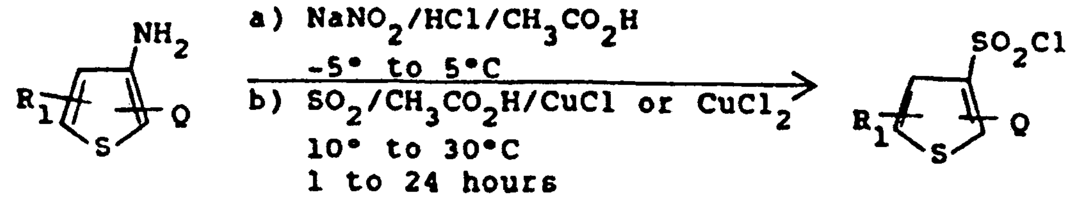


11 12

b)

12

$NH_4OH$ or $NH_3$
-20° to 40°C
0.5 to 10 hours

2

The reaction of Equation 5a is accomplished by treating a solution of amine 11 in a mixture of concentrated hydrochloric acid and glacial acetic acid with a solution of sodium nitrite in water at -5° to 5°C. After being stirred for 10-30 minutes at about 0°C to insure complete diazotization, the solution is added to a mixture of an excess of sulfur dioxide and a catalytic amount of copper(I) chloride or copper(II) chloride in glacial acetic acid at about 10°C. The temperature is kept at about 10°C for 1/4 to 1 hour, then raised to 20° to 30°C and held at that temperature for 2 to about 24 hours. This solution is then poured into a large excess of ice water. The sulfonyl chloride 12 can be isolated by filtration or by extraction into a solvent such as ethyl ether, methylene chloride or, preferably, 1-chlorobutane followed by evaporation of the solvent.

The amination described in the reaction of Equation 5b above is conveniently carried out by treating a

solution of the sulfonyl chloride 12 with at least two mole equivalents of anhydrous ammonia in a solvent such as ethyl ether or methylene chloride at -20° to 30° C. If the sulfonamide product 2 is insoluble, it may be isolated by filtration followed by washing out the salts with water. If product 2 is soluble in the reaction solvent, it may be isolated by filtering off the precipitated ammonium chloride and evaporation of the solvent. Alternatively, many sulfonamides 2 can be prepared by reaction of corresponding sulfonyl chlorides 12 with excess aqueous ammonium hydroxide in tetrahydrofuran at 0° to about 40° C for 0.5 to 10 hours. The sulfonamide product 2 is isolated by evaporation of the tetrahydrofuran solvent, addition of water to the residue and filtration.

Alternatively, the intermediate sulfonyl chloride 12 can be prepared as shown below in Equation 6.

Equation 6

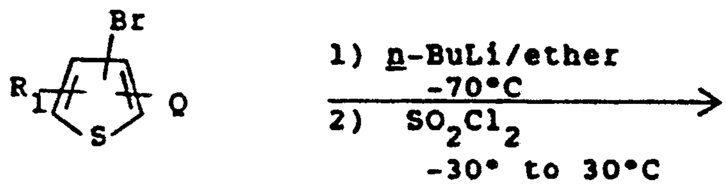


12

13

According to Equation 6, a lithium salt, prepared by reaction of 13 with n-butyllithium in ether at about -70° C, is added to sulfuryl chloride in hexane at about -30° to -20° C and stirred for 0.5 to 10 hours at -30° to 30° C to yield sulfonyl chloride 12, according to teachings of S. N. Bhattacharya, C. Earborn and D. R. M. Walsh, J. Chem Soc. (C) 1968, 1265 and H. Quast and F. Kee, Synthesis 1974, 489. Subsequent reaction of 12 with ammonia or ammonium hydroxide as described above provides the corresponding sulfonamide.

Starting with an appropriate bromothiophene, and carrying out the procedures described in Equation 6, or simple modifications thereof, one skilled in the art may prepare some of the other sulfonyl chlorides of Formula 12 described above. Of necessity, the reactions are limited to those cases in which the substituents Q and $R_1$ are inert to lithium reagents under the conditions of the reactions, which will be obvious to one skilled in the art. For reviews of metallation with lithium reagents, see H. W. Gschwend and H. R. Rodriguez, Org. Reactions 1979, 26, 1; and N. S. Narasimhan, R. S. Mali, Synthesis 1983, 957.

Some sulfonamides 2 are best prepared by the procedure of Equation 7 below.

Equation 7

13 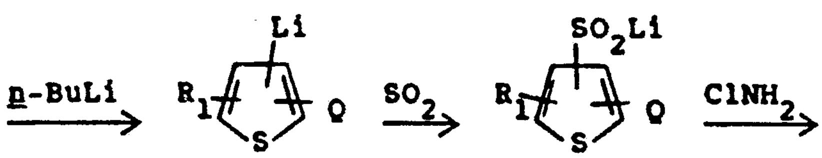


2

14

The preparation of sulfinic acid salts 14 by the procedure of Equation 7 is well known in the art; see U.S. 4,441,910 and H. W. Gschwend et al., loc. cit.. Sulfonamides 2 can be prepared by treatment of sulfinic acid salts with chloramine. In this procedure an ethereal solution or suspension of the salt 14 is treated at low temperature (25 to -30° C) with a dry ethereal solution of chloramine. The reaction is stirred for a period of several minutes to several hours. After filtration, the reaction mixture is washed with aqueous bisulfite, dried and the solvent removed on a rotary evaporator. The crude product is further purified by usual methods such as crystallization or chromatography.

In the reaction shown in Equation 8, a thienyl copper compound of Formula 15 is reacted with an iodo or bromo compound QX, where X is I or Br, in a solvent such as pyridine or quinoline. The copper

compounds of Formula 15 are prepared by reacting the corresponding lithium compounds with cuprous iodide or cuprous bromide in a solvent such a diethyl ether. Detailed procedures for these reactions are described in the following references: M. Nilsson and C. Ullenius, Acta Chem. Scand. 1970, 24. 2379-2388; C. Ullenius, Acta Chem. Scand. 1972, 26, 3383-3386.

Equation 8

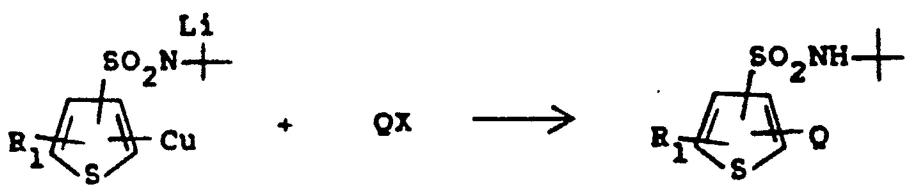


15 16

Alternatively, compounds of Formula 16 wherein Q is CN can also be prepared as shown in Equation 8a by the reaction of a bromothiophenesulfonamide of Formula 15a with cuprous cyanide in a solvent such as dimethylformamide at ambient to reflux temperature. Detailed procedures for this reaction are described below.

Equation 8a

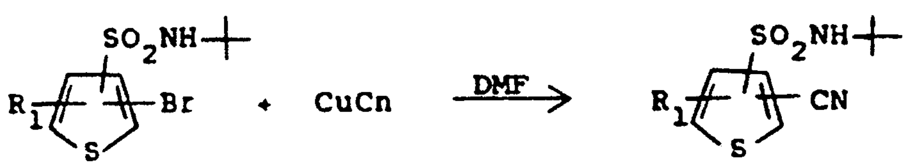


15a 16a

Treatment of the compounds of Formula 16 with an acid catalyst in an alcohol solvent or in trifluoroacetic acid removes the t-butyl group to yield compounds of Formula 2 as shown in Equation 9.

Equation 9

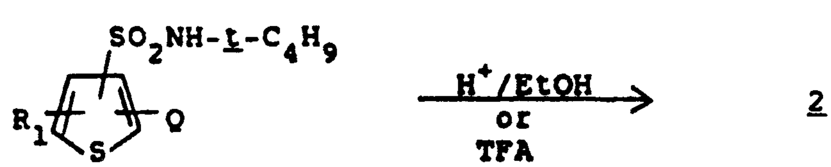


16

Starting with the appropriate 3-substituted thiophene and following the metallation procedure indicated in Equation 7 or simple modifications thereof, one skilled in the art may prepare many of the sulfonamides of Formula 2. Only those 3-substituents which are inert to metallating reagents relative to the desired 2-metallation of the thiophene nucleus are compatible with this synthetic strategy; such substituents will be obvious to one skilled in the art.

In complement to the metallation-chlorosulfonation sequence illustrated by Equation 6, equivalent

metallation-sulfination may be performed to obtain the sulfonamides of Formula 2a. The latter sequence is described by Equation 10 and is entirely analogous to that of Equation 7.

Equation 10

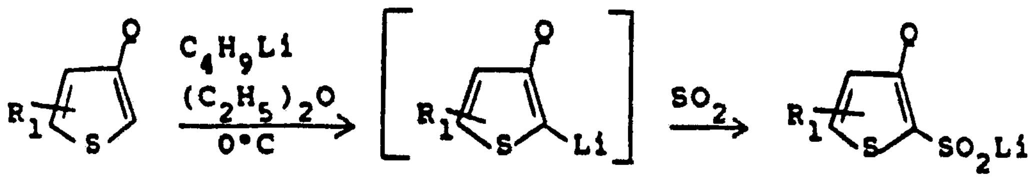


17 18

18

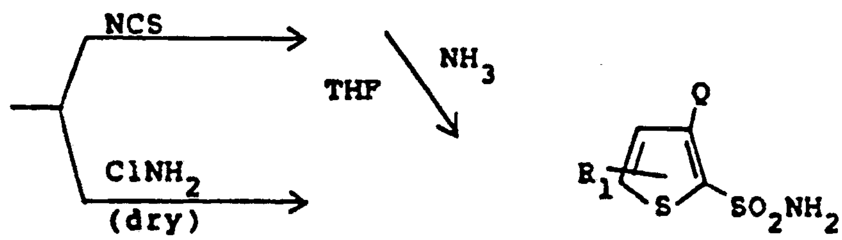


2a

Once sulfinate salts of Formula 18 have been made, they may be transformed into sulfonamides 2a directly by reaction with chloramine as described by G. H. Coleman and C. R. Hauser J. Am. Chem. Soc. 1928, 50. 1193. Sulfinate salts of Formula 18 also may be converted to 2a in a two-step process: chlorination to afford a sulfonyl chloride, as practiced by J. F. Sculley and E. V. Brown J. Org. Chem. 1954. 19, 894; W. E. Trull and E. Wellisch J. Am. Chem. Soc. 1952. 74, 5177 and Y. K. Yuriev and N. K. Sadavaya J. Gen. Chem. USSR 1964, 34, 1814 and treatment of that sulfonyl chloride with ammonia in an ethereal solvent such as THF. Any of these procedures also are compatible with those sulfinate salts of Formula 14 generated via Equation 7.

The use of the strategy of Equation 11 is especially appealing in those cases where metallation of the 3-substituted thiophene of Formula 17 in the 2-position is not possible due to the reactivity of Q under the conditions of metallation. Formation of the 2-brominated thiophene 19 allows for exceedingly facile, mild halogen-metal exchange conditions as shown in Equation 11.

Equation 11

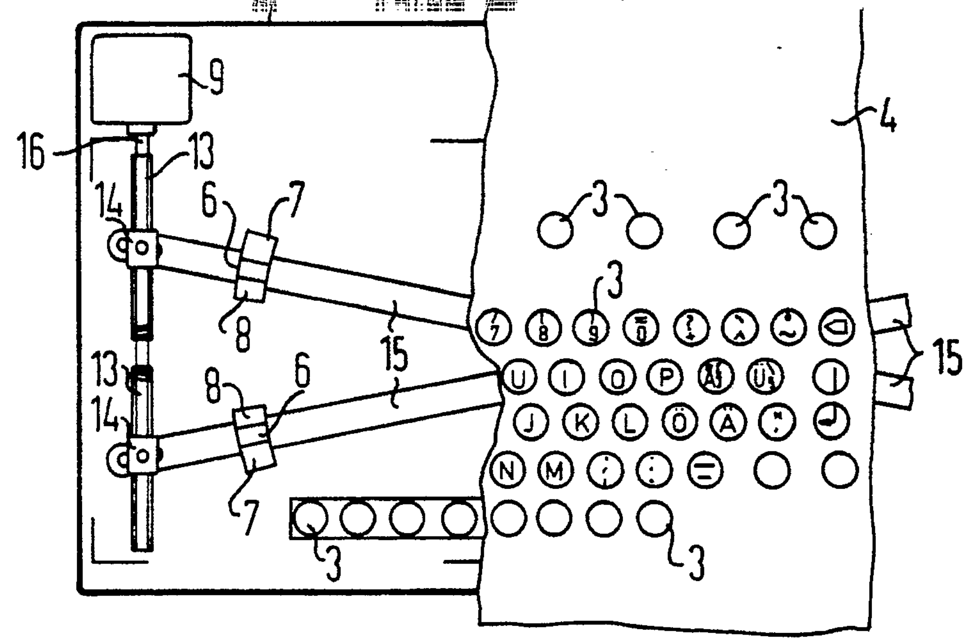


Electrophilic aromatic bromination of 3-substituted thiophenes of Formula 17 leads to the 2-brominated compounds of Formula 19 selectively, as shown in the teachings of S. Gronowitz, P. Moses and A. B. Hornfeldt Ark. Kem. 1962, 17. 165. Subsequent halogen-metal exchange and sulfination with sulfur dioxide gives the sulfinate salts 18, or sulfonation with sulfuryl chloride leads to the sulfonyl chlorides 20.

In still other cases where Q will suffer addition of most nucleophiles, Q can be placed at the 3-position of the thiophene nucleus after the sulfonamide moiety or its synthetic equivalent has been incorporated at the 2-position. This strategy is shown in Equation 12.

Equation 12

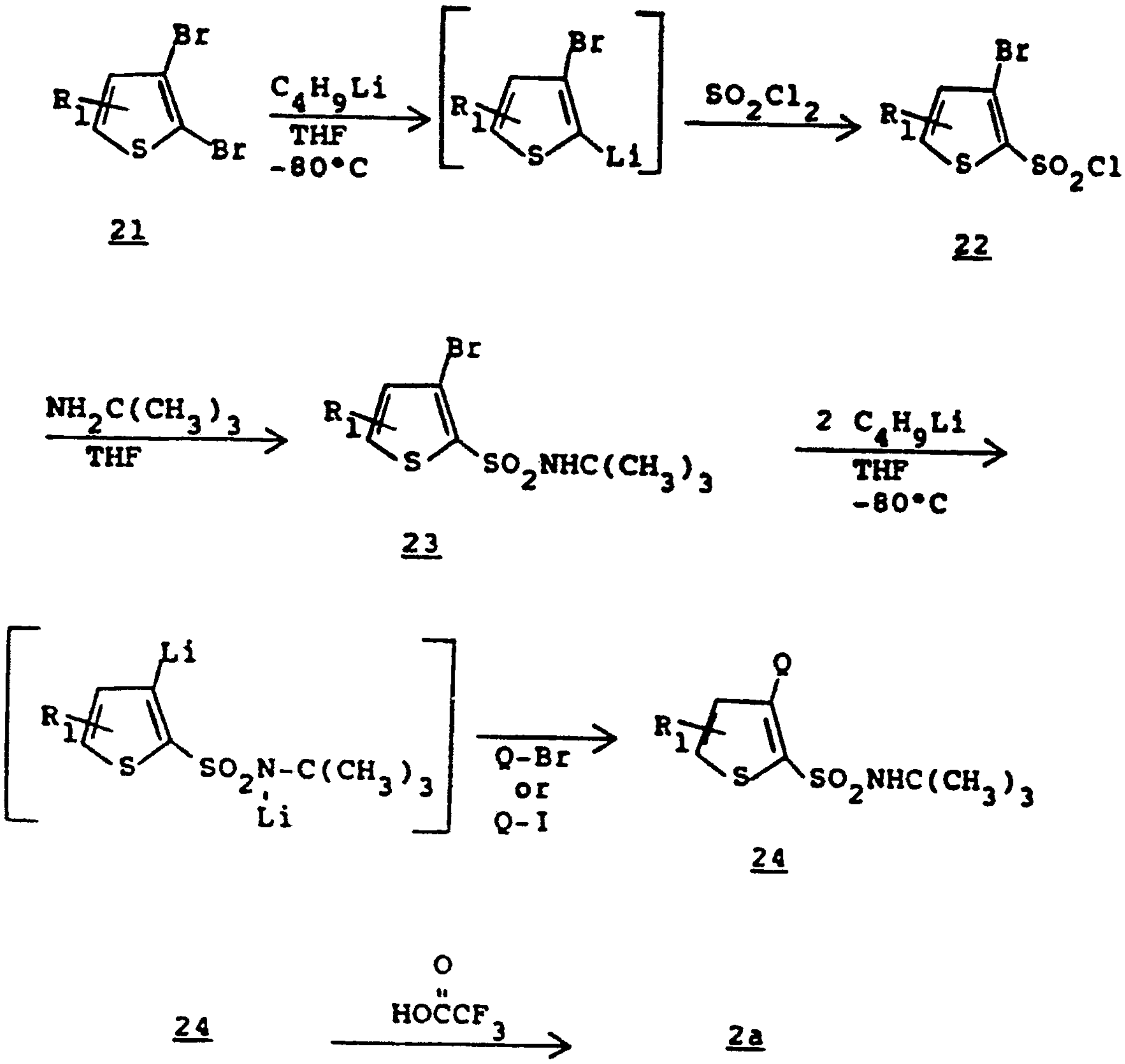


21 22 23 24 24 2a

2,3-Dibromothiophene 21 can be lithiated preferentially at the 2-position and treated with sulfuryl chloride to give the sulfonyl halide 22. Treatment with tert-butylamine results in the tert-butyl-protected sulfonamide of Formula 23. A second lithium-halogen exchange reaction allows for 3-substitution of the heterocycle, Q, which may be added as an intact entity or as a synthetically equivalent substructure.

A judicious choice of the appropriate methods for preparing compounds of Formula 2 must take into account the nature of the substituents Q and $R_1$, and their chemical compatibility with the reaction conditions of Equations 1-12.

The heterocyclic amines of Formula 5 in Equation 1a above can be prepared by methods known in the literature, or simple modifications thereof, by those skilled in the art. For instance, EP-A No. 84,224 (published in July 27, 1983) and W. Braker et al., J. Am. Chem. Soc.. 69. 3072 (1947) describe methods for preparing aminopyrimidines and triazines substituted by acetal groups such as dialkoxymethyl or 1,3-dioxolan-yl, among other groups. Also, for example, South African patent application Nos. 82/5045 and 82/5671 describe methods for preparing aminopyrimidines and triazines substituted by haloalkyl or haloalkylthio groups such as $OCH_2CH_2F$, $OCH_2CF_3$, $SCF_2H$, or $OCF_2H$, among other groups. South African patent application No. 83/7434 (published October 5, 1983) describes methods for the synthesis of cyclopropylpyrimidines and triazines substituted by such groups as alkyl, haloalkyl, alkoxy, haloalkoxy, alkylamino, dialkylamino or alkoxyalkyl.

The 5,6-dihydrofuro[2,3-d]pyrimidine-2-amines, the cyclopenta[d]pyrimidines-2-amines (5, A is A-2) and the 6,7-dihydro-5H-pyrano[2,3-d]pyrimidin-2-amines (5, A is A-3) can be prepared as described in EP-A No. 15,683. The furo[2,3-d]pyrimidin-2-amines (5, A is A-4) are described in EP-A No. 46,677.

Compounds of Formula 5 where A is A-5, are described in EP-A-73,562. Compounds of Formula 5 where A is A-6, are described in EP-A-94,260.

In addition, general methods for preparing aminopyrimidines and triazines have been reviewed in the following publications:

- "The Chemistry of Heterocyclic Compounds", a series published by Interscience Publishers, Inc., New York and London;
- "Pyrimidines", Vol. 16 of the same series by D. J. Brown;
- "s-Triazines and Derivatives", Vol. 13 of the same series by E. M. Smolin and L. Rappoport; and
- F. C. Schaefer, U.S. Patent 3,154,547 and K. R. Huffman and F. C. Schaefer. J. Org. Chem., 28, 1812 (1963), which describes the synthesis of triazines.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide or carbonate). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is further illustrated by the following specific examples.

## Example 1

### 3-(Methoxymethyl)thiophene-2-sulfonamide

To 22.3 g of 3-(methoxymethyl)thiophene dissolved in 180 mL of dry ether under nitrogen is added, dropwise with stirring at 0 °C, 120 mL of a 1.6M solution of n-butyllithium in hexane. The mixture is stirred at 0 °C for two hours, and 24.1 g of sulfuryl chloride is added while the temperature is maintained at -40 °C. After two hours 5 mL of ethyl acetate is added and the reaction mixture is poured into ice and water. The organic phase is separated and added to 25 mL of concentrated ammonium hydroxide at ambient temperature.

After evaporation of the ether and hexane from the reaction mixture, the desired sulfonamide can be separated from the aqueous residue, washed with water and dried. The product can be purified by chromatography using procedures described by W. C. Still et al., J. Org. Chem., 43. 2923 (1978).

## Example 2

### N-[(4,6-Dimethoxypyrimidin-2-yl)aminocarbonyl]-3-(methoxymethyl)thiophene-2-sulfonamide

To 0.41 g of 3-(methoxymethyl)thiophene-2-sulfonamide and 0.55 g of phenyl N-(4,6-dimethoxypyrimidin-2-yl)carbamate in 20 mL of acetonitrile is added 0.3 mL of 1,8-diazabicyclo-[5.4.0]-undec-7-ene (DBU). After being stirred for 2 hours at ambient temperature and pressure, the reaction mass is poured onto 25-50 g of ice and the resultant mixture acidified to pH 3 by the addition of hydrochloric acid. The desired solid product can be isolated by filtration. Alternatively, it can be extracted into methylene chloride which is then dried over magnesium sulfate, filtered and evaporated to dryness to yield the desired product in sufficiently pure form for the purposes of this invention.

## Example 3

### N-[(4-Methoxy-6-methylpyrimidin-2-yl)aminocarbonyl]-3-(methoxymethyl)thiophene-2-sulfonamide

To 0.41 g of 3-(methoxymethyl)thiophene-2-sulfonamide and 0.52 g of phenyl N-(4-methoxy-6-methylpyrimidin-2-yl)carbamate in 25 mL of 1,4-dioxane is added 0.3 mL of 1,8-diazabicyclo[5.4]undec-7-ene. After being stirred for two hours at ambient temperature and pressure, the reaction mixture is poured onto 25-50 g of ice and the resultant mixture is acidified to pH 3 by the addition of hydrochloric acid. The desired solid product can be isolated by filtration. Alternatively, it can be extracted into methylene chloride which is then dried over magnesium sulfate, filtered and evaporated to dryness to yield the desired product in sufficiently pure form for the purposes of this invention.

Example 4

3-(2-Methoxyethyl)thiophene-2-sulfonyl chloride

To 56 g of chlorosulfonic acid at -15° C was added with efficient stirring 14.2 g of 3-(2-methoxyethyl)-thiophene. The mixture was stirred for one hour at -10° C and then cautiously added to ice and the resultant mixture extracted with methylene chloride. The methylene chloride extract was washed with cold water, dried over magnesium sulfate, filtered and concentrated to yield 6.0 g of an oil. Infrared absorption spectroscopy showed peaks at 1160, 1180 and 1360 $cm^{-1}$ consistent for sulfonyl chloride. This product was sufficiently pure for use as an intermediate for the preparation of compounds of this invention.

Example 5

3-(2-Methoxyethyl)thiophene-2-sulfonamide

Six grams of crude 3-(2-methoxyethyl)thiophene-2-sulfonyl chloride was dissolved in 25 mL of acetone and added dropwise with stirring at ambient temperature to 25 mL of concentrated aqueous ammonium hydroxide. The reaction mixture was allowed to stand overnight and the acetone removed by evaporation. The aqueous residue was extracted with three 100 mL portions of methylene chloride which were combined, dried over magnesium sulfate, filtered and evaporated to yield a residue which was a mixture of an oil and a solid. The sulfonamide was isolated by passing the residue through a 30 cm high by 80 mm diameter column of silica gel using 1:1 ethyl acetate mixed hexanes solvent system and taking 100 mL fractions. The desired product came off in fractions 4 to 12. After evaporation of the solvent 1.84 grams was obtained, m.p. 64-73°. Analysis by mass spectroscopy showed a molecular weight of 221 AMU in agreement for the desired sulfonamide.

Example 6

N-[(4-Chloro-6-methoxypyrimidin-2-yl)aminocarbonyl]-3-(2-methoxyethyl)thiophene-2-sulfonamide

To a solution of 0.33 g of 3-(2-methoxyethyl)thiophene-2-sulfonamide and 0.42 g of phenyl N-(4-chloro-6-methoxypyrimidin-2-yl)carbamate in 20 mL of acetonitrile was added with stirring at ambient temperature 0.23 mL of DBU. After stirring for two hours the mixture was poured into 25 g of ice and water, acidified with 12 N hydrochloric acid and the resultant precipitate removed by filtration, washed with cold water and dried to yield 0.37 g of product, m.p. 135-141° C. It showed peaks by infrared absorption spectroscopy at 1700, 1600 and 1560 $cm^{-1}$ consistent for the desired structure.

NMR ($CDCl_3$) 3.27 δ, s, $CH_3O$;
3.27 δ, t, $CH_2$;
3.65 δ, t, $CH_2$;
4.02 δ, t, $CH_3O$ on pyrimidine;
6.49 δ, s, CH of pyrimidine;
7.05 δ, d, 7.60 δ, d, thiophene;
7.05 δ, ws, NH between

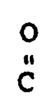

and hetero; and
12.0 δ, s, NH (imide).

Example 7

N-[(4,6-Dimethoxypyrimidin-2-yl)aminocarbonyl]-3-(2-methoxyethyl)-2-thiophenesulfonamide

To a mixture of 0.33 g of 3-(2-methoxyethyl)thiophene-2-sulfonamide and 0.41 g of phenyl N-(4,6-dimethoxypyrimidin-2-yl)carbamate in 20 mL of acetonitrile was added 0.23 mL of 1,8-diazabicyclo[5.4.0]-undec-7-ene (DBU) at ambient temperature with stirring. After 2 hours the mixture was poured onto ice and acidified with 12 N hydrochloric acid. The resultant precipitate was removed by filtration, washed with water and dried to yield 0.42 g, m.p. 173-176°.

NMR ($CDCl_3$): 3.268 δ, s, $CH_3O$;
3.27 δ, t, $CH_2$;
3.64 δ, t, $CH_2$;
3.98 δ, s, 2 X $CH_3O$ of pyrimidine;
5.78 δ, s, CH of pyrimidine; and
7.2, 7.6 δ, d, 2 X CH of thiophene.

Example 8

2-Hydroxymethylthiophene-3-sulfonamide

To 5.0 g of methyl 3-aminosulfonylthiophene-2-carboxylate in 200 mL of tetrahydrofuran was added 0.75 g of lithium borohydride. After heating at reflux for two hours the reaction was quenched with water and acidified with concentrated hydrochloric acid. The desired product was then extracted into methylene chloride which was washed with water, dried over magnesium sulfate, filtered and concentrated in vacuo to yield 1.8 g of a yellow solid, m.p. 85-89°.

NMR ($CDCl_3$): 7.3 δ, AB quartet, 2H on thiophene;
6.8 δ, sw, 2H of $NH_2$;
5.4 δ, s. 1H of OH; and
4.95 δ, d, 2H of $CH_2$.

Example 9

2-Chloromethylthiophene-3-sulfonamide

To a stirred suspension of 16 g of 2-hydroxymethylthiophene-3-sulfonamide, 150 mL of methylene chloride and 10.5 mL of N,N-dimethylaniline was added 6.0 mL of thionyl chloride. An ice cooling bath was applied when the reaction temperature rose to 40° and the mixture was cooled to room temperature. It was then diluted with 50 mL of 1-chlorobutane followed by the addition of 200 mL of ice and water. The precipitate thus formed was isolated by filtration, washed with water and air-dried to yield 12.2 g of white solid, m.p. 100° dec.

NMR ($CDCl_3$-DMSO): 7.4 δ, s, 2H, thiophene;
7.4-5.5 δ, vbs, 2H, $NH_2$; and
5.2 δ, s, 2H, $CH_2$.

Example 10

3-Cyano-N-(1,1-dimethylethyl)thiophene-2-sulfonamide

A mixture of 197.7 g of 3-bromo-N-(1,1-dimethylethyl)thiophene-2-sulfonamide, 195.9 g of cuprous cyanide and 500 mL of dimethylformamide was heated to 150° for two and one half hours at which time none of the bromothiophene remained as indicated by thin layer chromatography. The reaction was cooled to room temperature and poured into 1500 mL of 10% aqueous sodium cyanide. The aqueous mixture was extracted with five, 200 mL portions of ethyl ether and the combined extracts were washed with five, 150 mL portions of water followed by one, 200 mL aqueous sodium chloride wash. After drying over magnesium sulfate, filtration and evaporation of the solvent 140.8 g of the desired product was obtained.

Example 11

3-Cyanothiophene-2-sulfonamide

Ten grams of 3-cyano-N-(1,1-dimethylethyl)thiophene-2-sulfonamide was dissolved in 500 mL of trifluoroacetic acid and stirred overnight at room temperature. After removal of the solvent by evaporation, the residue was dissolved in ethyl acetate and washed with four portions of 100 mL of water followed by saturated sodium chloride. After drying over magnesium sulfate, the solvent was evaporated in vacuo to yield 6.42 g of white solid, m.p. 152-155°. It showed peaks by Nuclear Magnetic Resonance consistent for the desired structure.

Following the procedures described earlier and exemplified in Examples 1-11, one skilled in the art can prepare the compounds of Tables I-XI.

## General Formulas for Tables I-XIV

General Formula

I

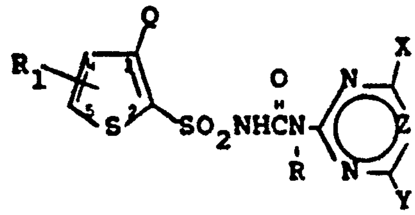

II

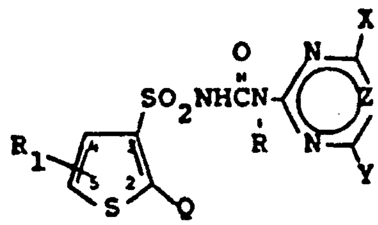

III

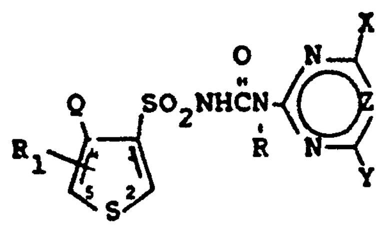

IV

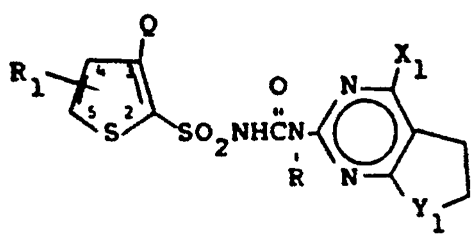

V

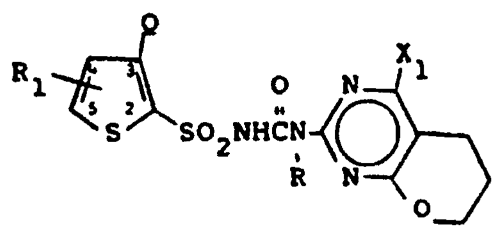

General Formulas (continued)

General Formula

VI

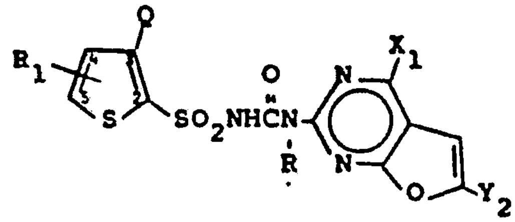

VII

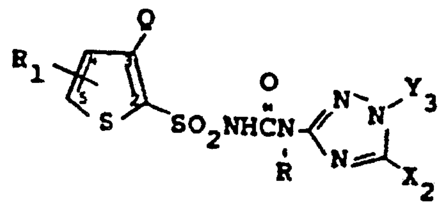

VIII

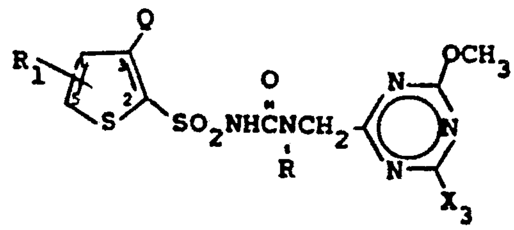

IX

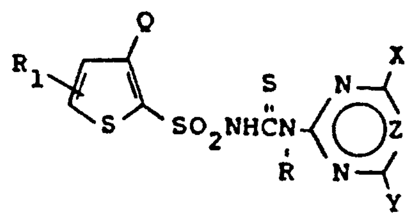

X

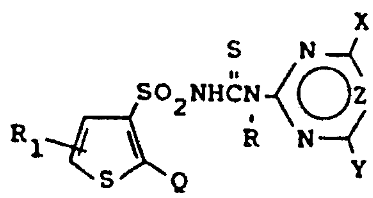

XI

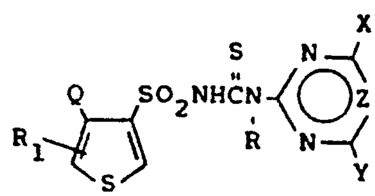

Table I

General Formula I

| R | $R_1$ | Q | X | Y | Z | m.p. (°C) |
|---|---|---|---|---|---|---|
| H | H | $S(CH_2)_2OCH_3$ | $CH_3$ | $OCH_3$ | CH | |
| H | H | $S(CH_2)_2OCH_3$ | $OCH_3$ | $OCH_3$ | CH | |
| H | H | $S(CH_2)_2OCH_3$ | Cl | $OCH_3$ | CH | |
| H | H | $S(CH_2)_2OCH_3$ | $CH_3$ | $OCH_3$ | N | |
| H | H | $S(CH_2)_3OCH_3$ | $CH_3$ | $OCH_3$ | CH | |
| H | H | $S(CH_2)_3OCH_3$ | $OCH_3$ | $OCH_3$ | CH | |
| H | H | $S(CH_2)_2OCH_2CN$ | $OCH_3$ | $OCH_3$ | CH | |
| H | H | $S(CH_2)_2OCH_2CN$ | $CH_3$ | $OCH_3$ | N | |
| H | H | $S(CH_2)_2SCH_3$ | $CH_3$ | $CH_3$ | CH | |
| H | H | $S(CH_2)_2SCH_3$ | $CH_3$ | $OCH_3$ | CH | |
| H | H | $S(CH_2)_2SCH_3$ | $OCH_3$ | $OCH_3$ | CH | |
| H | H | $S(CH_2)_2SCH_3$ | $CH_3$ | $OCH_3$ | N | |
| H | H | $S(CH_2)_2S(O)CH_3$ | $CH_3$ | $OCH_3$ | CH | |
| H | H | $S(CH_2)_2S(O)CH_3$ | $OCH_3$ | $OCH_3$ | CH | |
| H | H | $S(CH_2)_2S(O)CH_3$ | Cl | $OCH_3$ | CH | |
| H | H | $S(CH_2)_2S(O)CH_3$ | $CH_3$ | $OCH_3$ | N | |
| H | H | $S(CH_2)_2S(O)CH_2CH_3$ | $OCH_3$ | $OCH_3$ | CH | |
| H | H | $S(CH_2)_2S(O)CH_2CH_3$ | Cl | $OCH_3$ | CH | |
| H | H | $S(CH_2)_2SO_2CH_3$ | $OCH_3$ | $OCH_3$ | CH | |
| H | H | $S(CH_2)_2SO_2CH_3$ | Cl | $OCH_3$ | CH | |
| H | H | $S(CH_2)_2SO_2CH_3$ | $CH_3$ | $OCH_3$ | N | |
| H | H | $S(CH_2)_2SO_2CH_2CH_3$ | $CH_3$ | $OCH_3$ | CH | |
| H | H | $S(CH_2)_2SO_2CH_2CH_3$ | $OCH_3$ | $OCH_3$ | CH | |
| H | H | $SCH_2CO_2CH_3$ | $CH_3$ | $OCH_3$ | CH | |
| H | H | $SCH_2CO_2CH_3$ | $OCH_3$ | $OCH_3$ | CH | |
| H | H | $SCH_2CO_2CH_3$ | Cl | $OCH_3$ | CH | |
| H | H | $SCH_2CO_2CH_3$ | $CH_3$ | $OCH_3$ | N | |
| H | H | $S(CH_2)_3SO_2N(CH_3)_2$ | $OCH_3$ | $OCH_3$ | CH | |
| H | H | $S(CH_2)_2SO_2N(CH_2CH_3)_2$ | $CH_3$ | $OCH_3$ | CH | |
| H | H | $S(CH_2)_2SO_2N(CH_2CH_3)_2$ | $OCH_3$ | $OCH_3$ | CH | |
| H | H | $SCH_2C(O)CH_3$ | $OCH_3$ | $OCH_3$ | CH | |

Table I (Continued)

| R | $R_1$ | Q | X | Y | Z | m.p. (°C) |
|---|---|---|---|---|---|---|
| H | H | $SCH_2C(O)CH_3$ | $CH_3$ | $OCH_3$ | N | |
| H | H | $S(CH_2)_2CN$ | $CH_3$ | $OCH_3$ | CH | |
| H | H | $S(CH_2)_2CN$ | $OCH_3$ | $OCH_3$ | CH | |
| H | H | $S(CH_2)_2CN$ | $CH_3$ | $OCH_3$ | N | |
| H | H | $SCH_2C(O)N(CH_3)_2$ | $CH_3$ | $OCH_3$ | CH | |
| H | H | $SCH_2C(O)N(CH_3)_2$ | $OCH_3$ | $OCH_3$ | CH | |
| H | H | $SCH_2C(O)N(CH_3)_2$ | $CH_3$ | $OCH_3$ | N | |
| H | 4-$CH_3$ | $S(CH_2)_2C(O)N(CH_3)_2$ | $CH_3$ | $OCH_3$ | CH | |
| H | 4-$CH_3$ | $S(CH_2)_2C(O)N(CH_3)_2$ | $OCH_3$ | $OCH_3$ | CH | |
| H | 4-$CH_3$ | $S(CH_2)_2C(O)N(CH_3)_2$ | $CH_3$ | $OCH_3$ | N | |
| H | H | $S(CH_2)_2Cl$ | $CH_3$ | $CH_3$ | CH | |
| H | H | $S(CH_2)_2Cl$ | $CH_3$ | $OCH_3$ | CH | |
| H | H | $S(CH_2)_2Cl$ | $OCH_3$ | $OCH_3$ | CH | |
| H | H | $S(CH_2)_2Cl$ | Cl | $OCH_3$ | CH | |
| H | H | $S(CH_2)_2Cl$ | $CH_3$ | $OCH_3$ | N | |
| H | H | $S(CH_2)_2Cl$ | $OCH_3$ | $OCH_3$ | N | |
| H | H | $S(CH_2)_3Cl$ | $CH_3$ | $OCH_3$ | CH | |
| H | H | $S(CH_2)_3Cl$ | $OCH_3$ | $OCH_3$ | CH | |
| H | H | $S(CH_2)_3Cl$ | $CH_3$ | $OCH_3$ | N | |
| H | H | $S(CH_2)_2F$ | $OCH_3$ | $OCH_3$ | CH | |
| H | H | $S(CH_2)_2F$ | $CH_3$ | $OCH_3$ | N | |
| H | H | $S(CH_2)_2Br$ | $CH_3$ | $CH_3$ | CH | |
| H | H | $S(CH_2)_2Br$ | $OCH_3$ | $OCH_3$ | CH | |
| H | 4-Cl | $SCHClCH=CH_2$ | $CH_3$ | $OCH_3$ | CH | |
| H | 4-Cl | $SCHClCH=CH_2$ | $OCH_3$ | $OCH_3$ | CH | |
| H | 4-Cl | $SCHClCH=CH_2$ | $CH_3$ | $OCH_3$ | N | |
| H | H | $SCH_2C{\equiv}CH$ | $CH_3$ | $OCH_3$ | CH | |
| H | H | $SCH_2C{\equiv}CH$ | $OCH_3$ | $OCH_3$ | CH | |
| H | H | $SCH_2C{\equiv}CH$ | $CH_3$ | $OCH_3$ | N | |
| H | H | $S(CH_2)_2C{\equiv}CH$ | $CH_3$ | $OCH_3$ | CH | |
| H | H | $S(CH_2)_2C{\equiv}CH$ | $OCH_3$ | $OCH_3$ | CH | |
| H | H | $S(CH_2)_2C{\equiv}CH$ | $CH_3$ | $OCH_3$ | N | |

Table I (Continued)

| R | $R_1$ | Q | X | Y | Z | m.p. (°C) |
|---|---|---|---|---|---|---|
| H | 4-Cl | $SO_2(CH_2)_2OCH_3$ | $OCH_3$ | $OCH_3$ | CH | |
| H | 4-Cl | $SO_2(CH_2)_2OCH_3$ | $CH_3$ | $OCH_3$ | N | |
| H | H | $SO_2CH_2OCH_2CH_3$ | $OCH_3$ | $OCH_3$ | CH | |
| H | H | $SO_2CH_2OCH_2CH_3$ | $CH_3$ | $OCH_3$ | N | |
| H | H | $SO_2(CH_2)_2OCH_2CH_3$ | $OCH_3$ | $OCH_3$ | CH | |
| H | H | $SO_2(CH_2)_2OCH_2CH_3$ | $CH_3$ | $OCH_3$ | N | |
| H | H | $SO_2(CH_2)_2SCH_3$ | $CH_3$ | $OCH_3$ | CH | |
| H | H | $SO_2(CH_2)_2SCH_3$ | $OCH_3$ | $OCH_3$ | CH | |
| H | H | $SO_2(CH_2)_2SCH_3$ | $CH_3$ | $OCH_3$ | N | |
| H | H | $SO_2(CH_2)_2SCH_2CH_3$ | $OCH_3$ | $OCH_3$ | CH | |
| H | H | $SO_2(CH_2)_2SCH_2CH_3$ | $CH_3$ | $OCH_3$ | N | |
| H | H | $SO_2(CH_2)_2S(O)CH_3$ | $OCH_3$ | $OCH_3$ | CH | |
| H | H | $SO_2(CH_2)_2S(O)CH_3$ | $CH_3$ | $OCH_3$ | N | |
| H | H | $SO_2(CH_2)_2CO_2CH_3$ | $OCH_3$ | $OCH_3$ | CH | |
| H | H | $SO_2(CH_2)_2CO_2CH_3$ | $CH_3$ | $OCH_3$ | N | |
| H | H | $SO_2CH_2CO_2CH_2CH_3$ | $CH_3$ | $OCH_3$ | CH | |
| H | H | $SO_2CH_2CO_2CH_2CH_3$ | $OCH_3$ | $OCH_3$ | CH | |
| H | H | $SO_2CH_2CO_2CH_2CH_3$ | $CH_3$ | $OCH_3$ | N | |
| H | H | $SO_2(CH_2)_2C(O)CH_3$ | $CH_3$ | $OCH_3$ | CH | |
| H | H | $SO_2(CH_2)_2C(O)CH_3$ | $OCH_3$ | $OCH_3$ | CH | |
| H | H | $SO_2(CH_2)_2C(O)CH_3$ | $CH_3$ | $OCH_3$ | N | |
| H | H | $SO_2(CH_2)_2C(O)N(CH_3)_2$ | $OCH_3$ | $OCH_3$ | CH | |
| H | H | $SO_2(CH_2)_2C(O)N(CH_3)_2$ | $CH_3$ | $OCH_3$ | N | |
| H | H | $SO_2(CH_2)_2Br$ | $OCH_3$ | $OCH_3$ | CH | |
| H | H | $SO_2(CH_2)_2Br$ | $CH_3$ | $OCH_3$ | N | |
| H | H | $SO_2CH_2F$ | $OCH_3$ | $OCH_3$ | CH | |
| H | H | $SO_2CH_2F$ | $CH_3$ | $OCH_3$ | N | |
| H | H | $SO_2CHClCH{=}CH_2$ | $OCH_3$ | $OCH_3$ | CH | |
| H | H | $SO_2CHClCH{=}CH_2$ | $CH_3$ | $OCH_3$ | N | |
| H | 4-$CH_3$ | $SO_2CH_2C{\equiv}CH$ | $OCH_3$ | $OCH_3$ | CH | |
| H | 4-$CH_3$ | $SO_2CH_2C{\equiv}CH$ | $CH_3$ | $OCH_3$ | N | |
| H | H | $SO_2(CH_2)_2C{\equiv}CH$ | $CH_3$ | $OCH_3$ | CH | |

Table I (Continued)

| R | $R_1$ | Q | X | Y | Z | m.p. (°C) |
|---|---|---|---|---|---|---|
| H | H | $SO_2(CH_2)_2C{\equiv}CH$ | $OCH_3$ | $OCH_3$ | CH | |
| H | H | $SO_2(CH_2)_2C{\equiv}CH$ | $CH_3$ | $OCH_3$ | N | |
| H | H | $CH_2P(O)(OCH_3)_2$ | $OCH_3$ | $OCH_3$ | CH | |
| H | H | $CH_2P(O)(OCH_3)_2$ | $CH_3$ | $OCH_3$ | N | |
| H | H | $P(O)(OCH_3)_2$ | $OCH_3$ | $OCH_3$ | CH | |
| H | H | $P(O)(OCH_3)_2$ | $CH_3$ | $OCH_3$ | N | |
| H | H | $P(O)(SCH_3)_2$ | $OCH_3$ | $OCH_3$ | CH | |
| H | 4-Cl | $CH_2OH$ | $CH_3$ | $CH_3$ | CH | |
| H | 4-Cl | $CH_2OH$ | $CH_3$ | $OCH_3$ | CH | |
| H | 4-Cl | $CH_2OH$ | $OCH_3$ | $OCH_3$ | CH | |
| H | 4-Cl | $CH_2OH$ | Cl | $OCH_3$ | CH | |
| H | 4-Cl | $CH_2OH$ | $CH_3$ | $OCH_3$ | N | |
| H | 4-Cl | $CH_2OH$ | $OCH_3$ | $OCH_3$ | N | |
| H | H | $(CH_2)_2OH$ | $CH_3$ | $CH_3$ | CH | |
| H | H | $(CH_2)_2OH$ | $CH_3$ | $OCH_3$ | CH | |
| H | H | $(CH_2)_2OH$ | $OCH_3$ | $OCH_3$ | CH | |
| H | H | $(CH_2)_2OH$ | Cl | $OCH_3$ | CH | |
| H | H | $(CH_2)_2OH$ | $CH_3$ | $OCH_3$ | N | |
| H | H | $(CH_2)_2OH$ | $OCH_3$ | $OCH_3$ | N | |
| H | H | $CH_2OCH_3$ | $CH_3$ | $CH_3$ | CH | |
| H | H | $CH_2OCH_3$ | $CH_3$ | $OCH_3$ | CH | |
| H | H | $CH_2OCH_3$ | $OCH_3$ | $OCH_3$ | CH | |
| H | H | $CH_2OCH_3$ | Cl | $OCH_3$ | CH | |
| H | H | $CH_2OCH_3$ | $CH_3$ | $OCH_3$ | N | |
| H | H | $CH_2OCH_3$ | $OCH_3$ | $OCH_3$ | N | |
| H | H | $(CH_2)_2OCH_3$ | $CH_3$ | $CH_3$ | CH | |
| H | H | $(CH_2)_2OCH_3$ | $CH_3$ | $OCH_3$ | CH | 135.5-137 |
| H | H | $(CH_2)_2OCH_3$ | $OCH_3$ | $OCH_3$ | CH | 168.5-171 |
| H | H | $(CH_2)_2OCH_3$ | Cl | $OCH_3$ | CH | |
| H | H | $(CH_2)_2OCH_3$ | $CH_3$ | $OCH_3$ | N | 146.5-147.5 |
| H | H | $(CH_2)_2OCH_3$ | $OCH_3$ | $OCH_3$ | N | 145-148 |
| H | H | $(CH_2)_2OCH_2CH_3$ | $CH_3$ | $CH_3$ | CH | |

Table I (Continued)

| R | $R_1$ | Q | X | Y | Z | m.p. (°C) |
|---|---|---|---|---|---|---|
| H | H | $(CH_2)_2OCH_2CH_3$ | $CH_3$ | $OCH_3$ | CH | 147-148.5 |
| H | H | $(CH_2)_2OCH_2CH_3$ | $OCH_3$ | $OCH_3$ | CH | |
| H | H | $(CH_2)_2OCH_2CH_3$ | Cl | $OCH_3$ | CH | |
| H | H | $(CH_2)_2OCH_2CH_3$ | $OCH_3$ | $OCH_3$ | N | |
| H | H | $CH_2O(CH_2)_2CN$ | $CH_3$ | $CH_3$ | CH | |
| H | H | $CH_2O(CH_2)_2CN$ | $CH_3$ | $OCH_3$ | CH | |
| H | H | $CH_2O(CH_2)_2CN$ | $OCH_3$ | $OCH_3$ | CH | |
| H | H | $CH_2O(CH_2)_2CN$ | Cl | $OCH_3$ | CH | |
| H | H | $CH_2O(CH_2)_2CN$ | $CH_3$ | $OCH_3$ | N | |
| H | H | $CH_2O(CH_2)_2CN$ | $OCH_3$ | $OCH_3$ | N | |
| H | H | $CH_2SCH_3$ | $CH_3$ | $CH_3$ | CH | |
| H | H | $CH_2SCH_3$ | $CH_3$ | $OCH_3$ | CH | 128-130 |
| H | H | $CH_2SCH_3$ | $OCH_3$ | $OCH_3$ | CH | |
| H | H | $CH_2SCH_3$ | Cl | $OCH_3$ | CH | |
| H | H | $CH_2SCH_3$ | $CH_3$ | $OCH_3$ | N | |
| H | H | $CH_2SCH_3$ | $OCH_3$ | $OCH_3$ | N | |
| H | H | $(CH_2)_2SCH_3$ | $CH_3$ | $CH_3$ | CH | |
| H | H | $(CH_2)_2SCH_3$ | $CH_3$ | $OCH_3$ | CH | |
| H | H | $(CH_2)_2SCH_3$ | $OCH_3$ | $OCH_3$ | CH | |
| H | H | $(CH_2)_2SCH_3$ | Cl | $OCH_3$ | CH | |
| H | H | $(CH_2)_2SCH_3$ | $CH_3$ | $OCH_3$ | N | |
| H | H | $(CH_2)_2SCH_3$ | $OCH_3$ | $OCH_3$ | N | |
| H | H | $(CH_2)_3SCH_3$ | $CH_3$ | $CH_3$ | CH | |
| H | H | $(CH_2)_3SCH_3$ | $CH_3$ | $OCH_3$ | CH | |
| H | H | $(CH_2)_3SCH_3$ | $OCH_3$ | $OCH_3$ | CH | |
| H | H | $(CH_2)_3SCH_3$ | Cl | $OCH_3$ | CH | |
| H | H | $(CH_2)_3SCH_3$ | $CH_3$ | $OCH_3$ | N | |
| H | H | $(CH_2)_3SCH_3$ | $OCH_3$ | $OCH_3$ | N | |
| H | H | $CH_2S(O)CH_3$ | $CH_3$ | $CH_3$ | CH | |
| H | H | $CH_2S(O)CH_3$ | $CH_3$ | $OCH_3$ | CH | |
| H | H | $CH_2S(O)CH_3$ | $OCH_3$ | $OCH_3$ | CH | |
| H | H | $CH_2S(O)CH_3$ | Cl | $OCH_3$ | CH | |

Table I (Continued)

| R | $R_1$ | Q | X | Y | Z | m.p. (°C) |
|---|---|---|---|---|---|---|
| H | H | $CH_2S(O)CH_3$ | $CH_3$ | $OCH_3$ | N | |
| H | H | $CH_2S(O)CH_3$ | $OCH_3$ | $OCH_3$ | N | |
| H | H | $(CH_2)_2S(O)CH_3$ | $CH_3$ | $CH_3$ | CH | |
| H | H | $(CH_2)_2S(O)CH_3$ | $CH_3$ | $OCH_3$ | CH | |
| H | H | $(CH_2)_2S(O)CH_3$ | $OCH_3$ | $OCH_3$ | CH | |
| H | H | $(CH_2)_2S(O)CH_3$ | Cl | $OCH_3$ | CH | |
| H | H | $(CH_2)_2S(O)CH_3$ | $CH_3$ | $OCH_3$ | N | |
| H | H | $(CH_2)_2S(O)CH_3$ | $OCH_3$ | $OCH_3$ | N | |
| H | H | $(CH_2)_2S(O)CH_2CH_3$ | $CH_3$ | $CH_3$ | CH | |
| H | H | $(CH_2)_2S(O)CH_2CH_3$ | $CH_3$ | $OCH_3$ | CH | |
| H | H | $(CH_2)_2S(O)CH_2CH_3$ | $OCH_3$ | $OCH_3$ | CH | |
| H | H | $(CH_2)_2S(O)CH_2CH_3$ | Cl | $OCH_3$ | CH | |
| H | H | $(CH_2)_2S(O)CH_2CH_3$ | $CH_3$ | $OCH_3$ | N | |
| H | H | $(CH_2)_2S(O)CH_2CH_3$ | $OCH_3$ | $OCH_3$ | N | |
| H | 4-$CH_3$ | $CH_2SO_2CH_3$ | $CH_3$ | $CH_3$ | CH | |
| H | 4-$CH_3$ | $CH_2SO_2CH_3$ | $CH_3$ | $OCH_3$ | CH | |
| H | 4-$CH_3$ | $CH_2SO_2CH_3$ | $OCH_3$ | $OCH_3$ | CH | |
| H | 4-$CH_3$ | $CH_2SO_2CH_3$ | $CH_3$ | $OCH_3$ | N | |
| H | H | $(CH_2)_2SO_2CH_3$ | $CH_3$ | $CH_3$ | CH | |
| H | H | $(CH_2)_2SO_2CH_3$ | $CH_3$ | $OCH_3$ | CH | |
| H | H | $(CH_2)_2SO_2CH_3$ | $OCH_3$ | $OCH_3$ | CH | |
| H | H | $(CH_2)_2SO_2CH_3$ | Cl | $OCH_3$ | CH | |
| H | H | $(CH_2)_2SO_2CH_3$ | $CH_3$ | $OCH_3$ | N | |
| H | H | $(CH_2)_2SO_2CH_3$ | $OCH_3$ | $OCH_3$ | N | |
| H | H | $(CH_2)_3SO_2CH_3$ | $OCH_3$ | $OCH_3$ | CH | |
| H | H | $(CH_2)_3SO_2CH_3$ | $CH_3$ | $OCH_3$ | N | |
| H | H | $(CH_2)_3SO_2CH_3$ | $OCH_3$ | $OCH_3$ | N | |
| H | H | $CH_2SO_2CH_2CH_3$ | $OCH_3$ | $OCH_3$ | CH | |
| H | H | $CH_2SO_2CH_2CH_3$ | Cl | $OCH_3$ | CH | |
| H | H | $CH_2SO_2CH_2CH_3$ | $CH_3$ | $OCH_3$ | N | |
| H | H | $CH_2CO_2CH_3$ | $CH_3$ | $CH_3$ | CH | |
| H | H | $CH_2CO_2CH_3$ | $CH_3$ | $OCH_3$ | CH | |

Table I (Continued)

| R | $R_1$ | Q | X | Y | Z | m.p. (°C) |
|---|---|---|---|---|---|---|
| H | H | $CH_2CO_2CH_3$ | $OCH_3$ | $OCH_3$ | CH | |
| H | H | $CH_2CO_2CH_3$ | Cl | $OCH_3$ | CH | |
| H | H | $CH_2CO_2CH_3$ | $CH_3$ | $OCH_3$ | N | |
| H | H | $CH_2CO_2CH_2CH_3$ | $CH_3$ | $CH_3$ | CH | |
| H | H | $CH_2CO_2CH_2CH_3$ | $CH_3$ | $OCH_3$ | CH | |
| H | H | $CH_2CO_2CH_2CH_3$ | $OCH_3$ | $OCH_3$ | CH | |
| H | H | $CH_2CO_2CH_2CH_3$ | Cl | $OCH_3$ | CH | |
| H | H | $CH_2CO_2CH_2CH_3$ | $CH_3$ | $OCH_3$ | N | |
| H | H | $CH_2SO_2N(CH_3)_2$ | $CH_3$ | $CH_3$ | CH | |
| H | H | $CH_2SO_2N(CH_3)_2$ | $CH_3$ | $OCH_3$ | CH | |
| H | H | $CH_2SO_2N(CH_3)_2$ | $OCH_3$ | $OCH_3$ | CH | |
| H | H | $CH_2SO_2N(CH_3)_2$ | Cl | $OCH_3$ | CH | |
| H | H | $CH_2SO_2N(CH_3)_2$ | $CH_3$ | $OCH_3$ | N | |
| H | H | $CH_2SO_2N(CH_3)_2$ | $OCH_3$ | $OCH_3$ | N | |
| H | 4-Cl | $(CH_2)_2SO_2N(CH_3)_2$ | $CH_3$ | $OCH_3$ | CH | |
| H | 4-Cl | $(CH_2)_2SO_2N(CH_3)_2$ | $OCH_3$ | $OCH_3$ | CH | |
| H | 4-Cl | $(CH_2)_2SO_2N(CH_3)_2$ | $CH_3$ | $OCH_3$ | N | |
| H | H | $CH_2SO_2N(CH_2CH_3)_2$ | $CH_3$ | $OCH_3$ | CH | |
| H | H | $CH_2SO_2N(CH_2CH_3)_2$ | $OCH_3$ | $OCH_3$ | CH | |
| H | H | $CH_2SO_2N(CH_2CH_3)_2$ | $CH_3$ | $OCH_3$ | N | |
| H | H | $CH_2C(O)CH_3$ | $CH_3$ | $OCH_3$ | CH | |
| H | H | $CH_2C(O)CH_3$ | $OCH_3$ | $OCH_3$ | CH | |
| H | H | $CH_2C(O)CH_3$ | $CH_3$ | $OCH_3$ | N | |
| H | 4-Cl | $(CH_2)_2C(O)CH_3$ | $CH_3$ | $OCH_3$ | CH | |
| H | 4-Cl | $(CH_2)_2C(O)CH_3$ | $OCH_3$ | $OCH_3$ | CH | |
| H | 4-Cl | $(CH_2)_2C(O)CH_3$ | $CH_3$ | $OCH_3$ | N | |
| H | H | $CH_2CN$ | $CH_3$ | $CH_3$ | CH | |
| H | H | $CH_2CN$ | $CH_3$ | $OCH_3$ | CH | 127-138 |
| H | H | $CH_2CN$ | $OCH_3$ | $OCH_3$ | CH | 189-191.5 |
| H | H | $CH_2CN$ | Cl | $OCH_3$ | CH | 181-183 |
| H | H | $CH_2CN$ | $CH_3$ | $OCH_3$ | N | 155.5-159 |
| H | H | $CH_2CN$ | $CH_3$ | $CH_3$ | N | 173-176 |

Table I (Continued)

| R | $R_1$ | Q | X | Y | Z | m.p. (°C) |
|---|---|---|---|---|---|---|
| H | H | $CH_2CN$ | $OCH_3$ | $OCH_3$ | N | 167.5-169 |
| H | H | $(CH_2)_2CN$ | $CH_3$ | $CH_3$ | CH | |
| H | H | $(CH_2)_2CN$ | $CH_3$ | $OCH_3$ | CH | |
| H | H | $(CH_2)_2CN$ | $OCH_3$ | $OCH_3$ | CH | |
| H | H | $(CH_2)_2CN$ | Cl | $OCH_3$ | CH | |
| H | H | $(CH_2)_2CN$ | $CH_3$ | $OCH_3$ | N | |
| H | H | $(CH_2)_2CN$ | $OCH_3$ | $OCH_3$ | N | |
| H | H | $CH_2C(O)N(CH_3)_2$ | $CH_3$ | $OCH_3$ | CH | |
| H | H | $CH_2C(O)N(CH_3)_2$ | $OCH_3$ | $OCH_3$ | CH | |
| H | H | $CH_2C(O)N(CH_3)_2$ | $CH_3$ | $OCH_3$ | N | |
| H | H | $(CH_2)_2C(O)N(CH_3)_2$ | $CH_3$ | $OCH_3$ | CH | |
| H | H | $(CH_2)_2C(O)N(CH_3)_2$ | $OCH_3$ | $OCH_3$ | CH | |
| H | H | $(CH_2)_2C(O)N(CH_3)_2$ | $CH_3$ | $OCH_3$ | N | |
| H | H | $CH_2Cl$ | $CH_3$ | $CH_3$ | CH | 152-154 |
| H | H | $CH_2Cl$ | $CH_3$ | $OCH_3$ | CH | 155-156 |
| H | H | $CH_2Cl$ | $OCH_3$ | $OCH_3$ | CH | 183-184 |
| H | H | $CH_2Cl$ | Cl | $OCH_3$ | CH | 168-170 |
| H | H | $CH_2Cl$ | $CH_3$ | $OCH_3$ | N | 150-152 |
| H | H | $CH_2Cl$ | $OCH_3$ | $OCH_3$ | N | 108-110 |
| H | H | $CH_2Cl$ | $CH_3$ | $CH_3$ | N | 141-146 |
| $CH_3$ | H | $CH_2Cl$ | $CH_3$ | $OCH_3$ | N | 125-129 |
| H | H | $(CH_2)_2Cl$ | $OCH_3$ | $OCH_3$ | CH | |
| H | H | $(CH_2)_2Cl$ | Cl | $OCH_3$ | CH | |
| H | H | $(CH_2)_2Cl$ | $CH_3$ | $OCH_3$ | N | |
| H | H | $(CH_2)_2Cl$ | $OCH_3$ | $OCH_3$ | N | |
| H | H | $CH_2F$ | $CH_3$ | $CH_3$ | CH | |
| H | H | $CH_2F$ | $CH_3$ | $OCH_3$ | CH | |
| H | H | $CH_2F$ | $OCH_3$ | $OCH_3$ | CH | |
| H | H | $CH_2F$ | Cl | $OCH_3$ | CH | |
| H | H | $CH_2F$ | $CH_3$ | $OCH_3$ | N | |
| H | H | $CH_2F$ | $OCH_3$ | $OCH_3$ | N | |
| H | H | $(CH_2)_2F$ | $CH_3$ | $OCH_3$ | CH | |

Table I (Continued)

| R | $R_1$ | Q | X | Y | Z | m.p. (°C) |
|---|---|---|---|---|---|---|
| H | H | $(CH_2)_2F$ | $OCH_3$ | $OCH_3$ | CH | |
| H | H | $(CH_2)_2F$ | $CH_3$ | $OCH_3$ | N | |
| H | H | $CH_2Br$ | $CH_3$ | $CH_3$ | CH | |
| H | H | $CH_2Br$ | $CH_3$ | $OCH_3$ | CH | |
| H | H | $CH_2Br$ | $OCH_3$ | $OCH_3$ | CH | |
| H | H | $CH_2Br$ | Cl | $OCH_3$ | CH | |
| H | H | $CH_2Br$ | $CH_3$ | $OCH_3$ | N | |
| H | H | $CH_2Br$ | $OCH_3$ | $OCH_3$ | N | |
| H | H | $(CH_2)_2Br$ | $CH_3$ | $OCH_3$ | CH | |
| H | H | $(CH_2)_2Br$ | $OCH_3$ | $OCH_3$ | CH | |
| H | H | $(CH_2)_2Br$ | Cl | $OCH_3$ | CH | |
| H | H | $CH_2SCH_3$ | $OCH_2CH_3$ | $C_2H_5$ | CH | |
| H | H | $CH_2SCH_3$ | $OCH_2CH_3$ | $C_2H_5$ | N | |
| H | H | $CH_2SCH_3$ | $OCH_2CH_3$ | $CH_2OCH_3$ | CH | |
| H | H | $CH_2SCH_3$ | $OCH_2CH_3$ | $CH_2OCH_3$ | N | |
| H | H | $CH_2SCH_3$ | $OCH_2CH_3$ | $NHCH_3$ | N | |
| H | H | $CH_2SCH_3$ | $OCH_2CH_3$ | $CH(OCH_3)_2$ | CH | |
| H | H | $CH_2SCH_3$ | $OCF_2H$ | $C_2H_5$ | CH | |
| H | H | $CH_2SCH_3$ | $OCF_2H$ | $CH_2OCH_3$ | CH | |
| H | H | $CH_2SCH_3$ | $OCH_2CF_3$ | $C_2H_5$ | CH | |
| H | H | $CH_2SCH_3$ | $OCH_2CF_3$ | $C_2H_5$ | N | |
| H | H | $CH_2SCH_3$ | $OCH_2CF_3$ | $CH_2OCH_3$ | CH | |
| H | H | $CH_2SCH_3$ | $OCH_2CF_3$ | $CH_2OCH_3$ | N | |
| H | H | $CH_2SCH_3$ | $OCH_2CF_3$ | $NHCH_3$ | N | |
| H | H | $CH_2SCH_3$ | $OCH_2CF_3$ | $CH(OCH_3)_2$ | N | |
| H | H | $CH_2SCH_3$ | $CH_3$ | $C_2H_5$ | N | |
| H | H | $CH_2SCH_3$ | $CH_3$ | $CH_2OCH_3$ | CH | |
| H | H | $CH_2SCH_3$ | $CH_3$ | $CH_2OCH_3$ | N | |
| H | H | $CH_2SCH_3$ | $CH_3$ | $NHCH_3$ | N | |
| H | H | $CH_2SCH_3$ | $CH_3$ | $CH(OCH_3)_2$ | N | |
| H | H | $CH_2SCH_3$ | $OCH_3$ | $C_2H_5$ | CH | |
| H | H | $CH_2SCH_3$ | $OCH_3$ | $C_2H_5$ | N | |

Table I (Continued)

| R | $R_1$ | Q | X | Y | Z | m.p. (°C) |
|---|---|---|---|---|---|---|
| H | H | $CH_2SCH_3$ | $OCH_3$ | $CH_2OCH_3$ | CH | |
| H | H | $CH_2SCH_3$ | $OCH_3$ | $CH_2OCH_3$ | N | |
| H | H | $CH_2SCH_3$ | $OCH_3$ | $NHCH_3$ | N | |
| H | H | $CH_2SCH_3$ | $OCH_3$ | $CH(OCH_3)_2$ | CH | |
| H | H | $CH_2SCH_3$ | $OCH_3$ | $CH(OCH_3)_2$ | N | |
| H | 4-Cl | $CH_2OCH_3$ | $OCH_2CH_3$ | $C_2H_5$ | CH | |
| H | 4-Cl | $CH_2OCH_3$ | $OCH_2CH_3$ | $C_2H_5$ | N | |
| H | 4-Cl | $CH_2OCH_3$ | $OCH_2CH_3$ | $CH_2OCH_3$ | CH | |
| H | 4-Cl | $CH_2OCH_3$ | $OCH_2CH_3$ | $CH_2OCH_3$ | N | |
| H | 4-Cl | $CH_2OCH_3$ | $OCH_2CH_3$ | $NHCH_3$ | CH | |
| H | 4-Cl | $CH_2OCH_3$ | $OCH_2CH_3$ | $NHCH_3$ | N | |
| H | 4-Cl | $CH_2OCH_3$ | $OCH_2CH_3$ | $CH(OCH_3)_2$ | CH | |
| H | 4-Cl | $CH_2OCH_3$ | $OCH_2CH_3$ | $CH(OCH_3)_2$ | N | |
| H | 4-Cl | $CH_2OCH_3$ | $OCF_2H$ | $C_2H_5$ | CH | |
| H | 4-Cl | $CH_2OCH_3$ | $OCF_2H$ | $CH_2OCH_3$ | CH | |
| H | 4-Cl | $CH_2OCH_3$ | $OCF_2H$ | $NHCH_3$ | CH | |
| H | 4-Cl | $CH_2OCH_3$ | $OCH_2CF_3$ | $C_2H_5$ | CH | |
| H | 4-Cl | $CH_2OCH_3$ | $OCH_2CF_3$ | $CH_2OCH_3$ | N | |
| H | 4-Cl | $CH_2OCH_3$ | $OCH_2CF_3$ | $NHCH_3$ | CH | |
| H | 4-Cl | $CH_2OCH_3$ | $OCH_2CF_3$ | $NHCH_3$ | N | |
| H | 4-Cl | $CH_2OCH_3$ | $OCH_2CF_3$ | $CH(OCH_3)_2$ | CH | |
| H | 4-Cl | $CH_2OCH_3$ | $OCH_2CF_3$ | $CH(OCH_3)_2$ | N | |
| H | 4-Cl | $CH_2OCH_3$ | Cl | $NHCH_3$ | CH | |
| H | 4-Cl | $CH_2OCH_3$ | $CH_3$ | $C_2H_5$ | CH | |
| H | 4-Cl | $CH_2OCH_3$ | $CH_3$ | $C_2H_5$ | N | |
| H | 4-Cl | $CH_2OCH_3$ | $CH_3$ | $CH_2OCH_3$ | CH | |
| H | 4-Cl | $CH_2OCH_3$ | $CH_3$ | $CH_2OCH_3$ | N | |
| H | 4-Cl | $CH_2OCH_3$ | Cl | $NHCH_3$ | CH | |
| H | 4-Cl | $CH_2OCH_3$ | $CH_3$ | $C_2H_5$ | CH | |
| H | 4-Cl | $CH_2OCH_3$ | $CH_3$ | $C_2H_5$ | N | |
| H | 4-Cl | $CH_2OCH_3$ | $CH_3$ | $CH_2OCH_3$ | CH | |
| H | 4-Cl | $CH_2OCH_3$ | $CH_3$ | $CH_2OCH_3$ | N | |

Table I (Continued)

| R | $R_1$ | Q | X | Y | Z | m.p. (°C) |
|---|---|---|---|---|---|---|
| H | 4-Cl | $CH_2OCH_3$ | $CH_3$ | $NHCH_3$ | N | |
| H | 4-Cl | $CH_2OCH_3$ | $CH_3$ | $CH(OCH_3)_2$ | CH | |
| H | 4-Cl | $CH_2OCH_3$ | $CH_3$ | $CH(OCH_3)_2$ | N | |
| H | 4-Cl | $CH_2OCH_3$ | $OCH_3$ | $C_2H_5$ | CH | |
| H | 4-Cl | $CH_2OCH_3$ | $OCH_3$ | $C_2H_5$ | N | |
| H | 4-Cl | $CH_2OCH_3$ | $OCH_3$ | $CH_2OCH_3$ | CH | |
| H | 4-Cl | $CH_2OCH_3$ | $OCH_3$ | $CH_2OCH_3$ | N | |
| H | 4-Cl | $CH_2OCH_3$ | $OCH_3$ | $NHCH_3$ | CH | |
| H | 4-Cl | $CH_2OCH_3$ | $OCH_3$ | $NHCH_3$ | N | |
| H | 4-Cl | $CH_2OCH_3$ | $OCH_3$ | $CH(OCH_3)_2$ | CH | |
| H | 4-Cl | $CH_2OCH_3$ | $OCH_3$ | $CH(OCH_3)_2$ | N | |
| H | H | CN | $CH_3$ | $CH_3$ | CH | 165-167 |
| H | H | CN | $CH_3$ | $OCH_3$ | CH | 166-168 |
| H | H | CN | $OCH_3$ | $OCH_3$ | CH | 188-190 |
| H | H | CN | Cl | $OCH_3$ | CH | 169-171 |
| H | H | CN | $CH_3$ | $OCH_3$ | N | 150-152 |
| H | H | CN | $OCH_3$ | $OCH_3$ | N | 176-178 |
| H | H | $SO_2NHCH_3$ | $OCH_3$ | $OCH_3$ | CH | |
| H | H | $SO_2$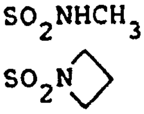 | $OCH_3$ | $OCH_3$ | CH | |
| H | H | $SO_2NHC_6H_5$ | $OCH_3$ | $OCH_3$ | CH | |

Table II

General Formula II

| R | $R_1$ | Q | X | Y | Z | m.p. (°C) |
|---|---|---|---|---|---|---|
| H | H | $S(CH_2)_2OCH_3$ | $CH_3$ | $OCH_3$ | CH | |
| H | H | $S(CH_2)_2OCH_3$ | $OCH_3$ | $OCH_3$ | CH | |
| H | H | $S(CH_2)_2OCH_3$ | Cl | $OCH_3$ | CH | |
| H | H | $S(CH_2)_2OCH_3$ | $CH_3$ | $OCH_3$ | N | |
| H | H | $S(CH_2)_3OCH_3$ | $CH_3$ | $OCH_3$ | CH | |
| H | H | $S(CH_2)_3OCH_3$ | $OCH_3$ | $OCH_3$ | CH | |
| H | H | $S(CH_2)_2OCH_2CN$ | $OCH_3$ | $OCH_3$ | CH | |
| H | H | $S(CH_2)_2OCH_2CN$ | $CH_3$ | $OCH_3$ | N | |
| H | H | $S(CH_2)_2SCH_3$ | $CH_3$ | $CH_3$ | CH | |
| H | H | $S(CH_2)_2SCH_3$ | $CH_3$ | $OCH_3$ | CH | |
| H | H | $S(CH_2)_2SCH_3$ | $OCH_3$ | $OCH_3$ | CH | |
| H | H | $S(CH_2)_2SCH_3$ | $CH_3$ | $OCH_3$ | N | |
| H | H | $S(CH_2)_2S(O)CH_3$ | $CH_3$ | $OCH_3$ | CH | |
| H | H | $S(CH_2)_2S(O)CH_3$ | $OCH_3$ | $OCH_3$ | CH | |
| H | H | $S(CH_2)_2S(O)CH_3$ | Cl | $OCH_3$ | CH | |
| H | H | $S(CH_2)_2S(O)CH_3$ | $CH_3$ | $OCH_3$ | N | |
| H | H | $S(CH_2)_2S(O)CH_2CH_3$ | $OCH_3$ | $OCH_3$ | CH | |
| H | H | $S(CH_2)_2S(O)CH_2CH_3$ | Cl | $OCH_3$ | CH | |
| H | H | $S(CH_2)_2SO_2CH_3$ | $OCH_3$ | $OCH_3$ | CH | |
| H | H | $S(CH_2)_2SO_2CH_3$ | Cl | $OCH_3$ | CH | |
| H | H | $S(CH_2)_2SO_2CH_3$ | $CH_3$ | $OCH_3$ | N | |
| H | H | $S(CH_2)_2SO_2CH_2CH_3$ | $CH_3$ | $OCH_3$ | CH | |
| H | H | $S(CH_2)_2SO_2CH_2CH_3$ | $OCH_3$ | $OCH_3$ | CH | |
| H | H | $SCH_2CO_2CH_3$ | $CH_3$ | $OCH_3$ | CH | |
| H | H | $SCH_2CO_2CH_3$ | $OCH_3$ | $OCH_3$ | CH | |
| H | H | $SCH_2CO_2CH_3$ | Cl | $OCH_3$ | CH | |
| H | H | $SCH_2CO_2CH_3$ | $CH_3$ | $OCH_3$ | N | |
| H | H | $S(CH_2)_3SO_2N(CH_3)_2$ | $OCH_3$ | $OCH_3$ | CH | |
| H | H | $S(CH_2)_2SO_2N(CH_2CH_3)_2$ | $CH_3$ | $OCH_3$ | CH | |
| H | H | $S(CH_2)_2SO_2N(CH_2CH_3)_2$ | $OCH_3$ | $OCH_3$ | CH | |
| H | H | $SCH_2C(O)CH_3$ | $OCH_3$ | $OCH_3$ | CH | |

Table II (Continued)

| R | $R_1$ | Q | X | Y | Z | m.p. (°C) |
|---|---|---|---|---|---|---|
| H | H | $SCH_2C(O)CH_3$ | $CH_3$ | $OCH_3$ | N | |
| H | H | $S(CH_2)_2CN$ | $CH_3$ | $CH_3$ | CH | |
| H | H | $S(CH_2)_2CN$ | $CH_3$ | $OCH_3$ | CH | |
| H | H | $S(CH_2)_2CN$ | $OCH_3$ | $OCH_3$ | CH | |
| H | H | $S(CH_2)_2CN$ | $CH_3$ | $OCH_3$ | N | |
| H | H | $SCH_2C(O)N(CH_3)_2$ | $CH_3$ | $OCH_3$ | CH | |
| H | H | $SCH_2C(O)N(CH_3)_2$ | $OCH_3$ | $OCH_3$ | CH | |
| H | H | $SCH_2C(O)N(CH_3)_2$ | $CH_3$ | $OCH_3$ | N | |
| H | 4-$CH_3$ | $S(CH_2)_2C(O)N(CH_3)_2$ | $CH_3$ | $OCH_3$ | CH | |
| H | 4-$CH_3$ | $S(CH_2)_2C(O)N(CH_3)_2$ | $OCH_3$ | $OCH_3$ | CH | |
| H | H | $S(CH_2)_2Cl$ | $CH_3$ | $CH_3$ | CH | |
| H | H | $S(CH_2)_2Cl$ | $CH_3$ | $OCH_3$ | CH | |
| H | H | $S(CH_2)_2Cl$ | $OCH_3$ | $OCH_3$ | CH | |
| H | H | $S(CH_2)_2Cl$ | Cl | $OCH_3$ | CH | |
| H | H | $S(CH_2)_2Cl$ | $CH_3$ | $OCH_3$ | N | |
| H | H | $S(CH_2)_2Cl$ | $OCH_3$ | $OCH_3$ | N | |
| H | H | $S(CH_2)_3Cl$ | $CH_3$ | $OCH_3$ | CH | |
| H | H | $S(CH_2)_3Cl$ | $OCH_3$ | $OCH_3$ | CH | |
| H | H | $S(CH_2)_3Cl$ | $CH_3$ | $OCH_3$ | N | |
| H | H | $S(CH_2)_2F$ | $OCH_3$ | $OCH_3$ | CH | |
| H | H | $S(CH_2)_2F$ | $CH_3$ | $OCH_3$ | N | |
| H | H | $S(CH_2)_2Br$ | $CH_3$ | $CH_3$ | CH | |
| H | H | $S(CH_2)_2Br$ | $OCH_3$ | $OCH_3$ | CH | |
| H | 5-Cl | $S-CHClCH=CH_2$ | $CH_3$ | $OCH_3$ | CH | |
| H | 5-Cl | $S-CHClCH=CH_2$ | $OCH_3$ | $OCH_3$ | CH | |
| H | 5-Cl | $S-CHClCH=CH_2$ | $CH_3$ | $OCH_3$ | N | |
| H | H | $SCH_2C{\equiv}CH$ | $CH_3$ | $OCH_3$ | CH | |
| H | H | $SCH_2C{\equiv}CH$ | $OCH_3$ | $OCH_3$ | CH | |
| H | H | $SCH_2C{\equiv}CH$ | $CH_3$ | $OCH_3$ | N | |
| H | H | $S(CH_2)_2C{\equiv}CH$ | $CH_3$ | $OCH_3$ | CH | |
| H | H | $S(CH_2)_2C{\equiv}CH$ | $OCH_3$ | $OCH_3$ | CH | |
| H | H | $S(CH_2)_2C{\equiv}CH$ | $CH_3$ | $OCH_3$ | N | |

Table II (Continued)

| R | $R_1$ | Q | X | Y | Z | m.p. (°C) |
|---|---|---|---|---|---|---|
| H | 5-Cl | $SO_2(CH_2)_2OCH_3$ | $OCH_3$ | $OCH_3$ | CH | |
| H | 5-Cl | $SO_2(CH_2)_2OCH_3$ | $CH_3$ | $OCH_3$ | N | |
| H | H | $SO_2CH_2OCH_2CH_3$ | $OCH_3$ | $OCH_3$ | CH | |
| H | H | $SO_2CH_2OCH_2CH_3$ | $CH_3$ | $OCH_3$ | N | |
| H | H | $SO_2(CH_2)_2OCH_2CH_3$ | $OCH_3$ | $OCH_3$ | CH | |
| H | H | $SO_2(CH_2)_2OCH_2CH_3$ | $CH_3$ | $OCH_3$ | N | |
| H | H | $SO_2(CH_2)_2SCH_3$ | $CH_3$ | $OCH_3$ | CH | |
| H | H | $SO_2(CH_2)_2SCH_3$ | $OCH_3$ | $OCH_3$ | CH | |
| H | H | $SO_2(CH_2)_2SCH_3$ | $CH_3$ | $OCH_3$ | N | |
| H | H | $SO_2(CH_2)_2SCH_2CH_3$ | $OCH_3$ | $OCH_3$ | CH | |
| H | H | $SO_2(CH_2)_2SCH_2CH_3$ | $CH_3$ | $OCH_3$ | N | |
| H | H | $SO_2(CH_2)_2S(O)CH_3$ | $OCH_3$ | $OCH_3$ | CH | |
| H | H | $SO_2(CH_2)_2S(O)CH_3$ | $CH_3$ | $OCH_3$ | N | |
| H | H | $SO_2(CH_2)_2CO_2CH_3$ | $OCH_3$ | $OCH_3$ | CH | |
| H | H | $SO_2(CH_2)_2CO_2CH_3$ | $CH_3$ | $OCH_3$ | N | |
| H | H | $SO_2CH_2CO_2CH_2CH_3$ | $CH_3$ | $OCH_3$ | CH | |
| H | H | $SO_2CH_2CO_2CH_2CH_3$ | $OCH_3$ | $OCH_3$ | CH | |
| H | H | $SO_2CH_2CO_2CH_2CH_3$ | $CH_3$ | $OCH_3$ | N | |
| H | H | $SO_2(CH_2)_2C(O)CH_3$ | $CH_3$ | $OCH_3$ | CH | |
| H | H | $SO_2(CH_2)_2C(O)CH_3$ | $OCH_3$ | $OCH_3$ | CH | |
| H | H | $SO_2(CH_2)_2C(O)CH_3$ | $CH_3$ | $OCH_3$ | N | |
| H | H | $SO_2(CH_2)_2C(O)N(CH_3)_2$ | $OCH_3$ | $OCH_3$ | CH | |
| H | H | $SO_2(CH_2)_2C(O)N(CH_3)_2$ | $CH_3$ | $OCH_3$ | N | |
| H | H | $SO_2(CH_2)_2Br$ | $OCH_3$ | $OCH_3$ | CH | |
| H | H | $SO_2(CH_2)_2Br$ | $CH_3$ | $OCH_3$ | N | |
| H | H | $SO_2CH_2F$ | $CH_3$ | $CH_3$ | CH | |
| H | H | $SO_2CH_2F$ | Cl | $OCH_3$ | CH | |
| H | H | $SO_2CHClCH{=}CH_2$ | $OCH_3$ | $OCH_3$ | CH | |
| H | H | $SO_2CHClCH{=}CH_2$ | $CH_3$ | $OCH_3$ | N | |
| H | 5-$CH_3$ | $SO_2CH_2C{\equiv}CH$ | $OCH_3$ | $OCH_3$ | CH | |
| H | 5-$CH_3$ | $SO_2CH_2C{\equiv}CH$ | $CH_3$ | $OCH_3$ | N | |
| H | H | $SO_2(CH_2)_2C{\equiv}CH$ | $CH_3$ | $OCH_3$ | CH | |

Table II (Continued)

| R | $R_1$ | Q | X | Y | Z | m.p. (°C) |
|---|---|---|---|---|---|---|
| H | H | $SO_2(CH_2)_2C{\equiv}CH$ | $OCH_3$ | $OCH_3$ | CH | |
| H | H | $SO_2(CH_2)_2C{\equiv}CH$ | $CH_3$ | $OCH_3$ | N | |
| H | H | $CH_2P(O)(OCH_3)_2$ | $OCH_3$ | $OCH_3$ | CH | |
| H | H | $CH_2P(O)(OCH_3)_2$ | $CH_3$ | $OCH_3$ | N | |
| H | H | $P(O)(OCH_3)_2$ | $OCH_3$ | $OCH_3$ | CH | |
| H | H | $P(O)(OCH_3)_2$ | $CH_3$ | $OCH_3$ | N | |
| H | H | $P(O)(SCH_3)_2$ | $OCH_3$ | $OCH_3$ | CH | |
| H | H | $P(O)(SCH_3)_2$ | $CH_3$ | $OCH_3$ | N | |
| H | 5-Cl | $CH_2OH$ | $CH_3$ | $CH_3$ | CH | |
| H | 5-Cl | $CH_2OH$ | $CH_3$ | $OCH_3$ | CH | |
| H | 5-Cl | $CH_2OH$ | $OCH_3$ | $OCH_3$ | CH | |
| H | 5-Cl | $CH_2OH$ | Cl | $OCH_3$ | CH | |
| H | 5-Cl | $CH_2OH$ | $CH_3$ | $OCH_3$ | N | |
| H | 5-Cl | $CH_2OH$ | $OCH_3$ | $OCH_3$ | N | |
| H | H | $(CH_2)_2OH$ | $CH_3$ | $CH_3$ | CH | |
| H | H | $(CH_2)_2OH$ | $CH_3$ | $OCH_3$ | CH | |
| H | H | $(CH_2)_2OH$ | $OCH_3$ | $OCH_3$ | CH | |
| H | H | $(CH_2)_2OH$ | Cl | $OCH_3$ | CH | |
| H | H | $(CH_2)_2OH$ | $CH_3$ | $OCH_3$ | N | |
| H | H | $(CH_2)_2OH$ | $OCH_3$ | $OCH_3$ | N | |
| H | H | $CH_2OCH_3$ | $CH_3$ | $CH_3$ | CH | 175-177 |
| H | H | $CH_2OCH_3$ | $CH_3$ | $OCH_3$ | CH | |
| H | H | $CH_2OCH_3$ | $OCH_3$ | $OCH_3$ | CH | 171-173 |
| H | H | $CH_2OCH_3$ | Cl | $OCH_3$ | CH | 154-155 |
| H | H | $CH_2OCH_3$ | $CH_3$ | $OCH_3$ | N | 155-156 |
| H | H | $CH_2OCH_3$ | $OCH_3$ | $OCH_3$ | N | 158 |
| H | H | $(CH_2)_2OCH_3$ | $CH_3$ | $CH_3$ | CH | |
| H | H | $(CH_2)_2OCH_3$ | $CH_3$ | $OCH_3$ | CH | |
| H | H | $(CH_2)_2OCH_3$ | $OCH_3$ | $OCH_3$ | CH | |
| H | H | $(CH_2)_2OCH_3$ | Cl | $OCH_3$ | CH | |
| H | H | $(CH_2)_2OCH_3$ | $CH_3$ | $OCH_3$ | N | |
| H | H | $(CH_2)_2OCH_3$ | $OCH_3$ | $OCH_3$ | N | |

Table II (Continued)

| R | $R_1$ | Q | X | Y | Z | m.p. (°C) |
|---|---|---|---|---|---|---|
| H | H | $CH_2OCH_2CH_3$ | $CH_3$ | $CH_3$ | CH | |
| H | H | $CH_2OCH_2CH_3$ | $CH_3$ | $OCH_3$ | CH | |
| H | H | $CH_2OCH_2CH_3$ | $OCH_3$ | $OCH_3$ | CH | 143-146 |
| H | H | $CH_2OCH_2CH_3$ | Cl | $OCH_3$ | N | |
| H | H | $CH_2OCH_2CH_3$ | $OCH_3$ | $OCH_3$ | N | |
| H | H | $CH_2O(CH_2)_2CN$ | $CH_3$ | $CH_3$ | CH | |
| H | H | $CH_2O(CH_2)_2CN$ | $CH_3$ | $OCH_3$ | CH | |
| H | H | $CH_2O(CH_2)_2CN$ | $OCH_3$ | $OCH_3$ | CH | |
| H | H | $CH_2O(CH_2)_2CN$ | Cl | $OCH_3$ | CH | |
| H | H | $CH_2O(CH_2)_2CN$ | $CH_3$ | $OCH_3$ | N | |
| H | H | $CH_2O(CH_2)_2CN$ | $OCH_3$ | $OCH_3$ | N | |
| H | H | $CH_2SCH_3$ | $CH_3$ | $CH_3$ | CH | |
| H | H | $CH_2SCH_3$ | $CH_3$ | $OCH_3$ | CH | |
| H | H | $CH_2SCH_3$ | $OCH_3$ | $OCH_3$ | CH | 155-159(d) |
| H | H | $CH_2SCH_3$ | Cl | $OCH_3$ | CH | |
| H | H | $CH_2SCH_3$ | $CH_3$ | $OCH_3$ | N | |
| H | H | $CH_2SCH_3$ | $OCH_3$ | $OCH_3$ | N | |
| H | H | $(CH_2)_2SCH_3$ | $CH_3$ | $CH_3$ | CH | |
| H | H | $(CH_2)_2SCH_3$ | $CH_3$ | $OCH_3$ | CH | |
| H | H | $(CH_2)_2SCH_3$ | $OCH_3$ | $OCH_3$ | CH | |
| H | H | $(CH_2)_2SCH_3$ | Cl | $OCH_3$ | CH | |
| H | H | $(CH_2)_2SCH_3$ | $CH_3$ | $OCH_3$ | N | |
| H | H | $(CH_2)_2SCH_3$ | $OCH_3$ | $OCH_3$ | N | |
| H | H | $(CH_2)_3SCH_3$ | $CH_3$ | $CH_3$ | CH | |
| H | H | $(CH_2)_3SCH_3$ | $CH_3$ | $OCH_3$ | CH | |
| H | H | $(CH_2)_3SCH_3$ | $OCH_3$ | $OCH_3$ | CH | |
| H | H | $(CH_2)_3SCH_3$ | Cl | $OCH_3$ | CH | |
| H | H | $(CH_2)_3SCH_3$ | $CH_3$ | $OCH_3$ | N | |
| H | H | $(CH_2)_3SCH_3$ | $OCH_3$ | $OCH_3$ | N | |
| H | H | $CH_2S(O)CH_3$ | $CH_3$ | $CH_3$ | CH | |
| H | H | $CH_2S(O)CH_3$ | $CH_3$ | $OCH_3$ | CH | |
| H | H | $CH_2S(O)CH_3$ | $OCH_3$ | $OCH_3$ | CH | |

Table II (Continued)

| R | $R_1$ | Q | X | Y | Z | m.p. (°C) |
|---|---|---|---|---|---|---|
| H | H | $CH_2S(O)CH_3$ | Cl | $OCH_3$ | CH | |
| H | H | $CH_2S(O)CH_3$ | $CH_3$ | $OCH_3$ | N | |
| H | H | $CH_2S(O)CH_3$ | $OCH_3$ | $OCH_3$ | N | |
| H | H | $(CH_2)_2S(O)CH_3$ | $CH_3$ | $CH_3$ | CH | |
| H | H | $(CH_2)_2S(O)CH_3$ | $CH_3$ | $OCH_3$ | CH | |
| H | H | $(CH_2)_2S(O)CH_3$ | $OCH_3$ | $OCH_3$ | CH | |
| H | H | $(CH_2)_2S(O)CH_3$ | Cl | $OCH_3$ | CH | |
| H | H | $(CH_2)_2S(O)CH_3$ | $CH_3$ | $OCH_3$ | N | |
| H | H | $(CH_2)_2S(O)CH_3$ | $OCH_3$ | $OCH_3$ | N | |
| H | H | $(CH_2)_2S(O)CH_2CH_3$ | $CH_3$ | $CH_3$ | CH | |
| H | H | $(CH_2)_2S(O)CH_2CH_3$ | $CH_3$ | $OCH_3$ | CH | |
| H | H | $(CH_2)_2S(O)CH_2CH_3$ | $OCH_3$ | $OCH_3$ | CH | |
| H | H | $(CH_2)_2S(O)CH_2CH_3$ | Cl | $OCH_3$ | CH | |
| H | H | $(CH_2)_2S(O)CH_2CH_3$ | $CH_3$ | $OCH_3$ | N | |
| H | H | $(CH_2)_2S(O)CH_2CH_3$ | $OCH_3$ | $OCH_3$ | N | |
| H | 4-$CH_3$ | $CH_2SO_2CH_3$ | $CH_3$ | $CH_3$ | CH | |
| H | 4-$CH_3$ | $CH_2SO_2CH_3$ | $CH_3$ | $OCH_3$ | CH | |
| H | 4-$CH_3$ | $CH_2SO_2CH_3$ | $OCH_3$ | $OCH_3$ | CH | |
| H | 4-$CH_3$ | $CH_2SO_2CH_3$ | $CH_3$ | $OCH_3$ | N | |
| H | H | $(CH_2)_2SO_2CH_3$ | $CH_3$ | $CH_3$ | CH | |
| H | H | $(CH_2)_2SO_2CH_3$ | $CH_3$ | $OCH_3$ | CH | |
| H | H | $(CH_2)_2SO_2CH_3$ | $OCH_3$ | $OCH_3$ | CH | |
| H | H | $(CH_2)_2SO_2CH_3$ | Cl | $OCH_3$ | CH | |
| H | H | $(CH_2)_2SO_2CH_3$ | $CH_3$ | $OCH_3$ | N | |
| H | H | $(CH_2)_2SO_2CH_3$ | $OCH_3$ | $OCH_3$ | N | |
| H | H | $(CH_2)_3SO_2CH_3$ | $OCH_3$ | $OCH_3$ | CH | |
| H | H | $(CH_2)_3SO_2CH_3$ | $CH_3$ | $OCH_3$ | N | |
| H | H | $CH_2SO_2CH_2CH_3$ | $CH_3$ | $OCH_3$ | CH | |
| H | H | $CH_2SO_2CH_2CH_3$ | $OCH_3$ | $OCH_3$ | CH | |
| H | H | $CH_2SO_2CH_2CH_3$ | Cl | $OCH_3$ | CH | |
| H | H | $CH_2SO_2CH_2CH_3$ | $CH_3$ | $OCH_3$ | N | |
| H | H | $CH_2CO_2CH_3$ | $CH_3$ | $CH_3$ | CH | |

Table II (Continued)

| R | $R_1$ | Q | X | Y | Z | m.p. (°C) |
|---|---|---|---|---|---|---|
| H | H | $CH_2CO_2CH_3$ | $CH_3$ | $OCH_3$ | CH | |
| H | H | $CH_2CO_2CH_3$ | $OCH_3$ | $OCH_3$ | CH | |
| H | H | $CH_2CO_2CH_3$ | Cl | $OCH_3$ | CH | |
| H | H | $CH_2CO_2CH_3$ | $CH_3$ | $OCH_3$ | N | |
| H | H | $CH_2CO_2CH_2CH_3$ | $CH_3$ | $CH_3$ | CH | |
| H | H | $CH_2CO_2CH_2CH_3$ | $CH_3$ | $OCH_3$ | CH | |
| H | H | $CH_2CO_2CH_2CH_3$ | $OCH_3$ | $OCH_3$ | CH | |
| H | H | $CH_2CO_2CH_2CH_3$ | Cl | $OCH_3$ | CH | |
| H | H | $CH_2CO_2CH_2CH_3$ | $CH_3$ | $OCH_3$ | N | |
| H | H | $CH_2SO_2N(CH_3)_2$ | $CH_3$ | $CH_3$ | CH | |
| H | H | $CH_2SO_2N(CH_3)_2$ | $CH_3$ | $OCH_3$ | CH | |
| H | H | $CH_2SO_2N(CH_3)_2$ | $OCH_3$ | $OCH_3$ | CH | |
| H | H | $CH_2SO_2N(CH_3)_2$ | Cl | $OCH_3$ | CH | |
| H | H | $CH_2SO_2N(CH_3)_2$ | $CH_3$ | $OCH_3$ | N | |
| H | H | $CH_2SO_2N(CH_3)_2$ | $OCH_3$ | $OCH_3$ | N | |
| H | 5-Cl | $(CH_2)_2SO_2N(CH_3)_2$ | $CH_3$ | $OCH_3$ | CH | |
| H | 5-Cl | $(CH_2)_2SO_2N(CH_3)_2$ | $OCH_3$ | $OCH_3$ | CH | |
| H | 5-Cl | $(CH_2)_2SO_2N(CH_3)_2$ | $CH_3$ | $OCH_3$ | N | |
| H | H | $CH_2SO_2N(CH_2CH_3)_2$ | $CH_3$ | $OCH_3$ | CH | |
| H | H | $CH_2SO_2N(CH_2CH_3)_2$ | $OCH_3$ | $OCH_3$ | CH | |
| H | H | $CH_2SO_2N(CH_2CH_3)_2$ | $CH_3$ | $OCH_3$ | N | |
| H | H | $CH_2C(O)CH_3$ | $CH_3$ | $OCH_3$ | CH | |
| H | H | $CH_2C(O)CH_3$ | $OCH_3$ | $OCH_3$ | CH | |
| H | H | $CH_2C(O)CH_3$ | $CH_3$ | $OCH_3$ | N | |
| H | 5-Cl | $(CH_2)_2C(O)CH_3$ | $CH_3$ | $OCH_3$ | CH | |
| H | 5-Cl | $(CH_2)_2C(O)CH_3$ | $OCH_3$ | $OCH_3$ | CH | |
| H | 5-Cl | $(CH_2)_2C(O)CH_3$ | $CH_3$ | $OCH_3$ | N | |
| H | H | $CH_2CN$ | $CH_3$ | $CH_3$ | CH | |
| H | H | $CH_2CN$ | $CH_3$ | $OCH_3$ | CH | |
| H | H | $CH_2CN$ | $OCH_3$ | $OCH_3$ | CH | |
| H | H | $CH_2CN$ | Cl | $OCH_3$ | CH | |
| H | H | $CH_2CN$ | $CH_3$ | $OCH_3$ | N | |

Table II (Continued)

| R | $R_1$ | Q | X | Y | Z | m.p. (°C) |
|---|---|---|---|---|---|---|
| H | H | $CH_2CN$ | $OCH_3$ | $OCH_3$ | N | 105-110 |
| H | H | $(CH_2)_2CN$ | $CH_3$ | $CH_3$ | CH | |
| H | H | $(CH_2)_2CN$ | $CH_3$ | $OCH_3$ | CH | |
| H | H | $(CH_2)_2CN$ | $OCH_3$ | $OCH_3$ | CH | |
| H | H | $(CH_2)_2CN$ | Cl | $OCH_3$ | CH | |
| H | H | $(CH_2)_2CN$ | $CH_3$ | $OCH_3$ | N | |
| H | H | $(CH_2)_2CN$ | $OCH_3$ | $OCH_3$ | N | |
| H | H | $CH_2C(O)N(CH_3)_2$ | $CH_3$ | $OCH_3$ | CH | |
| H | H | $CH_2C(O)N(CH_3)_2$ | $OCH_3$ | $OCH_3$ | CH | |
| H | H | $CH_2C(O)N(CH_3)_2$ | $CH_3$ | $OCH_3$ | N | |
| H | H | $(CH_2)_2C(O)N(CH_3)_2$ | $CH_3$ | $OCH_3$ | CH | |
| H | H | $(CH_2)_2C(O)N(CH_3)_2$ | $OCH_3$ | $OCH_3$ | CH | |
| H | H | $(CH_2)_2C(O)N(CH_3)_2$ | $CH_3$ | $OCH_3$ | N | |
| H | H | $CH_2Cl$ | $CH_3$ | $CH_3$ | CH | 186-187 |
| H | H | $CH_2Cl$ | $CH_3$ | $OCH_3$ | CH | 161.5-163 |
| H | H | $CH_2Cl$ | $OCH_3$ | $OCH_3$ | CH | 175-177 |
| H | H | $CH_2Cl$ | Cl | $OCH_3$ | CH | 151-156 |
| H | H | $CH_2Cl$ | $CH_3$ | $OCH_3$ | N | 157-159 |
| H | H | $CH_2Cl$ | $OCH_3$ | $OCH_3$ | N | 160-162 |
| H | H | $CH_2Cl$ | $CH_3$ | $CH_3$ | N | 179-181 |
| H | H | $(CH_2)_2Cl$ | $CH_3$ | $CH_3$ | CH | |
| H | H | $(CH_2)_2Cl$ | $CH_3$ | $OCH_3$ | CH | |
| H | H | $(CH_2)_2Cl$ | $OCH_3$ | $OCH_3$ | CH | |
| H | H | $(CH_2)_2Cl$ | Cl | $OCH_3$ | CH | |
| H | H | $(CH_2)_2Cl$ | $CH_3$ | $OCH_3$ | N | |
| H | H | $(CH_2)_2Cl$ | $OCH_3$ | $OCH_3$ | N | |
| H | H | $CH_2F$ | $CH_3$ | $CH_3$ | CH | |
| H | H | $CH_2F$ | $CH_3$ | $OCH_3$ | CH | |
| H | H | $CH_2F$ | $OCH_3$ | $OCH_3$ | CH | |
| H | H | $CH_2F$ | Cl | $OCH_3$ | CH | |
| H | H | $CH_2F$ | $CH_3$ | $OCH_3$ | N | |
| H | H | $CH_2F$ | $OCH_3$ | $OCH_3$ | N | |

Table II (Continued)

| R | $R_1$ | Q | X | Y | Z | m.p. (°C) |
|---|---|---|---|---|---|---|
| H | H | $(CH_2)_2F$ | $CH_3$ | $OCH_3$ | CH | |
| H | H | $(CH_2)_2F$ | $OCH_3$ | $OCH_3$ | CH | |
| H | H | $(CH_2)_2F$ | $CH_3$ | $OCH_3$ | N | |
| H | H | $CH_2Br$ | $CH_3$ | $CH_3$ | CH | |
| H | H | $CH_2Br$ | $CH_3$ | $OCH_3$ | CH | |
| H | H | $CH_2Br$ | $OCH_3$ | $OCH_3$ | CH | |
| H | H | $CH_2Br$ | Cl | $OCH_3$ | CH | |
| H | H | $CH_2Br$ | $CH_3$ | $OCH_3$ | N | |
| H | H | $CH_2Br$ | $OCH_3$ | $OCH_3$ | N | |
| H | H | $(CH_2)_2Br$ | $CH_3$ | $OCH_3$ | CH | |
| H | H | $(CH_2)_2Br$ | $OCH_3$ | $OCH_3$ | CH | |
| H | H | $(CH_2)_2Br$ | Cl | $OCH_3$ | CH | |
| H | H | $CH_2SCH_3$ | $OCH_2CH_3$ | $C_2H_5$ | CH | |
| H | H | $CH_2SCH_3$ | $OCH_2CH_3$ | $C_2H_5$ | N | |
| H | H | $CH_2SCH_3$ | $OCH_2CH_3$ | $CH_2OCH_3$ | CH | |
| H | H | $CH_2SCH_3$ | $OCH_2CH_3$ | $CH_2OCH_3$ | N | |
| H | H | $CH_2SCH_3$ | $OCH_2CH_3$ | $NHCH_3$ | N | |
| H | H | $CH_2SCH_3$ | $OCH_2CH_3$ | $CH(OCH_3)_2$ | CH | |
| H | H | $CH_2SCH_3$ | $OCF_2H$ | $C_2H_5$ | CH | |
| H | H | $CH_2SCH_3$ | $OCF_2H$ | $CH_2OCH_3$ | CH | |
| H | H | $CH_2SCH_3$ | $OCH_2CF_3$ | $C_2H_5$ | CH | |
| H | H | $CH_2SCH_3$ | $OCH_2CF_3$ | $C_2H_5$ | N | |
| H | H | $CH_2SCH_3$ | $OCH_2CF_3$ | $CH_2OCH_3$ | CH | |
| H | H | $CH_2SCH_3$ | $OCH_2CF_3$ | $CH_2OCH_3$ | N | |
| H | H | $CH_2SCH_3$ | $OCH_2CF_3$ | $NHCH_3$ | N | |
| H | H | $CH_2SCH_3$ | $OCH_2CF_3$ | $CH(OCH_3)_2$ | N | |
| H | H | $CH_2SCH_3$ | $CH_3$ | $C_2H_5$ | N | |
| H | H | $CH_2SCH_3$ | $CH_3$ | $CH_2OCH_3$ | CH | |
| H | H | $CH_2SCH_3$ | $CH_3$ | $CH_2OCH_3$ | N | |
| H | H | $CH_2SCH_3$ | $CH_3$ | $NHCH_3$ | N | |
| H | H | $CH_2SCH_3$ | $CH_3$ | $CH(OCH_3)_2$ | N | |
| H | H | $CH_2SCH_3$ | $OCH_3$ | $C_2H_5$ | CH | |

Table II (Continued)

| R | $R_1$ | Q | X | Y | Z | m.p. (°C) |
|---|---|---|---|---|---|---|
| H | H | $CH_2SCH_3$ | $OCH_3$ | $C_2H_5$ | N | |
| H | H | $CH_2SCH_3$ | $OCH_3$ | $CH_2OCH_3$ | CH | |
| H | H | $CH_2SCH_3$ | $OCH_3$ | $CH_2OCH_3$ | N | |
| H | H | $CH_2SCH_3$ | $OCH_3$ | $NHCH_3$ | N | |
| H | H | $CH_2SCH_3$ | $OCH_3$ | $CH(OCH_3)_2$ | CH | |
| H | H | $CH_2SCH_3$ | $OCH_3$ | $CH(OCH_3)_2$ | N | |
| H | 5-Cl | $CH_2OCH_3$ | $OCH_2CH_3$ | $C_2H_5$ | CH | |
| H | 5-Cl | $CH_2OCH_3$ | $OCH_2CH_3$ | $C_2H_5$ | N | |
| H | 5-Cl | $CH_2OCH_3$ | $OCH_2CH_3$ | $CH_2OCH_3$ | CH | |
| H | 5-Cl | $CH_2OCH_3$ | $OCH_2CH_3$ | $CH_2OCH_3$ | N | |
| H | 5-Cl | $CH_2OCH_3$ | $OCH_2CH_3$ | $NHCH_3$ | CH | |
| H | 5-Cl | $CH_2OCH_3$ | $OCH_2CH_3$ | $NHCH_3$ | N | |
| H | 5-Cl | $CH_2OCH_3$ | $OCH_2CH_3$ | $CH(OCH_3)_2$ | CH | |
| H | 5-Cl | $CH_2OCH_3$ | $OCH_2CH_3$ | $CH(OCH_3)_2$ | N | |
| H | 5-Cl | $CH_2OCH_3$ | $OCF_2H$ | $C_2H_5$ | CH | |
| H | 5-Cl | $CH_2OCH_3$ | $OCF_2H$ | $CH_2OCH_3$ | CH | |
| H | 5-Cl | $CH_2OCH_3$ | $OCF_2H$ | $NHCH_3$ | CH | |
| H | 5-Cl | $CH_2OCH_3$ | $OCH_2CF_3$ | $C_2H_5$ | CH | |
| H | 5-Cl | $CH_2OCH_3$ | $OCH_2CF_3$ | $CH_2OCH_3$ | N | |
| H | 5-Cl | $CH_2OCH_3$ | $OCH_2CF_3$ | $NHCH_3$ | CH | |
| H | 5-Cl | $CH_2OCH_3$ | $OCH_2CF_3$ | $NHCH_3$ | N | |
| H | 5-Cl | $CH_2OCH_3$ | $OCH_2CF_3$ | $CH(OCH_3)_2$ | CH | |
| H | 5-Cl | $CH_2OCH_3$ | $OCH_2CF_3$ | $CH(OCH_3)_2$ | N | |
| H | 5-Cl | $CH_2OCH_3$ | Cl | $NHCH_3$ | CH | |
| H | 5-Cl | $CH_2OCH_3$ | $CH_3$ | $C_2H_5$ | CH | |
| H | 5-Cl | $CH_2OCH_3$ | $CH_3$ | $CH_2OCH_3$ | CH | |
| H | 5-Cl | $CH_2OCH_3$ | $CH_3$ | $CH_2OCH_3$ | N | |
| H | 5-Cl | $CH_2OCH_3$ | Cl | $NHCH_3$ | CH | |
| H | 5-Cl | $CH_2OCH_3$ | $CH_3$ | $CH_2OCH_3$ | CH | |
| H | 5-Cl | $CH_2OCH_3$ | $CH_3$ | $CH_2OCH_3$ | N | |
| H | 5-Cl | $CH_2OCH_3$ | $CH_3$ | $NHCH_3$ | N | |
| H | 5-Cl | $CH_2OCH_3$ | $CH_3$ | $CH(OCH_3)_2$ | CH | |

Table II (Continued)

| R | $R_1$ | Q | X | Y | Z | m.p. (°C) |
|---|---|---|---|---|---|---|
| H | 5-Cl | $CH_2OCH_3$ | $CH_3$ | $CH(OCH_3)_2$ | N | |
| H | 5-Cl | $CH_2OCH_3$ | $OCH_3$ | $C_2H_5$ | CH | |
| H | 5-Cl | $CH_2OCH_3$ | $OCH_3$ | $C_2H_5$ | N | |
| H | 5-Cl | $CH_2OCH_3$ | $OCH_3$ | $CH_2OCH_3$ | CH | |
| H | 5-Cl | $CH_2OCH_3$ | $OCH_3$ | $CH_2OCH_3$ | N | |
| H | 5-Cl | $CH_2OCH_3$ | $OCH_3$ | $NHCH_3$ | CH | |
| H | 5-Cl | $CH_2OCH_3$ | $OCH_3$ | $NHCH_3$ | N | |
| H | 5-Cl | $CH_2OCH_3$ | $OCH_3$ | $CH(OCH_3)_2$ | CH | |
| H | 5-Cl | $CH_2OCH_3$ | $OCH_3$ | $CH(OCH_3)_2$ | N | |
| H | H | CN | $CH_3$ | $CH_3$ | CH | |
| H | H | CN | $CH_3$ | $OCH_3$ | CH | |
| H | H | CN | $OCH_3$ | $OCH_3$ | CH | |
| H | H | CN | Cl | $OCH_3$ | CH | |
| H | H | CN | $CH_3$ | $OCH_3$ | N | |
| H | H | CN | $OCH_3$ | $OCH_3$ | N | |
| $CH_3$ | H | CN | $CH_3$ | $OCH_3$ | N | |
| $CH_3$ | H | $CH_2OCH_3$ | $CH_3$ | $OCH_3$ | N | |
| $CH_3$ | H | $(CH_2)_2OCH_3$ | $CH_3$ | $OCH_3$ | N | |
| H | H | $CH_2OCH_3$ | $OCH_3$ | $SCF_2H$ | CH | |
| H | H | $CH_2OCH_3$ | $OCH_3$ | $SCH_3$ | CH | |
| H | H | $CH_2OCH_3$ | $OCH_3$ | $OCH_2C{\equiv}CH$ | CH | |
| H | H | $CH_2OCH_3$ | $OCH_3$ | $OCH_2CH{=}CH_2$ | CH | |
| H | H | $CH_2OCH_3$ | $OCH_3$ | $CH_2SCH_3$ | CH | |
| H | H | $CH_2OCH_3$ | $OCH_3$ | CN | CH | |

Table III

General Formula III

| R | $R_1$ | Q | X | Y | Z | m.p. (°C) |
|---|---|---|---|---|---|---|
| H | H | $S(CH_2)_2OCH_3$ | $CH_3$ | $OCH_3$ | CH | |
| H | H | $S(CH_2)_2OCH_3$ | $OCH_3$ | $OCH_3$ | CH | |
| H | H | $S(CH_2)_2OCH_3$ | Cl | $OCH_3$ | CH | |
| H | H | $S(CH_2)_2OCH_3$ | $CH_3$ | $OCH_3$ | N | |
| H | H | $S(CH_2)_3OCH_3$ | $CH_3$ | $OCH_3$ | CH | |
| H | H | $S(CH_2)_3OCH_3$ | $OCH_3$ | $OCH_3$ | CH | |
| H | H | $S(CH_2)_2OCH_2CN$ | $CH_3$ | $OCH_3$ | CH | |
| H | H | $S(CH_2)_2OCH_2CN$ | Cl | $OCH_3$ | CH | |
| H | H | $S(CH_2)_2OCH_2CN$ | $OCH_3$ | $OCH_3$ | N | |
| H | H | $S(CH_2)_2SCH_3$ | $CH_3$ | $OCH_3$ | CH | |
| H | H | $S(CH_2)_2SCH_3$ | $OCH_3$ | $OCH_3$ | CH | |
| H | H | $S(CH_2)_2SCH_3$ | $CH_3$ | $OCH_3$ | N | |
| H | H | $S(CH_2)_2S(O)CH_3$ | $CH_3$ | $OCH_3$ | CH | |
| H | H | $S(CH_2)_2S(O)CH_3$ | $OCH_3$ | $OCH_3$ | CH | |
| H | H | $S(CH_2)_2S(O)CH_3$ | Cl | $OCH_3$ | CH | |
| H | H | $S(CH_2)_2S(O)CH_3$ | $CH_3$ | $OCH_3$ | N | |
| H | H | $S(CH_2)_2S(O)CH_2CH_3$ | $OCH_3$ | $OCH_3$ | CH | |
| H | H | $S(CH_2)_2S(O)CH_2CH_3$ | Cl | $OCH_3$ | CH | |
| H | H | $S(CH_2)_2SO_2CH_3$ | $OCH_3$ | $OCH_3$ | CH | |
| H | H | $S(CH_2)_2SO_2CH_3$ | Cl | $OCH_3$ | CH | |
| H | H | $S(CH_2)_2SO_2CH_3$ | $CH_3$ | $OCH_3$ | N | |
| H | H | $S(CH_2)_2SO_2CH_2CH_3$ | $CH_3$ | $OCH_3$ | CH | |
| H | H | $S(CH_2)_2SO_2CH_2CH_3$ | $OCH_3$ | $OCH_3$ | CH | |
| H | H | $S(CH_2)_2SO_2CH_2CH_3$ | $OCH_3$ | $OCH_3$ | N | |
| H | H | $SCH_2CO_2CH_3$ | $CH_3$ | $CH_3$ | CH | |
| H | H | $S(CH_2)_3SO_2N(CH_3)_2$ | $OCH_3$ | $OCH_3$ | CH | |
| H | H | $S(CH_2)_3SO_2N(CH_3)_2$ | $CH_3$ | $OCH_3$ | N | |
| H | H | $S(CH_2)_2SO_2N(CH_2CH_3)_2$ | $CH_3$ | $OCH_3$ | CH | |
| H | H | $S(CH_2)_2SO_2N(CH_2CH_3)_2$ | $OCH_3$ | $OCH_3$ | CH | |
| H | H | $SCH_2C(O)CH_3$ | $OCH_3$ | $OCH_3$ | CH | |
| H | H | $SCH_2C(O)CH_3$ | $CH_3$ | $OCH_3$ | N | |

Table II (Continued)

| R | $R_1$ | Q | X | Y | Z | m.p. (°C) |
|---|---|---|---|---|---|---|
| H | 5-Cl | $CH_2OCH_3$ | $CH_3$ | $CH(OCH_3)_2$ | N | |
| H | 5-Cl | $CH_2OCH_3$ | $OCH_3$ | $C_2H_5$ | CH | |
| H | 5-Cl | $CH_2OCH_3$ | $OCH_3$ | $C_2H_5$ | N | |
| H | 5-Cl | $CH_2OCH_3$ | $OCH_3$ | $CH_2OCH_3$ | CH | |
| H | 5-Cl | $CH_2OCH_3$ | $OCH_3$ | $CH_2OCH_3$ | N | |
| H | 5-Cl | $CH_2OCH_3$ | $OCH_3$ | $NHCH_3$ | CH | |
| H | 5-Cl | $CH_2OCH_3$ | $OCH_3$ | $NHCH_3$ | N | |
| H | 5-Cl | $CH_2OCH_3$ | $OCH_3$ | $CH(OCH_3)_2$ | CH | |
| H | 5-Cl | $CH_2OCH_3$ | $OCH_3$ | $CH(OCH_3)_2$ | N | |
| H | H | CN | $CH_3$ | $CH_3$ | CH | |
| H | H | CN | $CH_3$ | $OCH_3$ | CH | |
| H | H | CN | $OCH_3$ | $OCH_3$ | CH | |
| H | H | CN | Cl | $OCH_3$ | CH | |
| H | H | CN | $CH_3$ | $OCH_3$ | N | |
| H | H | CN | $OCH_3$ | $OCH_3$ | N | |
| $CH_3$ | H | CN | $CH_3$ | $OCH_3$ | N | |
| $CH_3$ | H | $CH_2OCH_3$ | $CH_3$ | $OCH_3$ | N | |
| $CH_3$ | H | $(CH_2)_2OCH_3$ | $CH_3$ | $OCH_3$ | N | |
| H | H | $CH_2OCH_3$ | $OCH_3$ | $SCF_2H$ | CH | |
| H | H | $CH_2OCH_3$ | $OCH_3$ | $SCH_3$ | CH | |
| H | H | $CH_2OCH_3$ | $OCH_3$ | $OCH_2C\equiv CH$ | CH | |
| H | H | $CH_2OCH_3$ | $OCH_3$ | $OCH_2CH=CH_2$ | CH | |
| H | H | $CH_2OCH_3$ | $OCH_3$ | $CH_2SCH_3$ | CH | |
| H | H | $CH_2OCH_3$ | $OCH_3$ | CN | CH | |

Table III (Continued)

| R | $R_1$ | Q | X | Y | Z | m.p. (°C) |
|---|---|---|---|---|---|---|
| H | H | $S(CH_2)_2C{\equiv}CH$ | $OCH_3$ | $OCH_3$ | CH | |
| H | H | $S(CH_2)_2C{\equiv}CH$ | $CH_3$ | $OCH_3$ | N | |
| H | 5-Cl | $SO_2(CH_2)_2OCH_3$ | $OCH_3$ | $OCH_3$ | CH | |
| H | 5-Cl | $SO_2(CH_2)_2OCH_3$ | $CH_3$ | $OCH_3$ | N | |
| H | H | $SO_2CH_2OCH_2CH_3$ | $OCH_3$ | $OCH_3$ | CH | |
| H | H | $SO_2CH_2OCH_2CH_3$ | $CH_3$ | $OCH_3$ | N | |
| H | H | $SO_2(CH_2)_2OCH_2CH_3$ | $CH_3$ | $CH_3$ | CH | |
| H | H | $SO_2(CH_2)_2OCH_2CH_3$ | $OCH_3$ | $OCH_3$ | CH | |
| H | H | $SO_2(CH_2)_2OCH_2CH_3$ | $CH_3$ | $OCH_3$ | N | |
| H | H | $SO_2(CH_2)_2SCH_3$ | $CH_3$ | $OCH_3$ | CH | |
| H | H | $SO_2(CH_2)_2SCH_3$ | $OCH_3$ | $OCH_3$ | CH | |
| H | H | $SO_2(CH_2)_2SCH_3$ | $CH_3$ | $OCH_3$ | N | |
| H | H | $SO_2(CH_2)_2SCH_2CH_3$ | $OCH_3$ | $OCH_3$ | CH | |
| H | H | $SO_2(CH_2)_2SCH_2CH_3$ | $CH_3$ | $OCH_3$ | N | |
| H | H | $SO_2(CH_2)_2S(O)CH_3$ | $OCH_3$ | $OCH_3$ | CH | |
| H | H | $SO_2(CH_2)_2S(O)CH_3$ | $CH_3$ | $OCH_3$ | N | |
| H | H | $SO_2(CH_2)_2CO_2CH_3$ | $CH_3$ | $OCH_3$ | CH | |
| H | H | $SO_2(CH_2)_2CO_2CH_3$ | $OCH_3$ | $OCH_3$ | CH | |
| H | H | $SO_2(CH_2)_2CO_2CH_3$ | $CH_3$ | $OCH_3$ | N | |
| H | H | $SO_2CH_2CO_2CH_2CH_3$ | $CH_3$ | $CH_3$ | CH | |
| H | H | $SO_2CH_2CO_2CH_2CH_3$ | $CH_3$ | $OCH_3$ | CH | |
| H | H | $SO_2CH_2CO_2CH_2CH_3$ | $OCH_3$ | $OCH_3$ | CH | |
| H | H | $SO_2CH_2CO_2CH_2CH_3$ | $CH_3$ | $OCH_3$ | N | |
| H | H | $SO_2(CH_2)_2C(O)CH_3$ | $CH_3$ | $OCH_3$ | CH | |
| H | H | $SO_2(CH_2)_2C(O)CH_3$ | $OCH_3$ | $OCH_3$ | CH | |
| H | H | $SO_2(CH_2)_2C(O)CH_3$ | $CH_3$ | $OCH_3$ | N | |
| H | H | $SO_2(CH_2)_2C(O)N(CH_3)_2$ | $OCH_3$ | $OCH_3$ | CH | |
| H | H | $SO_2(CH_2)_2C(O)N(CH_3)_2$ | $CH_3$ | $OCH_3$ | N | |
| H | H | $SO_2(CH_2)_2Br$ | $OCH_3$ | $OCH_3$ | CH | |
| H | H | $SO_2(CH_2)_2Br$ | $CH_3$ | $OCH_3$ | N | |
| H | H | $SO_2CH_2F$ | $CH_3$ | $CH_3$ | CH | |
| H | H | $SO_2CH_2F$ | $OCH_3$ | $OCH_3$ | CH | |

Table III (Continued)

| R | $R_1$ | Q | X | Y | Z | m.p. (°C) |
|---|---|---|---|---|---|---|
| H | H | $SO_2CH_2F$ | Cl | $OCH_3$ | CH | |
| H | H | $SO_2CH_2F$ | $CH_3$ | $OCH_3$ | N | |
| H | H | $SO_2CHClCH=CH_2$ | $OCH_3$ | $OCH_3$ | CH | |
| H | 2-$CH_3$ | $SO_2CH_2C{\equiv}CH$ | $OCH_3$ | $OCH_3$ | CH | |
| H | 2-$CH_3$ | $SO_2CH_2C{\equiv}CH$ | $CH_3$ | $OCH_3$ | N | |
| H | H | $SO_2(CH_2)_2C{\equiv}CH$ | $CH_3$ | $OCH_3$ | CH | |
| H | H | $SO_2(CH_2)_2C{\equiv}CH$ | $OCH_3$ | $OCH_3$ | CH | |
| H | H | $SO_2(CH_2)_2C{\equiv}CH$ | $CH_3$ | $OCH_3$ | N | |
| H | H | $CH_2P(O)(OCH_3)_2$ | $OCH_3$ | $OCH_3$ | CH | |
| H | H | $CH_2P(O)(OCH_3)_2$ | $CH_3$ | $OCH_3$ | N | |
| H | H | $CH_2P(O)(OCH_3)_2$ | $OCH_3$ | $OCH_3$ | N | |
| H | H | $P(O)(OCH_3)_2$ | Cl | $OCH_3$ | CH | |
| H | H | $P(O)(SCH_3)_2$ | $CH_3$ | $OCH_3$ | CH | |
| H | H | $P(O)(SCH_3)_2$ | Cl | $OCH_3$ | CH | |
| H | H | $P(O)(SCH_3)_2$ | $OCH_3$ | $OCH_3$ | N | |
| H | 2-Cl | $CH_2OH$ | $CH_3$ | $CH_3$ | CH | |
| H | 2-Cl | $CH_2OH$ | $CH_3$ | $OCH_3$ | CH | |
| H | 2-Cl | $CH_2OH$ | $OCH_3$ | $OCH_3$ | CH | |
| H | 2-Cl | $CH_2OH$ | Cl | $OCH_3$ | CH | |
| H | 2-Cl | $CH_2OH$ | $CH_3$ | $OCH_3$ | N | |
| H | 2-Cl | $CH_2OH$ | $OCH_3$ | $OCH_3$ | N | |
| H | H | $(CH_2)_2OH$ | $CH_3$ | $CH_3$ | CH | |
| H | H | $(CH_2)_2OH$ | $CH_3$ | $OCH_3$ | CH | |
| H | H | $(CH_2)_2OH$ | $OCH_3$ | $OCH_3$ | CH | |
| H | H | $(CH_2)_2OH$ | Cl | $OCH_3$ | CH | |
| H | H | $(CH_2)_2OH$ | $CH_3$ | $OCH_3$ | N | |
| H | H | $(CH_2)_2OH$ | $OCH_3$ | $OCH_3$ | N | |
| H | H | $CH_2OCH_3$ | $CH_3$ | $CH_3$ | CH | |
| H | H | $CH_2OCH_3$ | $CH_3$ | $OCH_3$ | CH | |
| H | H | $CH_2OCH_3$ | $OCH_3$ | $OCH_3$ | CH | |
| H | H | $CH_2OCH_3$ | Cl | $OCH_3$ | CH | |
| H | H | $CH_2OCH_3$ | $CH_3$ | $OCH_3$ | N | |

Table III (Continued)

| R | $R_1$ | Q | X | Y | Z | m.p. (°C) |
|---|---|---|---|---|---|---|
| H | H | $CH_2OCH_3$ | $OCH_3$ | $OCH_3$ | N | |
| H | H | $(CH_2)_2OCH_3$ | $CH_3$ | $CH_3$ | CH | |
| H | H | $(CH_2)_2OCH_3$ | $CH_3$ | $OCH_3$ | CH | |
| H | H | $(CH_2)_2OCH_3$ | $OCH_3$ | $OCH_3$ | CH | |
| H | H | $(CH_2)_2OCH_3$ | Cl | $OCH_3$ | CH | |
| H | H | $(CH_2)_2OCH_3$ | $CH_3$ | $OCH_3$ | N | |
| H | H | $(CH_2)_2OCH_3$ | $OCH_3$ | $OCH_3$ | N | |
| H | 5-$CH_3$ | $CH_2OCH_2CH_3$ | $CH_3$ | $CH_3$ | CH | |
| H | 5-$CH_3$ | $CH_2OCH_2CH_3$ | $CH_3$ | $OCH_3$ | CH | |
| H | 5-$CH_3$ | $CH_2OCH_2CH_3$ | $OCH_3$ | $OCH_3$ | CH | |
| H | 5-$CH_3$ | $CH_2OCH_2CH_3$ | Cl | $OCH_3$ | CH | |
| H | 5-$CH_3$ | $CH_2OCH_2CH_3$ | $OCH_3$ | $OCH_3$ | N | |
| H | H | $CH_2O(CH_2)_2CN$ | $CH_3$ | $CH_3$ | CH | |
| H | H | $CH_2O(CH_2)_2CN$ | $CH_3$ | $OCH_3$ | CH | |
| H | H | $CH_2O(CH_2)_2CN$ | $OCH_3$ | $OCH_3$ | CH | |
| H | H | $CH_2O(CH_2)_2CN$ | Cl | $OCH_3$ | CH | |
| H | H | $CH_2O(CH_2)_2CN$ | $CH_3$ | $OCH_3$ | N | |
| H | H | $CH_2O(CH_2)_2CN$ | $OCH_3$ | $OCH_3$ | N | |
| H | H | $CH_2SCH_3$ | $CH_3$ | $CH_3$ | CH | |
| H | H | $CH_2SCH_3$ | $CH_3$ | $OCH_3$ | CH | |
| H | H | $CH_2SCH_3$ | $OCH_3$ | $OCH_3$ | CH | |
| H | H | $CH_2SCH_3$ | Cl | $OCH_3$ | CH | |
| H | H | $CH_2SCH_3$ | $CH_3$ | $OCH_3$ | N | |
| H | H | $CH_2SCH_3$ | $OCH_3$ | $OCH_3$ | N | |
| H | H | $(CH_2)_2SCH_3$ | $CH_3$ | $CH_3$ | CH | |
| H | H | $(CH_2)_2SCH_3$ | $CH_3$ | $OCH_3$ | CH | |
| H | H | $(CH_2)_2SCH_3$ | $OCH_3$ | $OCH_3$ | CH | |
| H | H | $(CH_2)_2SCH_3$ | Cl | $OCH_3$ | CH | |
| H | H | $(CH_2)_2SCH_3$ | $CH_3$ | $OCH_3$ | N | |
| H | H | $(CH_2)_2SCH_3$ | $OCH_3$ | $OCH_3$ | N | |
| H | H | $(CH_2)_3SCH_3$ | $CH_3$ | $CH_3$ | CH | |
| H | H | $(CH_2)_3SCH_3$ | $CH_3$ | $OCH_3$ | CH | |

Table III (Continued)

| R | $R_1$ | Q | X | Y | Z | m.p. (°C) |
|---|---|---|---|---|---|---|
| H | H | $(CH_2)_3SCH_3$ | $OCH_3$ | $OCH_3$ | CH | |
| H | H | $(CH_2)_3SCH_3$ | Cl | $OCH_3$ | CH | |
| H | H | $(CH_2)_3SCH_3$ | $CH_3$ | $OCH_3$ | N | |
| H | H | $(CH_2)_3SCH_3$ | $OCH_3$ | $OCH_3$ | N | |
| H | H | $CH_2S(O)CH_3$ | $CH_3$ | $CH_3$ | CH | |
| H | H | $CH_2S(O)CH_3$ | $CH_3$ | $OCH_3$ | CH | |
| H | H | $CH_2S(O)CH_3$ | $OCH_3$ | $OCH_3$ | CH | |
| H | H | $CH_2S(O)CH_3$ | Cl | $OCH_3$ | CH | |
| H | H | $CH_2S(O)CH_3$ | $CH_3$ | $OCH_3$ | N | |
| H | H | $CH_2S(O)CH_3$ | $OCH_3$ | $OCH_3$ | N | |
| H | H | $(CH_2)_2S(O)CH_3$ | $CH_3$ | $CH_3$ | CH | |
| H | H | $(CH_2)_2S(O)CH_3$ | $CH_3$ | $OCH_3$ | CH | |
| H | H | $(CH_2)_2S(O)CH_3$ | $OCH_3$ | $OCH_3$ | CH | |
| H | H | $(CH_2)_2S(O)CH_3$ | Cl | $OCH_3$ | CH | |
| H | H | $(CH_2)_2S(O)CH_3$ | $CH_3$ | $OCH_3$ | N | |
| H | H | $(CH_2)_2S(O)CH_3$ | $OCH_3$ | $OCH_3$ | N | |
| H | H | $(CH_2)_2S(O)CH_2CH_3$ | $CH_3$ | $CH_3$ | CH | |
| H | H | $(CH_2)_2S(O)CH_2CH_3$ | $CH_3$ | $OCH_3$ | CH | |
| H | H | $(CH_2)_2S(O)CH_2CH_3$ | $OCH_3$ | $OCH_3$ | CH | |
| H | H | $(CH_2)_2S(O)CH_2CH_3$ | Cl | $OCH_3$ | CH | |
| H | H | $(CH_2)_2S(O)CH_2CH_3$ | $CH_3$ | $OCH_3$ | N | |
| H | H | $(CH_2)_2S(O)CH_2CH_3$ | $OCH_3$ | $OCH_3$ | N | |
| H | 5-$CH_3$ | $CH_2SO_2CH_3$ | $CH_3$ | $CH_3$ | CH | |
| H | 5-$CH_3$ | $CH_2SO_2CH_3$ | $CH_3$ | $OCH_3$ | CH | |
| H | 5-$CH_3$ | $CH_2SO_2CH_3$ | $OCH_3$ | $OCH_3$ | CH | |
| H | 5-$CH_3$ | $CH_2SO_2CH_3$ | $CH_3$ | $OCH_3$ | N | |
| H | H | $(CH_2)_2SO_2CH_3$ | $CH_3$ | $CH_3$ | CH | |
| H | H | $(CH_2)_2SO_2CH_3$ | $CH_3$ | $OCH_3$ | CH | |
| H | H | $(CH_2)_2SO_2CH_3$ | $OCH_3$ | $OCH_3$ | CH | |
| H | H | $(CH_2)_2SO_2CH_3$ | Cl | $OCH_3$ | CH | |
| H | H | $(CH_2)_2SO_2CH_3$ | $CH_3$ | $OCH_3$ | N | |
| H | H | $(CH_2)_2SO_2CH_3$ | $OCH_3$ | $OCH_3$ | N | |

Table III (Continued)

| R | $R_1$ | Q | X | Y | Z | m.p. (°C) |
|---|---|---|---|---|---|---|
| H | H | $(CH_2)_3SO_2CH_3$ | $OCH_3$ | $OCH_3$ | CH | |
| H | H | $(CH_2)_3SO_2CH_3$ | Cl | $OCH_3$ | CH | |
| H | H | $CH_2SO_2CH_2CH_3$ | $CH_3$ | $CH_3$ | CH | |
| H | H | $CH_2SO_2CH_2CH_3$ | $CH_3$ | $OCH_3$ | CH | |
| H | H | $CH_2SO_2CH_2CH_3$ | $OCH_3$ | $OCH_3$ | CH | |
| H | H | $CH_2SO_2CH_2CH_3$ | Cl | $OCH_3$ | CH | |
| H | H | $CH_2SO_2CH_2CH_3$ | $CH_3$ | $OCH_3$ | N | |
| H | H | $CH_2CO_2CH_3$ | $CH_3$ | $CH_3$ | CH | |
| H | H | $CH_2CO_2CH_3$ | $CH_3$ | $OCH_3$ | CH | |
| H | H | $CH_2CO_2CH_3$ | $OCH_3$ | $OCH_3$ | CH | |
| H | H | $CH_2CO_2CH_3$ | Cl | $OCH_3$ | CH | |
| H | H | $CH_2CO_2CH_3$ | $CH_3$ | $OCH_3$ | N | |
| H | H | $CH_2CO_2CH_2CH_3$ | $CH_3$ | $CH_3$ | CH | |
| H | H | $CH_2CO_2CH_2CH_3$ | $CH_3$ | $OCH_3$ | CH | |
| H | H | $CH_2CO_2CH_2CH_3$ | $OCH_3$ | $OCH_3$ | CH | |
| H | H | $CH_2CO_2CH_2CH_3$ | Cl | $OCH_3$ | CH | |
| H | H | $CH_2CO_2CH_2CH_3$ | $CH_3$ | $OCH_3$ | N | |
| H | H | $CH_2SO_2N(CH_3)_2$ | $CH_3$ | $CH_3$ | CH | |
| H | H | $CH_2SO_2N(CH_3)_2$ | $CH_3$ | $OCH_3$ | CH | |
| H | H | $CH_2SO_2N(CH_3)_2$ | $OCH_3$ | $OCH_3$ | CH | |
| H | H | $CH_2SO_2N(CH_3)_2$ | Cl | $OCH_3$ | CH | |
| H | H | $CH_2SO_2N(CH_3)_2$ | $CH_3$ | $OCH_3$ | N | |
| H | H | $CH_2SO_2N(CH_3)_2$ | $OCH_3$ | $OCH_3$ | N | |
| H | 5-Cl | $(CH_2)_2SO_2N(CH_3)_2$ | $CH_3$ | $OCH_3$ | CH | |
| H | 5-Cl | $(CH_2)_2SO_2N(CH_3)_2$ | $OCH_3$ | $OCH_3$ | CH | |
| H | 5-Cl | $(CH_2)_2SO_2N(CH_3)_2$ | $CH_3$ | $OCH_3$ | N | |
| H | H | $CH_2SO_2N(CH_2CH_3)_2$ | $CH_3$ | $CH_3$ | CH | |
| H | H | $CH_2SO_2N(CH_2CH_3)_2$ | $CH_3$ | $OCH_3$ | CH | |
| H | H | $CH_2SO_2N(CH_2CH_3)_2$ | $OCH_3$ | $OCH_3$ | CH | |
| H | H | $CH_2SO_2N(CH_2CH_3)_2$ | $CH_3$ | $OCH_3$ | N | |
| H | H | $CH_2C(O)CH_3$ | $CH_3$ | $OCH_3$ | CH | |
| H | H | $CH_2C(O)CH_3$ | $OCH_3$ | $OCH_3$ | CH | |

Table III (Continued)

| R | R1 | Q | X | Y | Z | m.p. (°C) |
|---|---|---|---|---|---|---|
| H | H | $CH_2C(O)CH_3$ | $CH_3$ | $OCH_3$ | N | |
| H | 2-Cl | $(CH_2)_2C(O)CH_3$ | $CH_3$ | $OCH_3$ | CH | |
| H | 2-Cl | $(CH_2)_2C(O)CH_3$ | $OCH_3$ | $OCH_3$ | CH | |
| H | 2-Cl | $(CH_2)_2C(O)CH_3$ | $CH_3$ | $OCH_3$ | N | |
| H | H | $CH_2CN$ | $CH_3$ | $CH_3$ | CH | |
| H | H | $CH_2CN$ | $CH_3$ | $OCH_3$ | CH | |
| H | H | $CH_2CN$ | $OCH_3$ | $OCH_3$ | CH | |
| H | H | $CH_2CN$ | Cl | $OCH_3$ | CH | |
| H | H | $CH_2CN$ | $CH_3$ | $OCH_3$ | N | |
| H | H | $CH_2CN$ | $OCH_3$ | $OCH_3$ | N | |
| H | H | $(CH_2)_2CN$ | $CH_3$ | $CH_3$ | CH | |
| H | H | $(CH_2)_2CN$ | $CH_3$ | $OCH_3$ | CH | |
| H | H | $(CH_2)_2CN$ | $OCH_3$ | $OCH_3$ | CH | |
| H | H | $(CH_2)_2CN$ | Cl | $OCH_3$ | CH | |
| H | H | $(CH_2)_2CN$ | $CH_3$ | $OCH_3$ | N | |
| H | H | $(CH_2)_2CN$ | $OCH_3$ | $OCH_3$ | N | |
| H | H | $CH_2C(O)N(CH_3)_2$ | $CH_3$ | $OCH_3$ | CH | |
| H | H | $CH_2C(O)N(CH_3)_2$ | $OCH_3$ | $OCH_3$ | CH | |
| H | H | $CH_2C(O)N(CH_3)_2$ | $CH_3$ | $OCH_3$ | N | |
| H | H | $(CH_2)_2C(O)N(CH_3)_2$ | $CH_3$ | $OCH_3$ | CH | |
| H | H | $(CH_2)_2C(O)N(CH_3)_2$ | $OCH_3$ | $OCH_3$ | CH | |
| H | H | $(CH_2)_2C(O)N(CH_3)_2$ | $CH_3$ | $OCH_3$ | N | |
| H | H | $CH_2Cl$ | $CH_3$ | $CH_3$ | CH | |
| H | H | $CH_2Cl$ | $CH_3$ | $OCH_3$ | CH | |
| H | H | $CH_2Cl$ | $OCH_3$ | $OCH_3$ | CH | |
| H | H | $CH_2Cl$ | Cl | $OCH_3$ | CH | |
| H | H | $CH_2Cl$ | $CH_3$ | $OCH_3$ | N | |
| H | H | $CH_2Cl$ | $OCH_3$ | $OCH_3$ | N | |
| H | H | $(CH_2)_2Cl$ | $CH_3$ | $CH_3$ | CH | |
| H | H | $(CH_2)_2Cl$ | $CH_3$ | $OCH_3$ | CH | |
| H | H | $(CH_2)_2Cl$ | $OCH_3$ | $OCH_3$ | CH | |
| H | H | $(CH_2)_2Cl$ | Cl | $OCH_3$ | CH | |

Table III (Continued)

| R | $R_1$ | Q | X | Y | Z | m.p. (°C) |
|---|---|---|---|---|---|---|
| H | H | $(CH_2)_2Cl$ | $CH_3$ | $OCH_3$ | N | |
| H | H | $(CH_2)_2Cl$ | $OCH_3$ | $OCH_3$ | N | |
| H | H | $CH_2F$ | $CH_3$ | $CH_3$ | CH | |
| H | H | $CH_2F$ | $CH_3$ | $OCH_3$ | CH | |
| H | H | $CH_2F$ | $OCH_3$ | $OCH_3$ | CH | |
| H | H | $CH_2F$ | Cl | $OCH_3$ | CH | |
| H | H | $CH_2F$ | $CH_3$ | $OCH_3$ | N | |
| H | H | $CH_2F$ | $OCH_3$ | $OCH_3$ | N | |
| H | H | $(CH_2)_2F$ | $CH_3$ | $OCH_3$ | CH | |
| H | H | $(CH_2)_2F$ | $OCH_3$ | $OCH_3$ | CH | |
| H | H | $(CH_2)_2F$ | $CH_3$ | $OCH_3$ | N | |
| H | H | $CH_2Br$ | $CH_3$ | $CH_3$ | CH | |
| H | H | $CH_2Br$ | $CH_3$ | $OCH_3$ | CH | |
| H | H | $CH_2Br$ | $OCH_3$ | $OCH_3$ | CH | |
| H | H | $CH_2Br$ | Cl | $OCH_3$ | CH | |
| H | H | $CH_2Br$ | $CH_3$ | $OCH_3$ | N | |
| H | H | $CH_2Br$ | $OCH_3$ | $OCH_3$ | N | |
| H | H | $(CH_2)_2Br$ | $CH_3$ | $OCH_3$ | CH | |
| H | H | $(CH_2)_2Br$ | $OCH_3$ | $OCH_3$ | CH | |
| H | H | $(CH_2)_2Br$ | Cl | $OCH_3$ | CH | |
| H | H | $CH_2SCH_3$ | $OCH_2CH_3$ | $C_2H_5$ | CH | |
| H | H | $CH_2SCH_3$ | $OCH_2CH_3$ | $C_2H_5$ | N | |
| H | H | $CH_2SCH_3$ | $OCH_2CH_3$ | $CH_2OCH_3$ | CH | |
| H | H | $CH_2SCH_3$ | $OCH_2CH_3$ | $CH_2OCH_3$ | N | |
| H | H | $CH_2SCH_3$ | $OCH_2CH_3$ | $NHCH_3$ | N | |
| H | H | $CH_2SCH_3$ | $OCH_2CH_3$ | $CH(OCH_3)_2$ | CH | |
| H | H | $CH_2SCH_3$ | $OCF_2H$ | $C_2H_5$ | CH | |
| H | H | $CH_2SCH_3$ | $OCF_2H$ | $CH_2OCH_3$ | CH | |
| H | H | $CH_2SCH_3$ | $OCH_2CF_3$ | $C_2H_5$ | CH | |
| H | H | $CH_2SCH_3$ | $OCH_2CF_3$ | $C_2H_5$ | N | |
| H | H | $CH_2SCH_3$ | $OCH_2CF_3$ | $CH_2OCH_3$ | CH | |
| H | H | $CH_2SCH_3$ | $OCH_2CF_3$ | $CH_2OCH_3$ | N | |

Table III (Continued)

| R | $R_1$ | Q | X | Y | Z | m.p. (°C) |
|---|---|---|---|---|---|---|
| H | H | $CH_2SCH_3$ | $OCH_2CF_3$ | $NHCH_3$ | N | |
| H | H | $CH_2SCH_3$ | $OCH_2CF_3$ | $CH(OCH_3)_2$ | N | |
| H | H | $CH_2SCH_3$ | $CH_3$ | $CH_2OCH_3$ | CH | |
| H | H | $CH_2SCH_3$ | $CH_3$ | $CH_2OCH_3$ | N | |
| H | H | $CH_2SCH_3$ | $CH_3$ | $NHCH_3$ | N | |
| H | H | $CH_2SCH_3$ | $CH_3$ | $CH(OCH_3)_2$ | N | |
| H | H | $CH_2SCH_3$ | $OCH_3$ | $C_2H_5$ | CH | |
| H | H | $CH_2SCH_3$ | $OCH_3$ | $C_2H_5$ | N | |
| H | H | $CH_2SCH_3$ | $OCH_3$ | $CH_2OCH_3$ | CH | |
| H | H | $CH_2SCH_3$ | $OCH_3$ | $CH_2OCH_3$ | N | |
| H | H | $CH_2SCH_3$ | $OCH_3$ | $NHCH_3$ | N | |
| H | H | $CH_2SCH_3$ | $OCH_3$ | $CH(OCH_3)_2$ | CH | |
| H | H | $CH_2SCH_3$ | $OCH_3$ | $CH(OCH_3)_2$ | N | |
| H | 5-Cl | $CH_2OCH_3$ | $OCH_2CH_3$ | $C_2H_5$ | CH | |
| H | 5-Cl | $CH_2OCH_3$ | $OCH_2CH_3$ | $C_2H_5$ | N | |
| H | 5-Cl | $CH_2OCH_3$ | $OCH_2CH_3$ | $CH_2OCH_3$ | CH | |
| H | 5-Cl | $CH_2OCH_3$ | $OCH_2CH_3$ | $CH_2OCH_3$ | N | |
| H | 5-Cl | $CH_2OCH_3$ | $OCH_2CH_3$ | $NHCH_3$ | CH | |
| H | 5-Cl | $CH_2OCH_3$ | $OCH_2CH_3$ | $NHCH_3$ | N | |
| H | 5-Cl | $CH_2OCH_3$ | $OCH_2CH_3$ | $CH(OCH_3)_2$ | CH | |
| H | 5-Cl | $CH_2OCH_3$ | $OCH_2CH_3$ | $CH(OCH_3)_2$ | N | |
| H | 5-Cl | $CH_2OCH_3$ | $OCF_2H$ | $C_2H_5$ | CH | |
| H | 5-Cl | $CH_2OCH_3$ | $OCF_2H$ | $CH_2OCH_3$ | CH | |
| H | 5-Cl | $CH_2OCH_3$ | $OCF_2H$ | $NHCH_3$ | CH | |
| H | 5-Cl | $CH_2OCH_3$ | $OCH_2CF_3$ | $C_2H_5$ | CH | |
| H | 5-Cl | $CH_2OCH_3$ | $OCH_2CF_3$ | $CH_2OCH_3$ | N | |
| H | 5-Cl | $CH_2OCH_3$ | $OCH_2CF_3$ | $NHCH_3$ | CH | |
| H | 5-Cl | $CH_2OCH_3$ | $OCH_2CF_3$ | $NHCH_3$ | N | |
| H | 5-Cl | $CH_2OCH_3$ | $OCH_2CF_3$ | $CH(OCH_3)_2$ | CH | |
| H | 5-Cl | $CH_2OCH_3$ | $OCH_2CF_3$ | $CH(OCH_3)_2$ | N | |
| H | 5-Cl | $CH_2OCH_3$ | Cl | $NHCH_3$ | CH | |
| H | 5-Cl | $CH_2OCH_3$ | $CH_3$ | $C_2H_5$ | CH | |

Table III (Continued)

| R | $R_1$ | Q | X | Y | Z | m.p. (°C) |
|---|---|---|---|---|---|---|
| H | 5-Cl | $CH_2OCH_3$ | $CH_3$ | $CH_2OCH_3$ | CH | |
| H | 5-Cl | $CH_2OCH_3$ | $CH_3$ | $CH_2OCH_3$ | N | |
| H | 5-Cl | $CH_2OCH_3$ | Cl | $NHCH_3$ | CH | |
| H | 5-Cl | $CH_2OCH_3$ | $CH_3$ | $CH_2OCH_3$ | CH | |
| H | 5-Cl | $CH_2OCH_3$ | $CH_3$ | $CH_2OCH_3$ | N | |
| H | 5-Cl | $CH_2OCH_3$ | $CH_3$ | $NHCH_3$ | N | |
| H | 5-Cl | $CH_2OCH_3$ | $CH_3$ | $CH(OCH_3)_2$ | CH | |
| H | 5-Cl | $CH_2OCH_3$ | $CH_3$ | $CH(OCH_3)_2$ | N | |
| H | 5-Cl | $CH_2OCH_3$ | $OCH_3$ | $C_2H_5$ | CH | |
| H | 5-Cl | $CH_2OCH_3$ | $OCH_3$ | $C_2H_5$ | N | |
| H | 5-Cl | $CH_2OCH_3$ | $OCH_3$ | $CH_2OCH_3$ | CH | |
| H | 5-Cl | $CH_2OCH_3$ | $OCH_3$ | $CH_2OCH_3$ | N | |
| H | 5-Cl | $CH_2OCH_3$ | $OCH_3$ | $NHCH_3$ | CH | |
| H | 5-Cl | $CH_2OCH_3$ | $OCH_3$ | $NHCH_3$ | N | |
| H | 5-Cl | $CH_2OCH_3$ | $OCH_3$ | $CH(OCH_3)_2$ | CH | |
| H | 5-Cl | $CH_2OCH_3$ | $OCH_3$ | $CH(OCH_3)_2$ | N | |
| H | H | CN | $OCH_3$ | $CH_3$ | N | |
| H | H | CN | $OCH_3$ | $OCH_3$ | N | |

**Table IV**

**General Formula IV**

| R | $R_1$ | Q | $X_1$ | $Y_1$ | m.p.(°C) |
|---|---|---|---|---|---|
| H | H | $CH_2OCH_3$ | $CH_3$ | O | |
| H | H | $CH_2OCH_3$ | $OCH_3$ | O | |
| H | H | $CH_2OCH_3$ | $OC_2H_5$ | O | |
| H | H | $CH_2OCH_3$ | $OCF_2H$ | O | |
| H | H | $CH_2OCH_3$ | $CH_3$ | $CH_2$ | |
| H | H | $CH_2OCH_3$ | $OCH_3$ | $CH_2$ | |
| H | H | $CH_2OCH_3$ | $OC_2H_5$ | $CH_2$ | |
| H | H | $CH_2OCH_3$ | $OCF_2H$ | $CH_2$ | |
| H | H | $SCH_2CH_2OCH_3$ | $OCH_3$ | O | |
| H | H | $SCH_2CH_2OCH_3$ | $CH_3$ | O | |
| H | H | $S(CH_2)_3Cl$ | $CH_3$ | O | |
| H | H | $S(CH_2)_3Cl$ | $OCH_3$ | O | |
| H | H | $SCH_2C\equiv CH$ | $OCH_3$ | O | |
| H | H | $SCH_2C\equiv CH$ | $CH_3$ | O | |
| H | H | $CH_2Cl$ | $OCH_3$ | O | |
| H | H | $CHClCH_3$ | $OCH_3$ | O | |

**Table V**

**General Formula V**

| R | $R_1$ | Q | $X_1$ | m.p.(°C) |
|---|---|---|---|---|
| H | H | $SCH_2CH=CH_2$ | $CH_3$ | |
| H | H | $SCH_2CH=CH_2$ | $OCH_3$ | |
| H | H | $SCH_2CH=CH_2$ | $OCF_2H$ | |
| H | H | $CH_2OCH_3$ | $OCH_3$ | |
| H | H | $CH_2OCH_3$ | $CH_3$ | |
| H | H | $S(CH_2)_2SCH_3$ | $OCH_3$ | |
| H | H | $S(CH_2)_2SCH_3$ | $CH_3$ | |
| H | H | $O(CH_2)_2OCH_3$ | $OCH_3$ | |
| H | H | $O(CH_2)_2OCH_3$ | $CH_3$ | |

Table VI

General Formula VI

| R | $R_1$ | Q | $X_1$ | $Y_2$ | m.p.(°C) |
|---|---|---|---|---|---|
| H | H | $S(CH_2)_2SCH_3$ | $CH_3$ | H | |
| H | H | $S(CH_2)_2SCH_3$ | $OCH_3$ | H | |
| H | H | $S(CH_2)_2SCH_3$ | $OC_2H_5$ | H | |
| H | H | $S(CH_2)_2SCH_3$ | $OCF_2H$ | H | |
| H | H | $S(CH_2)_2SCH_3$ | $CH_3$ | $CH_3$ | |
| H | H | $S(CH_2)_2SCH_3$ | $OCH_3$ | $CH_3$ | |
| H | H | $S(CH_2)_2SCH_3$ | $OC_2H_5$ | $CH_3$ | |
| H | H | $S(CH_2)_2SCH_3$ | $OCF_2H$ | $CH_3$ | |
| H | H | $S(CH_2)_3Cl$ | $OCH_3$ | H | |
| H | H | $S(CH_2)_3Cl$ | $CH_3$ | H | |
| H | H | $SCH_2CO_2CH_3$ | $OCH_3$ | H | |
| H | H | $SCH_2CO_2CH_3$ | $CH_3$ | H | |
| H | H | $SO_2CH_2C{\equiv}CH$ | $OCH_3$ | H | |
| H | H | $SO_2CH_2C{\equiv}CH$ | $CH_3$ | H | |

Table VII

General Formula VII

| R | $R_1$ | Q | $X_2$ | $Y_3$ | m.p.(°C) |
|---|---|---|---|---|---|
| H | H | $(CH_2)_2OCH_3$ | $CH_3$ | $CH_3$ | |
| H | H | $(CH_2)_2OCH_3$ | $OCH_3$ | $CH_3$ | |
| H | H | $(CH_2)_2OCH_3$ | $SCH_3$ | $CH_3$ | |
| H | H | $(CH_2)_2OCH_3$ | $CH_3$ | $CH_2CH_3$ | |
| H | H | $(CH_2)_2OCH_3$ | $SCH_3$ | $CH_2CH_3$ | |
| H | H | $(CH_2)_2OCH_3$ | $SCH_3$ | $CH_2CF_3$ | |
| H | H | $(CH_2)_2OCH_3$ | $CH_3$ | $CH_2CF_3$ | |
| H | H | $(CH_2)_2OCH_3$ | $OCH_3$ | $CH_2CF_3$ | |
| H | H | $CH_2CN$ | $SCH_3$ | $CH_2CH_3$ | |
| H | H | $CH_2CN$ | $OCH_3$ | $CH_3$ | |

**Table VIII**

**General Formula VIII**

| R | $R_1$ | Q | $X_3$ | m.p.(°C) |
|---|---|---|---|---|
| H | H | $CH_2OCH_3$ | $CH_3$ | |
| H | H | $CH_2OCH_3$ | $OCH_3$ | |
| H | H | $S(CH_2)_2SCH_3$ | $OCH_3$ | |
| H | H | $SCH_2CO_2CH_3$ | $OCH_3$ | |
| H | H | $(CH_2)_2Br$ | $OCH_3$ | |
| H | H | $CH_2F$ | $OCH_3$ | |
| H | H | $CH_2CO_2CH_3$ | $OCH_3$ | |
| H | H | $CH_2SO_2N(CH_3)_2$ | $OCH_3$ | |

Table IX

General Formula IX

| R | $R_1$ | Q | X | Y | Z | m.p.(°C) |
|---|---|---|---|---|---|---|
| H | H | $CH_2OCH_3$ | $CH_3$ | $CH_3$ | CH | |
| H | H | $CH_2OCH_3$ | $OCH_3$ | $OCH_3$ | CH | |
| H | H | $CH_2OCH_3$ | Cl | $OCH_3$ | CH | |
| H | H | $CH_2OCH_3$ | $CH_3$ | $OCH_3$ | N | |
| H | H | $(CH_2)_2OCH_3$ | $OCH_3$ | $OCH_3$ | CH | |
| H | H | $(CH_2)_2OCH_3$ | Cl | $OCH_3$ | CH | |
| H | H | $(CH_2)_2OCH_3$ | $CH_3$ | $OCH_3$ | N | |
| H | H | $(CH_2)_2OCH_3$ | $CH_3$ | $CH_3$ | CH | |
| H | H | $CH_2Cl$ | $CH_3$ | $CH_3$ | CH | |
| H | H | $CH_2Cl$ | $OCH_3$ | $OCH_3$ | CH | |
| H | H | $CH_2Cl$ | Cl | $OCH_3$ | CH | |
| H | H | $CH_2Cl$ | $CH_3$ | $OCH_3$ | CH | |
| H | H | $CH_2OH$ | $CH_3$ | $CH_3$ | CH | |
| H | H | $CH_2OH$ | $CH_3$ | $OCH_3$ | CH | |
| H | H | $CH_2OH$ | $OCH_3$ | $OCH_3$ | CH | |
| H | H | $CH_2OH$ | Cl | $OCH_3$ | CH | |
| H | H | CN | $CH_3$ | $CH_3$ | CH | |
| H | H | CN | $CH_3$ | $OCH_3$ | CH | |
| H | H | CN | Cl | $OCH_3$ | CH | |
| H | H | $CH_2CN$ | $CH_3$ | $OCH_3$ | CH | |
| H | H | $CH_2CN$ | Cl | $OCH_3$ | CH | |
| H | H | $CH_2CN$ | $OCH_3$ | $OCH_3$ | CH | |
| H | H | $CH_2SCH_3$ | $CH_3$ | $CH_3$ | CH | |
| H | H | $CH_2SCH_3$ | Cl | $OCH_3$ | CH | |
| H | H | $CH_2SCH_3$ | $OCH_3$ | $OCH_3$ | CH | |
| H | H | $CH_2SCH_3$ | $CH_3$ | $OCH_3$ | N | |

Table X

General Formula X

| R | $R_1$ | Q | X | Y | Z | m.p.(°C) |
|---|---|---|---|---|---|---|
| H | H | $CH_2OCH_3$ | $CH_3$ | $CH_3$ | CH | |
| H | H | $CH_2OCH_3$ | $OCH_3$ | $OCH_3$ | CH | |
| H | H | $CH_2OCH_3$ | Cl | $OCH_3$ | CH | |
| H | H | $CH_2OCH_3$ | $CH_3$ | $OCH_3$ | N | |
| H | H | $(CH_2)_2OCH_3$ | $OCH_3$ | $OCH_3$ | CH | |
| H | H | $(CH_2)_2OCH_3$ | Cl | $OCH_3$ | CH | |
| H | H | $(CH_2)_2OCH_3$ | $CH_3$ | $OCH_3$ | N | |
| H | H | $(CH_2)_2OCH_3$ | $CH_3$ | $CH_3$ | CH | |
| H | H | $CH_2Cl$ | $CH_3$ | $CH_3$ | CH | |
| H | H | $CH_2Cl$ | $OCH_3$ | $OCH_3$ | CH | |
| H | H | $CH_2Cl$ | Cl | $OCH_3$ | CH | |
| H | H | $CH_2Cl$ | $CH_3$ | $OCH_3$ | CH | |
| H | H | $CH_2OH$ | $CH_3$ | $CH_3$ | CH | |
| H | H | $CH_2OH$ | $CH_3$ | $OCH_3$ | CH | |
| H | H | $CH_2OH$ | $OCH_3$ | $OCH_3$ | CH | |
| H | H | $CH_2OH$ | Cl | $OCH_3$ | CH | |
| H | H | CN | $CH_3$ | $CH_3$ | CH | |
| H | H | CN | $CH_3$ | $OCH_3$ | CH | |
| H | H | CN | Cl | $OCH_3$ | CH | |
| H | H | $CH_2CN$ | $CH_3$ | $OCH_3$ | CH | |
| H | H | $CH_2CN$ | Cl | $OCH_3$ | CH | |
| H | H | $CH_2CN$ | $OCH_3$ | $OCH_3$ | CH | |
| H | H | $CH_2SCH_3$ | $CH_3$ | $CH_3$ | CH | |
| H | H | $CH_2SCH_3$ | Cl | $OCH_3$ | CH | |
| H | H | $CH_2SCH_3$ | $OCH_3$ | $OCH_3$ | CH | |
| H | H | $CH_2SCH_3$ | $CH_3$ | $OCH_3$ | N | |

**Table XI**

**General Formula XI**

| R | $R_1$ | Q | X | Y | Z | m.p.(°C) |
|---|---|---|---|---|---|---|
| H | H | $CH_2OCH_3$ | $CH_3$ | $CH_3$ | CH | |
| H | H | $CH_2OCH_3$ | $OCH_3$ | $OCH_3$ | CH | |
| H | H | $CH_2OCH_3$ | Cl | $OCH_3$ | CH | |
| H | H | $CH_2OCH_3$ | $CH_3$ | $OCH_3$ | N | |
| H | H | $(CH_2)_2OCH_3$ | $OCH_3$ | $OCH_3$ | CH | |
| H | H | $(CH_2)_2OCH_3$ | Cl | $OCH_3$ | CH | |
| H | H | $(CH_2)_2OCH_3$ | $CH_3$ | $OCH_3$ | N | |
| H | H | $(CH_2)_2OCH_3$ | $CH_3$ | $CH_3$ | CH | |
| H | H | $CH_2Cl$ | $CH_3$ | $CH_3$ | CH | |
| H | H | $CH_2Cl$ | $OCH_3$ | $OCH_3$ | CH | |
| H | H | $CH_2Cl$ | Cl | $OCH_3$ | CH | |
| H | H | $CH_2Cl$ | $CH_3$ | $OCH_3$ | CH | |
| H | H | $CH_2OH$ | $CH_3$ | $CH_3$ | CH | |
| H | H | $CH_2OH$ | $CH_3$ | $OCH_3$ | CH | |
| H | H | $CH_2OH$ | $OCH_3$ | $OCH_3$ | CH | |
| H | H | $CH_2OH$ | Cl | $OCH_3$ | CH | |
| H | H | CN | $CH_3$ | $CH_3$ | CH | |
| H | H | CN | $CH_3$ | $OCH_3$ | CH | |
| H | H | CN | Cl | $OCH_3$ | CH | |
| H | H | $CH_2CN$ | $CH_3$ | $OCH_3$ | CH | |
| H | H | $CH_2CN$ | Cl | $OCH_3$ | CH | |
| H | H | $CH_2CN$ | $OCH_3$ | $OCH_3$ | CH | |
| H | H | $CH_2SCH_3$ | $CH_3$ | $CH_3$ | CH | |
| H | H | $CH_2SCH_3$ | Cl | $OCH_3$ | CH | |
| H | H | $CH_2SCH_3$ | $OCH_3$ | $OCH_3$ | CH | |
| H | H | $CH_2SCH_3$ | $CH_3$ | $OCH_3$ | N | |

Formulations

Useful formulations of the compounds of Formula I be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table XV

| | Active Ingredient | Weight Percent* Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a surfactant or a diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden. "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of addi- tives to reduce foaming, caking, corrosion, microbio- logical growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 12

Wettable Powder

| | |
|---|---|
| N-[(4,6-dimethoxypyrimidin-2-yl)aminocarbonyl]-2-[2-(dimethylaminosulfonyl)ethyl]-3-thiophenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 13

Wettable Powder

| | |
|---|---|
| 2-[2-(dimethylaminosulfonyl)ethyl]-N-[(4-methoxy-6-methyl-1,3,5-triazin-2-yl)aminocarbonyl]-3-thiophenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 14

Granule

| | |
|---|---|
| Wettable Powder of Example 13 | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

Example 15

Extruded Pellet

| | |
|---|---|
| N-[(4,6-dimethoxypyrimidin-2-yl)aminocarbonyl]-2-(methoxymethyl)-3-thiophenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 16

Low Strength Granule

| | |
|---|---|
| N-[(4-methoxy-6-methyl-1,3,5-triazin-2-yl)-aminocarbonyl]-2-(methoxymethyl)-3-thiophenesulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 17

Granule

| | |
|---|---|
| N-[(4,6-dimethoxypyrimidin-2-yl)aminocarbonyl]-2-[2-(dimethylaminosulfonyl)ethyl]-3-thiophenesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged,

screened to the desired size range, generally 14-100 mesh (1410-149 microns), and packaged for use.

Example 18

Low Strength Granule

| | |
|---|---|
| 2-[2-(dimethylaminosulfonyl)ethyl]-N-[(4-methoxy-6-methyl-1,3,5-triazin-2-yl)aminocarbonyl]-3-thiophenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 19

Aqueous Suspension

| | |
|---|---|
| N-[(4,6-dimethoxypyrimidin-2-yl)aminocarbonyl]-2-(methoxymethyl)-3-thiophenesulfonamide | 40.0% |
| polyacrylic acid thickener | 0.3% |
| dodecylphenol polyethylene glycol ether | 0.5% |
| disodium phosphate | 1.0% |
| monosodium phosphate | 0.5% |
| polyvinyl alcohol | 1.0% |
| water | 56.7% |

The ingredients are blended and ground together in a sand mill to produce particles essentially all under 5 microns in size.

Example 20

Solution

| | |
|---|---|
| N-[(4-methoxy-6-methyl-1,3,5-triazin-2-yl)aminocarbonyl]-2-(methoxymethyl)-3-thiophenesulfonamide, sodium salt | 5% |
| water | 95% |

The salt is added directly to the water with stirring to produce the solution, which may then be packaged for use.

Example 21

High Strength Concentrate

| | |
|---|---|
| N-[(4,6-dimethoxypyrimidin-2-yl)aminocarbonyl]-2-[2-(dimethylaminosulfonyl)ethyl]-3-thiophenesulfonamide | 99.0% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 22

Wettable Powder

| | |
|---|---|
| 2-[2-(dimethylaminosulfonyl)ethyl]-N-[(4-methoxy-6-methyl-1,3,5-triazin-2-yl)aminocarbonyl]-3-thiophenesulfonamide | 90.0% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 23

Wettable Powder

| | |
|---|---|
| N-[(4,6-dimethoxypyrimidin-2-yl)aminocarbonyl]-2-(methoxymethyl)-3-thiophenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 24

Oil Suspension

| | |
|---|---|
| N-[(4-methoxy-6-methyl-1,3,5-triazin-2-yl)-aminocarbonyl]-2-(methoxymethyl)-3-thiophenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 25

Dust

| | |
|---|---|
| N-[(4,6-dimethoxypyrimidin-2-yl)aminocarbonyl]-2-[2-(dimethylaminosulfonyl)ethyl]-3-thiophenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 26

Oil Suspension

| | |
|---|---|
| 2-[2-(dimethylaminosulfonyl)ethyl]-N-[(4-methoxy-6-methyl-1,3,5-triazin-2-yl)aminocarbonyl]-3-thiophenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 27

Wettable Powder

| | |
|---|---|
| N-[(4,6-dimethoxypyrimidin-2-yl)aminocarbonyl]-2-(methoxymethyl)-3-thiophenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Other formulations may be prepared analogously to Examples 29 to 45 of our EP-A-207 609.

## Utility

The compounds of the present invention ore expected to be highly active preemergent or postemergent herbicides or plant growth regulants. Many of them should have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Some of the compounds should have utility for selective weed control in crops such as rice and wheat. Alternatively, the subject compounds should be useful to modify plant growth.

The rates of application for the compounds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of around 0.01 to 10 kg/ha, the lower rates being suggested for use on lighter soils and/or those having a low organic matter content, for plant growth modification or for situations where only short-term persistence is required. Those compounds which exhibited no activity at test rates indicated in the biological tables are expected to show activity at higher application rates.

The compounds of the invention may be used in combination with any other commercial herbicide, examples of which are those of the triazine, triazole, uracil, urea, amide, diphenylether, carbamate and bipyridylium types.

The herbicidal properties of the subject compounds were discovered in a number of greenhouse tests. The test procedures and results follow.

## Test A

Seeds of crabgrass (Digitaria spp.), barnyardgrass (Echinochloa crusgalli), wild oats (Avena fatua), morningglory (Ipomoea spp.), cocklebur (Xanthium pensylvanicum), velvetleaf (Abutilon theophrasti), giant foxtail (Setaria faberii), cheatgrass (Bromus secalinus L.), sicklepod (Cassia obtusifolia), sorghum, corn, soybean, sugarbeet, cotton, rice, barley, wheat and purple nutsedge (Cyperus rotundus) tubers were planted and treated preemergence with the test chemicals dissolved in a nonphytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis;
B = burn;
D = defoliation;
E = emergence inhibition;
G = growth retardation;
H = formative effect;
U = unusual pigmentation;

X = axillary stimulation;
S = albinism; and
6Y = abscised buds or flowers.

Compounds

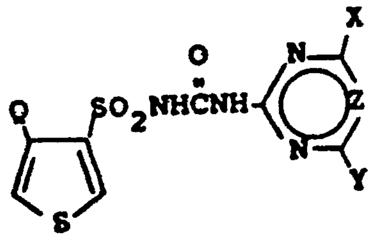

| Compound | Q | X | Y | Z |
|---|---|---|---|---|
| 1 | CN | $CH_3$ | $CH_3$ | CH |
| 2 | CN | $CH_3$ | $OCH_3$ | CH |
| 3 | CN | $CH_3$ | $OCH_3$ | N |
| 4 | CN | $OCH_3$ | $OCH_3$ | N |
| 5 | CN | Cl | $OCH_3$ | CH |

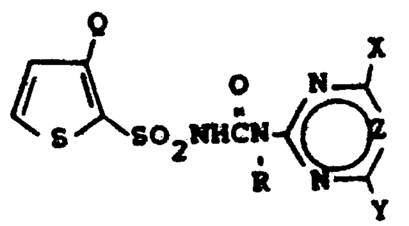

| Compound | Q | R | X | Y | Z |
|---|---|---|---|---|---|
| 6 | CN | H | $CH_3$ | $CH_3$ | CH |
| 7 | CN | H | $OCH_3$ | $CH_3$ | CH |
| 8 | CN | H | $OCH_3$ | $OCH_3$ | CH |
| 9 | CN | H | Cl | $OCH_3$ | CH |
| 10 | CN | H | $OCH_3$ | $CH_3$ | N |
| 11 | CN | H | $OCH_3$ | $OCH_3$ | N |
| 12 | $CH_2CH_2OCH_3$ | H | $OCH_3$ | $OCH_3$ | CH |
| 13 | $CH_2CH_2OCH_3$ | H | $OCH_3$ | $CH_3$ | N |
| 14 | $CH_2CH_2OCH_3$ | H | $CH_3$ | $CH_3$ | CH |
| 15 | $CH_2CH_2OCH_3$ | H | Cl | $OCH_3$ | CH |
| 16 | $CH_2Cl$ | H | $CH_3$ | $CH_3$ | CH |
| 17 | $CH_2Cl$ | H | $CH_3$ | $OCH_3$ | CH |
| 18 | $CH_2Cl$ | H | $OCH_3$ | $OCH_3$ | CH |
| 19 | $CH_3Cl$ | H | $CH_3$ | $OCH_3$ | N |

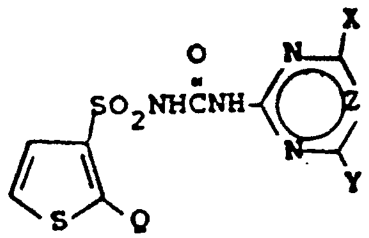

| Compound | Q | X | Y | Z |
|---|---|---|---|---|
| 20 | $CH_2OCH_3$ | $CH_3$ | $CH_3$ | CH |
| 21 | $CH_2OCH_3$ | $OCH_3$ | $OCH_3$ | CH |
| 22 | $CH_2OCH_3$ | Cl | $OCH_3$ | CH |
| 23 | $CH_2OCH_3$ | $OCH_3$ | $CH_3$ | N |
| 24 | $CH_2OCH_3$ | $OCH_3$ | $OCH_3$ | N |
| 25 | $CH_2OCH_2CH_3$ | $OCH_3$ | $OCH_3$ | CH |
| 26 | $CH_2Cl$ | $OCH_3$ | $CH_3$ | CH |
| 27 | $CH_2Cl$ | Cl | $OCH_3$ | CH |
| 28 | $CH_2Cl$ | $OCH_3$ | $CH_3$ | N |
| 29 | $CH_2Cl$ | $OCH_3$ | $OCH_3$ | N |

## Table A

| | Compound 1 | | Compound 2 | |
|---|---|---|---|---|
| Rate kg/ha | 0.05 | 0.01 | 0.05 | 0.01 |
| POSTEMERGENCE | | | | |
| Morningglory | 9C | 9H | 10C | 9C |
| Cocklebur | 10C | 2C,9G | 10C | 9C |
| Velvetleaf | 10C | 9C | 10C | 9C |
| Nutsedge | 9C | 3C,9G | 4C,9G | 8G |
| Crabgrass | 8G | 7G | 3C,8G | 2C,7G |
| Giant Foxtail | 3C,6G | 1H | 3C,8H | 2C,6H |
| Barnyardgrass | 9C | 2C,5H | 10C | 3C,8H |
| Cheatgrass | 6C,9G | 8G | 9C | 2C,8G |
| Wild Oats | 9G | 3C,9G | 6C,9G | 3C,6G |
| Wheat | 9G | 2G | 4C,9G | 5G |
| Corn | 10C | 5U,9G | 10C | 4U,9C |
| Barley | 5C,9G | 5G | 3C,9H | 8G |
| Soybean | 9C | 9C | 9C | 9C |
| Rice | 5C,9G | 4C,9G | 9C | 5C,9G |
| Sorghum | 2U,9G | 2C,8G | 5C,9G | 2C,8G |
| Sugar beet | 9C | 9C | 10C | 9C |
| Cotton | 9C | 10C | 10C | 10C |
| PREEMERGENCE | | | | |
| Morningglory | 9G | 9G | 9G | 8G |
| Cocklebur | 7H | 4G | 3C,7H | 1H |
| Velvetleaf | 8G | 3G | 8G | 7G |
| Nutsedge | 10E | 10E | 10E | 2C,9G |
| Crabgrass | 9G | 3G | 3C,8G | 6G |
| Giant Foxtail | 0 | 0 | 7G | 0 |
| Barnyardgrass | 8G | 2G | 9H | 3G |
| Cheatgrass | 9H | 8H | 4C,9H | 8G |
| Wild Oats | 2C,8H | 7G | 4C,8H | 5G |
| Wheat | 9G | 7G | 8G | 5G |
| Corn | 9G | 8G | 9G | 9G |
| Barley | 9G | 6G | 9G | 8G |
| Soybean | 3C,9H | 6H | 9G | 3C,7H |
| Rice | 10E | 9H | 10E | 9H |
| Sorghum | 9H | 2C,9G | 10E | 10E |
| Sugar beet | 9G | 5G | 5C,9G | 9G |
| Cotton | 9G | 8G | 9G | 9G |

Table A (continued)

| | Compound 3 | | Compound 4 | |
|---|---|---|---|---|
| Rate kg/ha | 0.05 | 0.01 | 0.05 | 0.01 |
| POSTEMERGENCE | | | | |
| Morningglory | 9C | 5C,9G | 4C,9H | 5G |
| Cocklebur | 9C | 5C,9G | 0 | 0 |
| Velvetleaf | 9C | 3C,7H | 5C,9G | 4G |
| Nutsedge | 5G | 2G | 0 | 0 |
| Crabgrass | 2C,5G | 0 | 0 | 0 |
| Giant Foxtail | 3C,8G | 0 | 0 | 0 |
| Barnyardgrass | 9C | 3C,9H | 3C,8H | 0 |
| Cheatgrass | 3C,9G | 2G | 2G | 0 |
| Wild Oats | 3C,7G | 0 | 2C | 0 |
| Wheat | 3C,9G | 4G | 3G | 0 |
| Corn | 3U,9C | 9H | 9H | 0 |
| Barley | 4G | 0 | 0 | 0 |
| Soybean | 9C | 5C,9G | 5C,9G | 4C,9G |
| Rice | 9C | 9G | 2C,9G | 6G |
| Sorghum | 3C,9G | 3C,8G | 5G | 0 |
| Sugar beet | 9C | 9C | 9C | 4C,5G |
| Cotton | 10C | 3C,8G | 4C,9H | 2C,5G |
| PREEMERGENCE | | | | |
| Morningglory | 9G | 7H | 8H | 0 |
| Cocklebur | 9H | 0 | 0 | 0 |
| Velvetleaf | 2G | 0 | 0 | 0 |
| Nutsedge | 0 | 0 | 0 | 0 |
| Crabgrass | 0 | 0 | 0 | 0 |
| Giant Foxtail | 2G | 0 | 0 | 0 |
| Barnyardgrass | 7G | 0 | 0 | 0 |
| Cheatgrass | 7G | 0 | 0 | 0 |
| Wild Oats | 4G | 0 | 0 | 0 |
| Wheat | 8G | 0 | 6G | 0 |
| Corn | 9G | 7H | 7G | 0 |
| Barley | 3G | 0 | 0 | 0 |
| Soybean | 9H | 3C,4H | 3C,6H | 0 |
| Rice | 9H | 6G | 9H | 0 |
| Sorghum | 9H | 3G | 2G | 0 |
| Sugar beet | 9G | 2H | 3G | 0 |
| Cotton | 9G | 2G | 6G | 0 |

Table A (continued)

| | Compound 5 | | Compound 6 | |
|---|---|---|---|---|
| Rate kg/ha | 0.05 | 0.01 | 0.05 | 0.01 |
| POSTEMERGENCE | | | | |
| Morningglory | 10C | 10C | 9C | 3G |
| Cocklebur | 10C | 5G | 10C | 8H |
| Velvetleaf | 9C | 3C,8H | 10C | 9C |
| Nutsedge | 4C,8G | 5G | 7G | 3G |
| Crabgrass | 2G | 0 | 2G | 0 |
| Giant Foxtail | 3G | 0 | 2C | 0 |
| Barnyardgrass | 4C,9H | 3C,8H | 3C,8H | 0 |
| Cheatgrass | 2C,7G | 0 | 5G | 0 |
| Wild Oats | 0 | 0 | 2G | 0 |
| Wheat | 0 | 0 | 3G | 0 |
| Corn | 5C,9G | 9H | 5C,9G | 2C,7H |
| Barley | 2C | 0 | 0 | 0 |
| Soybean | 9C | 4C,8G | 2C,8H | 6H |
| Rice | 9C | 2C,7G | 9C | 4C,9G |
| Sorghum | 4C,9H | 3C,9H | 9C | 2C,6G |
| Sugar beet | 9C | 10C | 10C | 10C |
| Cotton | 9C | 5C,9G | 10C | 4C,9G |
| PREEMERGENCE | | | | |
| Morningglory | 9G | 7G | 6G | 4G |
| Cocklebur | 0 | - | 5G | 4G |
| Velvetleaf | 3G | 2G | 6G | 2G |
| Nutsedge | 9G | 9G | 0 | 0 |
| Crabgrass | 0 | 0 | 0 | 0 |
| Giant Foxtail | 0 | 0 | 0 | 0 |
| Barnyardgrass | 8H | 4G | 0 | 0 |
| Cheatgrass | 7G | 0 | 0 | 0 |
| Wild Oats | 0 | 0 | 0 | 0 |
| Wheat | 7G | 0 | 2G | 0 |
| Corn | 8G | 5G | 2C,8H | 2C,7G |
| Barley | 4G | 0 | 6G | 5G |
| Soybean | 2C,6G | 1H | 2C,7H | 2C,3H |
| Rice | 10E | 9H | 10E | 7G |
| Sorghum | 9H | 9H | 2G | 0 |
| Sugar beet | 9G | 7G | 4C,9G | 7G |
| Cotton | 8G | 6G | 2C,8G | 9G |

## Table A (continued)

| | Compound 7 | | Compound 8 | |
|---|---|---|---|---|
| Rate kg/ha | 0.05 | 0.01 | 0.05 | 0.01 |
| POSTEMERGENCE | | | | |
| Morningglory | 10C | 9C | 10C | 10C |
| Cocklebur | 10C | 2C,8G | 10C | 10C |
| Velvetleaf | 10C | 10C | 10C | 10C |
| Nutsedge | 10C | 7G | 10C | 10C |
| Crabgrass | 2C,6G | 0 | 3H | 0 |
| Giant Foxtail | 2C,6H | 2G | 6G | 5G |
| Barnyardgrass | 3C,7H | 2H | 3C,8H | 4G |
| Cheatgrass | 9C | 5G | 9C | 2C,8G |
| Wild Oats | 2C,5G | 0 | 2G | 0 |
| Wheat | 3C,5G | 0 | 2G | 0 |
| Corn | 9C | 3C,9H | 3C,8H | 5H |
| Barley | 3C,5G | 0 | 2G | 0 |
| Soybean | 4C,9G | 4C,9G | 5C,9G | 5C,9G |
| Rice | 9C | 3C,8G | 8G | 6G |
| Sorghum | 5C,9G | 4C,9G | 9H | 8H |
| Sugar beet | 10C | 9C | 10C | 10C |
| Cotton | 10C | 10C | 10C | 10C |
| PREEMERGENCE | | | | |
| Morningglory | 9G | 9G | 9C | 9G |
| Cocklebur | 9H | 8G | 9H | 9H |
| Velvetleaf | 10C | 9G | 9C | 9G |
| Nutsedge | 8G | 0 | 10E | 10E |
| Crabgrass | 5G | 0 | 0 | 0 |
| Giant Foxtail | 5G | 0 | 5G | 0 |
| Barnyardgrass | 5G | 0 | 6G | 0 |
| Cheatgrass | 10E | 5G | 8G | 7G |
| Wild Oats | 5G | 0 | 0 | 0 |
| Wheat | 0 | 0 | 0 | 0 |
| Corn | 8H | 3C,9H | 3C,7G | 2C,7G |
| Barley | 9G | 7G | 5G | 0 |
| Soybean | 9H | 9H | 9G | 8H |
| Rice | 10E | 9H | 8G | 5G |
| Sorghum | 4C,9G | 9H | 7G | 3G |
| Sugar beet | 9C | 9G | 9C | 5C,9G |
| Cotton | 9G | 9G | 9G | 9G |

Table A (continued)

| | Compound 9 | | Compound 10 | |
|---|---|---|---|---|
| Rate kg/ha | 0.05 | 0.01 | 0.05 | 0.01 |
| POSTEMERGENCE | | | | |
| Morningglory | 10C | 5C,9G | 4C,9G | 2C,4G |
| Cocklebur | 10C | 9H | 5C,9G | 3C,8H |
| Velvetleaf | 10C | 10C | 9C | 2C,7G |
| Nutsedge | 5C,9G | 2C,6G | 0 | 0 |
| Crabgrass | 0 | 0 | 0 | 0 |
| Giant Foxtail | 2C,5G | 0 | 0 | 0 |
| Barnyardgrass | 5H | 0 | 3G | 0 |
| Cheatgrass | 4G | 0 | 0 | 0 |
| Wild Oats | 0 | 0 | 0 | 0 |
| Wheat | 0 | 0 | 0 | 0 |
| Corn | 2C,7H | 0 | 2C,7H | 0 |
| Barley | 0 | 0 | 0 | 0 |
| Soybean | 5H | 5G | 3C,9G | 2G |
| Rice | 3C,8G | 4G | 3G | 0 |
| Sorghum | 8H | 5G | 2C,3G | 0 |
| Sugar beet | 9C | 10C | 9C | 10C |
| Cotton | 6C,9G | 4C,8G | 8G | 5G |
| PREEMERGENCE | | | | |
| Morningglory | 8G | 4G | 7G | 0 |
| Cocklebur | 5G | 8G | 0 | 0 |
| Velvetleaf | 7G | 5G | 0 | 0 |
| Nutsedge | 9G | 0 | 0 | 0 |
| Crabgrass | 0 | 0 | 0 | 0 |
| Giant Foxtail | 5G | 0 | 0 | 0 |
| Barnyardgrass | 4G | 0 | 0 | 0 |
| Cheatgrass | 3G | 0 | 0 | 0 |
| Wild Oats | 0 | 0 | 0 | 0 |
| Wheat | 0 | 0 | 0 | 0 |
| Corn | 7G | 0 | 9H | 2G |
| Barley | 4G | 0 | 0 | 0 |
| Soybean | 2C | 0 | 0 | 0 |
| Rice | 3C,8H | 6G | 8G | 2G |
| Sorghum | 3C,8G | 2G | 0 | 0 |
| Sugar beet | 2C,8G | 8G | 8G | 5G |
| Cotton | 7G | 4H | 4G | 0 |

Table A (continued)

| | Compound 11 | | Compound 12 | |
|---|---|---|---|---|
| Rate kg/ha | 0.05 | 0.01 | 0.05 | 0.01 |
| POSTEMERGENCE | | | | |
| Morningglory | 2C,6G | 0 | 10C | 5C,9G |
| Cocklebur | 4H | 0 | 9C | 9C |
| Velvetleaf | 3H | 0 | 10C | 10C |
| Nutsedge | 0 | 0 | 10C | 6C,9G |
| Crabgrass | 0 | 0 | 2C,5G | 4G |
| Giant Foxtail | 0 | 0 | 9C | 3C,7G |
| Barnyardgrass | 0 | 0 | 9C | 3C,9H |
| Cheatgrass | 0 | 0 | 5C,9G | 5G |
| Wild Oats | 0 | 0 | 4G | 0 |
| Wheat | 0 | 0 | 2G | 0 |
| Corn | 0 | 0 | 9G | 2C,6H |
| Barley | 0 | 0 | 4G | 2C,3G |
| Soybean | 2C,7G | 0 | 9C | 9C |
| Rice | 0 | 0 | 5C,9G | 8G |
| Sorghum | 2G | 0 | 4C,9H | 4C,9H |
| Sugar beet | 4C,9G | 2C,6G | 10C | 9C |
| Cotton | 3C,7G | 3G | 10C | 9C |
| PREEMERGENCE | | | | |
| Morningglory | 0 | 0 | 9G | 3C,9G |
| Cocklebur | 0 | 0 | 9H | 8H |
| Velvetleaf | 0 | 0 | 9G | 9G |
| Nutsedge | 0 | 0 | 10E | 10E |
| Crabgrass | 0 | 0 | 5G | 2G |
| Giant Foxtail | 0 | 0 | 6G | 2G |
| Barnyardgrass | 0 | 0 | 3C,9H | 3C,8H |
| Cheatgrass | 0 | 0 | 8G | 7G |
| Wild Oats | 0 | 0 | 5G | 2C,4G |
| Wheat | 0 | 0 | 3G | 0 |
| Corn | 0 | 0 | 3C,9H | 7G |
| Barley | 0 | 0 | 7G | 6G |
| Soybean | 0 | 0 | 3C,8H | 7H |
| Rice | 0 | 0 | 2C,9G | 3C,8G |
| Sorghum | 0 | 0 | 2C,9H | 3C,8H |
| Sugar beet | 0 | 0 | 9C | 9C |
| Cotton | 0 | 0 | 9G | 9C |

Table A (continued)

| | Compound 13 | | Compound 14 | |
|---|---|---|---|---|
| Rate kg/ha | 0.05 | 0.01 | 0.05 | 0.01 |
| POSTEMERGENCE | | | | |
| Morningglory | 10C | 10C | 10C | 5C,9G |
| Cocklebur | 10C | 9C | 10C | 9C |
| Velvetleaf | 10C | 10C | 9C | 9C |
| Nutsedge | 2C,5G | 2C,5G | 4C,9G | 3C,5G |
| Crabgrass | 0 | 0 | 2G | 0 |
| Giant Foxtail | 2G | 0 | 2G | 2G |
| Barnyardgrass | 2H | 0 | 4C,9H | 3C,8H |
| Cheatgrass | 3G | 0 | 8G | 6G |
| Wild Oats | 0 | 0 | 2C,5G | 0 |
| Wheat | 0 | 0 | 4G | 0 |
| Corn | 3C,8H | 0 | 3C,9H | 2C,7G |
| Barley | 0 | 0 | 2C,5G | 0 |
| Soybean | 9C | 3C,9G | 4C,9G | 2C,8G |
| Rice | 8G | 3G | 9C | 4C,9G |
| Sorghum | 2G | 0 | 4C,9H | 3C,7H |
| Sugar beet | 10C | 10C | 9C | 9C |
| Cotton | 10C | 9C | 9C | 4C,9G |
| PREEMERGENCE | | | | |
| Morningglory | 9C | 8G | 9G | 8G |
| Cocklebur | - | 8H | 9H | - |
| Velvetleaf | 4C,8G | 3H | 4C,9G | 8G |
| Nutsedge | 2C | 0 | 3C,7G | 0 |
| Crabgrass | 0 | 0 | 3G | 0 |
| Giant Foxtail | 0 | 0 | 3C,3H | 0 |
| Barnyardgrass | 0 | 0 | 4C,8H | 3C,7G |
| Cheatgrass | 0 | 0 | 4C,9H | 5G |
| Wild Oats | 0 | 0 | 2C,9G | 0 |
| Wheat | 0 | 0 | 7G | 0 |
| Corn | 2C,8G | 0 | 4C,9H | 3C,7G |
| Barley | 0 | 0 | 8G | 4G |
| Soybean | 8H | 7H | 3C,7H | 3G |
| Rice | 7G | 0 | 3C,8H | 3C,5G |
| Sorghum | 5G | 0 | 3C,9H | 3C,8H |
| Sugar beet | 5C,9G | 4C,9G | 5C,9G | 6G |
| Cotton | 4C,9G | 2C,8G | 4C,9G | 9G |

Table A (continued)

| | Compound 15 | | Compound 16 | |
|---|---|---|---|---|
| Rate kg/ha | 0.05 | 0.01 | 0.05 | 0.01 |
| POSTEMERGENCE | | | | |
| Morningglory | 10C | 4C,9G | 1C | 0 |
| Cocklebur | 9C | 3G | 5G | 0 |
| Velvetleaf | 10C | 4C,9G | 3C,8G | 0 |
| Nutsedge | 4C,9G | 5G | 0 | 0 |
| Crabgrass | 0 | 0 | 5G | 0 |
| Giant Foxtail | 2G | 0 | 3G | 0 |
| Barnyardgrass | 3C,9H | 5G | 0 | 0 |
| Cheatgrass | 4G | 0 | 0 | 0 |
| Wild Oats | 0 | 0 | 0 | 0 |
| Wheat | 0 | 0 | 0 | 0 |
| Corn | 3G | 0 | 0 | 0 |
| Barley | 0 | 0 | 0 | 0 |
| Soybean | 2H,4G | 2G | 1C | 0 |
| Rice | 8G | 4G | 5G | 0 |
| Sorghum | 3C,9H | 6G | 4G | 0 |
| Sugar beet | 9C | 9C | 3C,7G | 1H |
| Cotton | 9C | 5C,9G | 0 | 0 |
| PREEMERGENCE | | | | |
| Morningglory | 9G | 9C | 0 | 0 |
| Cocklebur | 9H | 5H | 2H | - |
| Velvetleaf | 9G | 5H | 0 | 0 |
| Nutsedge | 10E | 0 | 0 | 0 |
| Crabgrass | 0 | 0 | 0 | 0 |
| Giant Foxtail | 0 | 0 | 0 | 0 |
| Barnyardgrass | 3C,7H | 0 | 0 | 0 |
| Cheatgrass | 0 | 0 | 0 | 0 |
| Wild Oats | 0 | 0 | 0 | 0 |
| Wheat | 0 | 0 | 0 | 0 |
| Corn | 5G | 0 | 0 | 0 |
| Barley | 3G | 0 | 0 | 0 |
| Soybean | 0 | 0 | 0 | 0 |
| Rice | 2C,7G | 0 | 5G | 0 |
| Sorghum | 3C,7G | 2C,6G | 3G | 0 |
| Sugar beet | 4C,9G | 8G | 5G | 0 |
| Cotton | 9G | 8G | 0 | 0 |

Table A (continued)

| | Compound 17 | | Compound 18 | |
|---|---|---|---|---|
| Rate kg/ha | 0.05 | 0.01 | 0.05 | 0.01 |
| POSTEMERGENCE | | | | |
| Morningglory | 3C,8H | 3C,6G | 3C,8G | 2G |
| Cocklebur | 9H | 2H | 3H | 2H |
| Velvetleaf | 5C,9G | 5C,9G | 9C | 2C,7H |
| Nutsedge | 3C,6G | 0 | 9G | 0 |
| Crabgrass | 2C,7G | 3G | 0 | 0 |
| Giant Foxtail | 7G | 5G | 3G | 0 |
| Barnyardgrass | 9H | 3H | 4H | 0 |
| Cheatgrass | 5G | 3G | 2G | 0 |
| Wild Oats | 6G | 0 | 0 | 0 |
| Wheat | 2G | 0 | 0 | 0 |
| Corn | 9G | 3C,7H | 2C,5H | 5G |
| Barley | 3G | 0 | 0 | 0 |
| Soybean | 3C,8H | 3C,3G | 4C,9G | 2C,5G |
| Rice | 8G | 6G | 9G | 2G |
| Sorghum | 2C,8H | 2C,4G | 2C,2H | 0 |
| Sugar beet | 3C,9H | 3C,7G | 5C,9G | 2C,4G |
| Cotton | 4C,9G | 3C,8G | 2C,5G | 0 |
| PREEMERGENCE | | | | |
| Morningglory | 3G | 3G | 8G | 0 |
| Cocklebur | - | 2H | 5G | 0 |
| Velvetleaf | 2G | 0 | 0 | 0 |
| Nutsedge | 5G | 0 | 0 | 0 |
| Crabgrass | 5G | 3G | 0 | 0 |
| Giant Foxtail | 7G | 3G | 0 | 0 |
| Barnyardgrass | 5G | 0 | 0 | 0 |
| Cheatgrass | 3G | 0 | 0 | 0 |
| Wild Oats | 3G | 0 | 2G | 0 |
| Wheat | 0 | 0 | 0 | 0 |
| Corn | 2C,8G | 2C,2G | 2G | 0 |
| Barley | 0 | 0 | 0 | 0 |
| Soybean | 2C,5H | 2C,2H | 1C,5G | 0 |
| Rice | 8G | 4G | 7G | 0 |
| Sorghum | 3C,6G | 3G | 2C,5G | 0 |
| Sugar beet | 5H | 0 | 3H | 0 |
| Cotton | 2G | 0 | 3G | 0 |

Table A (continued)

| | Compound 19 | |
|---|---|---|
| Rate kg/ha | 0.05 | 0.01 |
| POSTEMERGENCE | | |
| Morningglory | 1C | 1C |
| Cocklebur | 2C,5H | 1H |
| Velvetleaf | 3C,9G | 4G |
| Nutsedge | 0 | 0 |
| Crabgrass | 0 | 0 |
| Giant Foxtail | 0 | 0 |
| Barnyardgrass | 0 | 0 |
| Cheatgrass | 0 | 0 |
| Wild Oats | 0 | 0 |
| Wheat | 0 | 0 |
| Corn | 2C,8G | 3G |
| Barley | 0 | 0 |
| Soybean | 3C,7G | 2H |
| Rice | 0 | 0 |
| Sorghum | 2C | 0 |
| Sugar beet | 4C,9G | 3C,6G |
| Cotton | 3C,6G | 0 |
| PREEMERGENCE | | |
| Morningglory | 0 | 0 |
| Cocklebur | 0 | 0 |
| Velvetleaf | 0 | 0 |
| Nutsedge | 0 | 0 |
| Crabgrass | 0 | 0 |
| Giant Foxtail | 0 | 0 |
| Barnyardgrass | 0 | 0 |
| Cheatgrass | 0 | 0 |
| Wild Oats | 0 | 0 |
| Wheat | 0 | 0 |
| Corn | 0 | 0 |
| Barley | 0 | 0 |
| Soybean | 0 | 0 |
| Rice | 0 | 0 |
| Sorghum | 0 | 0 |
| Sugar beet | 0 | 0 |
| Cotton | 0 | 0 |

Table A (continued)

| | Compound 20 | | Compound 21 | |
|---|---|---|---|---|
| Rate kg/ha | 0.05 | 0.01 | 0.05 | 0.01 |
| POSTEMERGENCE | | | | |
| Morningglory | 2G | 0 | 4C,9H | 3G |
| Cocklebur | 2C,8G | 6G | 8H | 2G |
| Velvetleaf | 10C | 6G | 6C,9G | 10C |
| Nutsedge | 7G | 5G | 8G | 3C,8G |
| Crabgrass | 4G | 5G | 4G | 5G |
| Giant Foxtail | 2G | 4G | 2C,5H | 2C,6H |
| Barnyardgrass | 9H | 9H | 4C,9H | 7H |
| Cheatgrass | 8G | 7G | 3G | 6G |
| Wild Oats | 2C,7G | 2C,3G | 0 | 0 |
| Wheat | 4G | 0 | 0 | 2G |
| Corn | 9G | 5H | 5G | 1H |
| Barley | 4C,9G | 3C,7G | 3G | 0 |
| Soybean | 5C,9G | 3C,6H | 9C | 5C,9G |
| Rice | 5C,9G | 4C,9H | 4G | 3C,6G |
| Sorghum | 9H | 3C,9H | 2G | 2C |
| Sugar beet | 10C | 9H | 9C | 9C |
| Cotton | 9C | 3C,8H | 9C | 5C,9G |
| PREEMERGENCE | | | | |
| Morningglory | 0 | 0 | 5G | 3G |
| Cocklebur | 4G | 0 | 8H | - |
| Velvetleaf | 4C,9G | 5H | 5G | 3H |
| Nutsedge | 5G | 0 | 9G | 9G |
| Crabgrass | 0 | 0 | 0 | 0 |
| Giant Foxtail | 2G | 0 | 5G | 0 |
| Barnyardgrass | 9H | 3C,8H | 3C,9H | 5G |
| Cheatgrass | 8G | 7G | 7G | 0 |
| Wild Oats | 5G | 3G | 2G | 0 |
| Wheat | 6G | 0 | 2G | 0 |
| Corn | 3C,7G | 3C,3H | 2C,8G | 3G |
| Barley | 8G | 5G | 1C | 0 |
| Soybean | 1H | 1C | 3C,7H | 2C,2H |
| Rice | 9H | 9H | 9H | 5G |
| Sorghum | 9H | 9H | 6G | 0 |
| Sugar beet | 8G | 3C,5H | 9G | 5G |
| Cotton | 7G | 2G | 3G | 2G |

Table A (continued)

| | Compound 22 | | Compound 23 | |
|---|---|---|---|---|
| Rate kg/ha | 0.05 | 0.01 | 0.05 | 0.01 |
| POSTEMERGENCE | | | | |
| Morningglory | 3C,8G | 3H | 10C | 2C,6G |
| Cocklebur | 8H | 1H | 10C | 5H |
| Velvetleaf | 5C,9G | 5G | 10C | 8G |
| Nutsedge | 8G | 7G | 0 | 4G |
| Crabgrass | 3G | 0 | 4G | 0 |
| Giant Foxtail | 4G | 0 | 2C,7G | 0 |
| Barnyardgrass | 3H | 0 | 3C,9H | 5H |
| Cheatgrass | 2G | 0 | 0 | 0 |
| Wild Oats | 0 | 0 | 0 | 0 |
| Wheat | 0 | 0 | 0 | 0 |
| Corn | 0 | 0 | 3C,9H | 8H |
| Barley | 0 | 0 | 0 | 0 |
| Soybean | 5H | 0 | 5C,9G | 5C,9G |
| Rice | 8G | 5G | 5C,9G | 5G |
| Sorghum | 2C,3G | 0 | 3C,9H | 2G |
| Sugar beet | 9C | 4C,9G | 10C | 9C |
| Cotton | 3C,8G | 7G | 9C | 5C,9G |
| PREEMERGENCE | | | | |
| Morningglory | 5G | 3G | 9G | 5G |
| Cocklebur | 2H | 0 | - | 6H |
| Velvetleaf | 5G | 0 | 4C,9G | 5G |
| Nutsedge | 10E | 0 | 0 | 0 |
| Crabgrass | 0 | 0 | 0 | 0 |
| Giant Foxtail | 0 | 0 | 3G | 0 |
| Barnyardgrass | 3G | 3G | 3C,7G | 0 |
| Cheatgrass | 2G | 0 | 0 | 0 |
| Wild Oats | 0 | 0 | 0 | 0 |
| Wheat | 0 | 0 | 0 | 0 |
| Corn | 0 | 0 | 3C,8G | 2C,7G |
| Barley | 0 | 0 | 5G | 0 |
| Soybean | 0 | 0 | 3C,9H | 2C,5H |
| Rice | 5G | 4G | 9H | 6G |
| Sorghum | 5G | 2G | 3C,9G | 3C,6G |
| Sugar beet | 7G | 5G | 9G | 9G |
| Cotton | 2G | 0 | 2C,8G | 8G |

Table A (continued)

| | Compound 24 | | Compound 25 | |
|---|---|---|---|---|
| Rate kg/ha | 0.05 | 0.01 | 0.05 | 0.01 |
| POSTEMERGENCE | | | | |
| Morningglory | 10C | 5C,9G | 2C,8G | 1H |
| Cocklebur | 1H | 6G | 2C,9G | 8G |
| Velvetleaf | 10C | 5C,9H | 10C | 2C,8G |
| Nutsedge | 4G | 0 | 9G | 2C,9G |
| Crabgrass | 0 | 0 | 3G | 0 |
| Giant Foxtail | 2G | 0 | 5G | 0 |
| Barnyardgrass | 7H | 0 | 6G | 0 |
| Cheatgrass | 0 | 0 | 7G | 2G |
| Wild Oats | 0 | 0 | 0 | 0 |
| Wheat | 0 | 0 | 0 | 0 |
| Corn | 3C,9H | 0 | 0 | 0 |
| Barley | 0 | 0 | 0 | 0 |
| Soybean | 9C | 4C,9G | 2C,8H | 2C,6H |
| Rice | 7G | 2G | 8G | 3G |
| Sorghum | 2C,4H | 0 | 1C,6G | 0 |
| Sugar beet | 10C | 9C | 10C | 9C |
| Cotton | 5C,9G | 3C,7H | 10C | 10C |
| PREEMERGENCE | | | | |
| Morningglory | 9G | 5H | 8G | 5G |
| Cocklebur | 4G | 0 | 2C,7G | 0 |
| Velvetleaf | 3C,6G | 1C | 3G | 2G |
| Nutsedge | 4G | 0 | 9G | 5G |
| Crabgrass | 4G | 0 | 0 | 0 |
| Giant Foxtail | 5G | 0 | 7G | 5G |
| Barnyardgrass | 3G | 0 | 2C,6G | 2G |
| Cheatgrass | 0 | 0 | 6G | 4G |
| Wild Oats | 2G | 0 | 0 | 0 |
| Wheat | 0 | 0 | 0 | 0 |
| Corn | 2C,7G | 3G | 1C | 0 |
| Barley | 0 | 0 | 0 | 0 |
| Soybean | 3C,7H | 2C,2H | 2G | 0 |
| Rice | 3C,8G | 3G | 2C,7G | 0 |
| Sorghum | 2C,5G | 1C | 2C,6G | 0 |
| Sugar beet | 2C,9G | 3C,5H | 8G | 2C,6G |
| Cotton | 2C,9G | 3H | 6G | 5G |

Table A (continued)

| | Compound 26 | | Compound 27 | |
|---|---|---|---|---|
| Rate kg/ha | 0.05 | 0.01 | 0.05 | 0.01 |
| POSTEMERGENCE | | | | |
| Morningglory | 3C,6G | 4G | 1H | 0 |
| Cocklebur | 3C,8H | 3H | 1H | 0 |
| Velvetleaf | 5C,9G | 7H | 1H | 0 |
| Nutsedge | 4C,9G | 3C,7G | 2C,6G | 3G |
| Crabgrass | 3C,7G | 6G | 3G | 0 |
| Giant Foxtail | 4C,9G | 8G | 2G | 0 |
| Barnyardgrass | 5C,9H | 9H | 3G | 0 |
| Cheatgrass | 7G | 2G | 0 | 0 |
| Wild Oats | 3C,9G | 3C,5G | 0 | 0 |
| Wheat | 3C,6G | 2G | 0 | 0 |
| Corn | 9H | 9H | 5H | 0 |
| Barley | 8G | 3C,8G | 0 | 0 |
| Soybean | 3C,8H | 3C,3H | 2H | 0 |
| Rice | 5C,9G | 4C,8H | 3C,8G | 3G |
| Sorghum | 4C,9G | 4C,8G | 3C,8G | 3G |
| Sugar beet | 4C,9G | 4C,8H | 3G | 2G |
| Cotton | 4C,9H | 3C,6G | 0 | 0 |
| PREEMERGENCE | | | | |
| Morningglory | 9G | 8G | 3H | 0 |
| Cocklebur | 9H | 9H | 6G | 0 |
| Velvetleaf | 3C,9G | 8G | 5G | 0 |
| Nutsedge | 3C,9G | 3G | 3C,8G | 0 |
| Crabgrass | 6G | 5G | 0 | 0 |
| Giant Foxtail | 3C,8G | 5G | 0 | 0 |
| Barnyardgrass | 3C,9H | 3C,7G | 0 | 0 |
| Cheatgrass | 6G | 4G | 0 | 0 |
| Wild Oats | 2C,8G | 3C,6G | 0 | 0 |
| Wheat | 7G | 5G | 0 | 0 |
| Corn | 3C,9H | 2C,7G | 0 | 0 |
| Barley | 9G | 8G | 0 | 0 |
| Soybean | 7H | 7H | 2H | 0 |
| Rice | 9H | 9H | 0 | 0 |
| Sorghum | 3C,9H | 3C,9H | 2C,5G | 0 |
| Sugar beet | 3C,9G | 3C,8G | 8G | 4G |
| Cotton | 9G | 9G | 7G | 0 |

Table A (continued)

| | Compound 28 | | Compound 29 | |
|---|---|---|---|---|
| Rate kg/ha | 0.05 | 0.01 | 0.05 | 0.01 |
| POSTEMERGENCE | | | | |
| Morningglory | 3G | 0 | 0 | 3G |
| Cocklebur | 5G | 0 | 0 | 0 |
| Velvetleaf | 3C,8H | 3C,6G | 3C,6H | 2H |
| Nutsedge | 0 | 0 | 0 | 0 |
| Crabgrass | 0 | 0 | 0 | 0 |
| Giant Foxtail | 0 | 0 | 0 | 0 |
| Barnyardgrass | 5C,9H | 6G | 5G | 0 |
| Cheatgrass | 0 | 0 | 0 | 0 |
| Wild Oats | 0 | 0 | 0 | 0 |
| Wheat | 0 | 0 | 0 | 0 |
| Corn | 2C,9H | 6H | 2H | 0 |
| Barley | 0 | 0 | 0 | 0 |
| Soybean | 3C,4H | 1H | 3C,3H | 1C,1H |
| Rice | 4G | 2G | 3G | 0 |
| Sorghum | 4C,7G | 5G | 2G | 0 |
| Sugar beet | 3C,8H | 3C,7G | 3C,7G | 4G |
| Cotton | 2C,5G | 2C,4G | 3C,3H | 2G |
| PREEMERGENCE | | | | |
| Morningglory | 0 | 0 | 3G | 0 |
| Cocklebur | 0 | 0 | - | 3G |
| Velvetleaf | 0 | 0 | 0 | 0 |
| Nutsedge | 0 | 0 | 0 | 0 |
| Crabgrass | 0 | 0 | 0 | 0 |
| Giant Foxtail | 0 | 0 | 0 | 0 |
| Barnyardgrass | 6G | 0 | 5G | 0 |
| Cheatgrass | 0 | 0 | 0 | 0 |
| Wild Oats | 0 | 0 | 0 | 0 |
| Wheat | 0 | 0 | 0 | 0 |
| Corn | 3C,6G | 2C,2G | 4G | 0 |
| Barley | 0 | 0 | 0 | 0 |
| Soybean | 0 | 0 | 2C | 0 |
| Rice | 6G | 0 | 1C | 0 |
| Sorghum | 3C,9H | 2G | 2C,4G | 0 |
| Sugar beet | 4G | 4G | 3H | 3G |
| Cotton | 2G | 0 | 3G | 0 |

## Claims

**Claims for the following Contracting States : BE, CH, DE, FR, GB, IT, LI, LU, NL, SE**

**1.** A compound of the formula

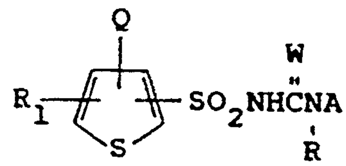

I

wherein

R is H or $CH_3$;

$R_1$ is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, CN, $CO_2R_c$, $C_1$-$C_3$ haloalkoxy 1 or 3 $C_1$-$C_3$ haloalkylthio;

$R_a$ is H, $C_1$-$C_4$ alkyl, $C_2$-$C_3$ cyanoalkyl, methoxy, ethoxy or $C_3$-$C_4$ alkenyl;

$R_b$ is H or $C_1$-$C_3$ alkyl; or

$R_a$ and $R_b$ may be taken together to form $(CH_2)_3$, $(CH_2)_4$, $(CH_2)_5$ or $CH_2CH_2OCH_2CH_2$;

$R_c$ is $C_1$-$C_4$ alkyl, $C_3$-$C_4$ alkenyl, $C_3$-$C_4$ alkynyl, $C_2$-$C_4$ haloalkyl, $C_2$-$C_3$ cyanoalkyl, cyclopropylmethyl or $C_2$-$C_4$ alkoxyalkyl;

Q is $ER_2$, $NR_3R_4$,

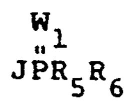

$OSO_2R_7$, $C_1$-$C_4$ haloalkyl, CN, $SO_2NHR_{21}$,

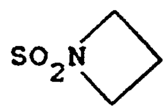
,
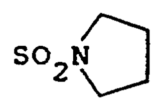
,

$SO_2NR_{22}NR_{23}R_{24}$ or $C_1$-$C_4$ alkyl substituted with $R_8$;

E is O, S, SO or $SO_2$;

W and $W_1$ are independently O or S;

J is O, S, NH, $NCH_3$, $CH_2$ or a single bond;

$R_2$ is $C_1$-$C_6$ alkyl substituted with $R_8$, $C_2$-$C_6$ alkenyl substituted with $R_8$, $C_3$-$C_6$ alkynyl, $C_3$-$C_6$ alkynyl substituted with $R_8$, $C_1$-$C_6$ haloalkyl, $C_2$-$C_6$ haloalkenyl or $C_3$-$C_6$ haloalkynyl;

$R_3$ is $C_1$-$C_4$ alkyl;

$R_4$ is H or $C_1$-$C_4$ alkyl; or

$R_3$ and $R_4$ may be taken together to form $(CH_2)_4$, $(CH_2)_5$ or $CH_2CH_2OCH_2CH_2$;

$R_5$ and $R_6$ are independently $C_1$-$C_2$ alkyl, $C_1$-$C_2$ alkoxy, $C_1$-$C_2$ alkylthio, $C_1$-$C_2$ alkylamino or di($C_1$-$C_2$ alkyl)amino;

$R_7$ is $C_1$-$C_4$ alkyl, $C_1$-$C_4$ haloalkyl, $C_3$-$C_4$ alkenyl, $C_3$-$C_4$ haloalkenyl, $C_3$-$C_4$ alkynyl, $C_3$-$C_4$ haloalkynyl or $NR_{19}R_{20}$;

$R_8$ is $OR_9$, $S(O)_nR_{10}$, $CO_2R_{10}$, $SO_2NR_{11}R_{12}$, $NR_{11}R_{12}$, $CONR_{11}R_{12}$, $C(O)R_{13}$,

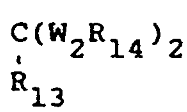
,
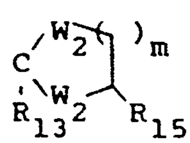
.

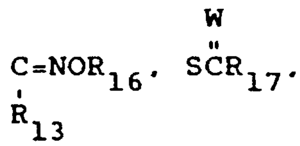

| | |
|---|---|
| | CN, SCN, SH, $NO_2$ or $N_3$; |
| $R_9$ | is H, $C_1$-$C_4$ alkyl, $C_3$-$C_4$ alkenyl, $C_3$-$C_4$ alkynyl, $C_1$-$C_4$ haloalkyl, $C_3$-$C_4$ haloalkenyl, $C_3$-$C_4$ haloalkynyl, $C_2$-$C_4$ alkylcarbonyl, $C_1$-$C_4$ alkylsulfonyl, $C_2$-$C_4$ alkoxyalkyl, $C_2$-$C_4$ alkylthioalkyl or $C_2$-$C_4$ cyanoalkyl; |
| $R_{10}$ | is H, $C_1$-$C_4$ alkyl, $C_3$-$C_4$ alkenyl, $C_3$-$C_4$ alkynyl, $C_1$-$C_4$ haloalkyl, $C_3$-$C_4$ haloalkenyl, $C_3$-$C_4$ haloalkynyl, $C_2$-$C_4$ alkoxyalkyl, $C_2$-$C_4$ alkylthioalkyl or $C_2$-$C_4$ cyanoalkyl; |
| $R_{11}$ | is H or $C_1$-$C_3$ alkyl; |
| $R_{12}$ | is H, $C_1$-$C_4$ alkyl, $C_1$-$C_4$ haloalkyl, $C_3$-$C_6$ cycloalkyl, $C_2$-$C_4$ alkoxyalkyl, $C_2$-$C_4$ alkylthioalkyl, $C_2$-$C_4$ cyanoalkyl, $C_1$-$C_3$ alkoxy, $C_3$-$C_4$ alkenyl or $C_3$-$C_4$ alkynyl; or |
| $R_{11}$ and $R_{12}$ | may be taken together to form $(CH_2)_3$, $(CH_2)_4$, $(CH_2)_5$ or $CH_2CH_2OCH_2CH_2$; |
| $R_{13}$ | is H, $C_1$-$C_4$ alkyl or $C_1$-$C_4$ haloalkyl; |
| $R_{14}$ | is $C_1$-$C_2$ alkyl; |
| $R_{15}$ | is H or $CH_3$; |
| $R_{16}$ | is H, $C_1$-$C_4$ alkyl, $C_3$-$C_4$ alkenyl or $C_3$-$C_4$ alkynyl; |
| $R_{17}$ | is $C_1$-$C_4$ alkyl, $C_1$-$C_4$ alkoxy, $C_1$-$C_4$ alkylthio, $C_1$-$C_4$ alkyl amino or di($C_1$-$C_4$ alkyl)-amino; |
| $R_{18}$ | is F, Cl, Br, CN, $CH_3$, $OCH_3$, $SCH_3$ or $NO_2$; |
| $R_{19}$ | is H, $C_1$-$C_3$ alkyl, $C_3$-$C_4$ alkenyl or $C_3$-$C_4$ alkynyl; or |
| $R_{20}$ | is H or $C_1$-$C_3$ alkyl; or |
| $R_{19}$ and $R_{20}$ | may be taken together to form $(CH_2)_4$, $(CH_2)_5$ or $CH_2CH_2OCH_2CH_2$; |
| $R_{21}$ | is $C_1$-$C_4$ alkyl, $C_1$-$C_3$ haloalkyl, $C_2$-$C_3$ cyanoalkyl, cyclopropyl, $C_3$-$C_4$ alkenyl, $C_3$-$C_4$ alkynyl, $C_2$-$C_4$ alkoxyalkyl or $C_1$-$C_2$ alkoxy; |
| $R_{22}$ | is H or $C_1$-$C_4$ alkyl; |
| $R_{23}$ | is H or $C_1$-$C_4$ alkyl; |
| $R_{24}$ | is H, $C_1$-$C_4$ alkyl, $C_3$-$C_4$ alkenyl, $C_3$-$C_4$ alkynyl, $C_1$-$C_4$ haloalkyl or phenyl which may be optionally substituted with $R_{25}$; or |
| $R_{23}$ and $R_{24}$ | may be taken together to form $(CH_2)_4$, $(CH_2)_5$ or $CH_2CH_2OCH_2CH_2$; |
| $R_{25}$ | is H, $CH_3$, Cl, F, Br, $NO_2$, $CF_3$, CN or $OCH_3$; |
| m | is 1 or 2; |
| n | is 0, 1 or 2; |
| $W_2$ and $W_3$ | are independently O or S; |

A is 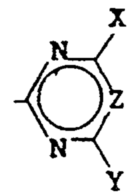 , 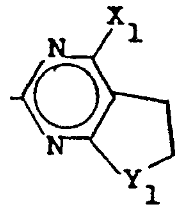 , 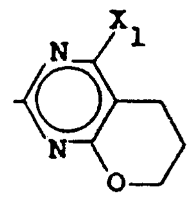 ,

A-1 A-2 A-3

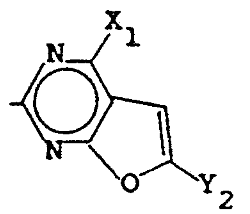 , 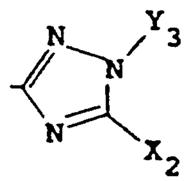 , 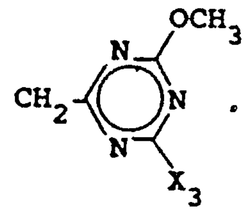 ,

A-4 A-5 A-6

or 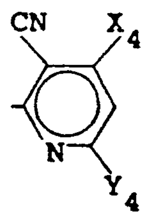 ;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino or di($C_1$-$C_3$ alkyl)amino;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_2$-$C_5$ alkylsulfinylalkyl, $C_2$-$C_5$ alkylsulfonylalkyl, $C_1$-$C_4$ haloalkyl, $C_4$-$C_5$ cycloalkyl, $C_2$-$C_4$ alkynyl, cyano,

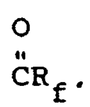 , 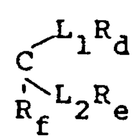 , 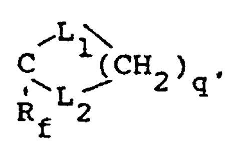 ,

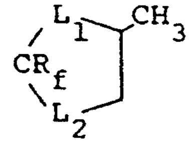

or $N(OCH_3)CH_3$;

q is 2 or 3;

$L_1$ and $L_2$ are independently O or S;

$R_d$ and $R_e$ are independently $C_1$-$C_2$ alkyl;

| | |
|---|---|
| $R_f$ | is H or $CH_3$; |
| Z | is CH or N; |
| Y | $_1$ is O or $CH_2$; |
| $X_1$ | is $CH_3$, $OCH_3$, $OC_2H_5$ or $OCF_2H$; |
| $Y_2$ | is H or $CH_3$; |
| $X_2$ | is $CH_3$, $OCH_3$ or $SCH_3$; |
| $Y_3$ | is $CH_3$, $CH_2CH_3$ or $CH_2CF_3$; |
| $X_3$ | is $CH_3$ or $OCH_3$; |
| $X_4$ | is $CH_3$, $OCH_3$, $OC_2H_5$, $CH_2OCH_3$ or Cl; and |
| $Y_4$ | is $CH_3$, $OCH_3$, $OC_2H_5$ or Cl; |

and their agriculturally suitable salts; provided that

a) when X is Cl, F, Br or I, then Z is CH and Y is $OCH_3$, $OC_2H_5$, $N(OCH_3)CH_3$, $NHCH_3$, $N(CH_3)_2$ or $OCF_2H$;

b) when X or Y is $C_1$ haloalkoxy, then Z is CH;

c) when Q is $CF_3$, then A is A-4, A-5, A-6 or A-7;

d) when E is O or S and $R_9$ is H, then $R_2$ is other than $CH_2OR_9$;

e) when W is S, then A is A-1, R is H and Y is $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$ or

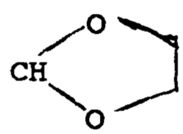 ;

f) the total number of carbon atoms in $R_1$ and Q is less than or equal to 10;

g) when Q is $C_1$-$C_4$ haloalkyl or

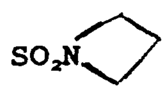 ,

then X and Y are other than $OCF_2H$ or $SCF_2H$;

h) $X_4$ and $Y_4$ cannot simultaneously be Cl;

i) the substituent Q and the sulfonylurea bridge are on adjacent carbon atoms;

j) the total number of carbon atoms in $R_{22}$, $R_{23}$ and $R_{24}$ is less than or equal to 10;

k) when $R_{21}$ is $C_1$-$C_4$ alkyl, $C_2$-$C_3$ cyanoalkyl, $C_1$-$C_2$ alkoxy or $C_3$-$C_4$ alkenyl, then X and Y are other than $OCF_2H$ or $SCF_2H$;

l) when Y is CN and $R_1$ is H, F, Cl or $CH_3$ then $R_{21}$ is other than $C_1$-$C_3$ alkyl;

m) when $R_{21}$ is $C_1$-$C_4$ alkyl, then Y is other than $C_1$-$C_3$ alkylamino, di($C_1$-$C_3$ alkyl)amino, $N(OCH_3)$-$CH_3$ or $C_2$-$C_4$ alkynyl and X is other than halogen, $C_1$-$C_3$ alkylamino or di($C_1$-$C_3$ alkyl)amino.

**2.** Compounds of Claim 1, wherein

| | |
|---|---|
| R | is H; |
| W | is Q; |
| $R_1$ | is H, $CH_3$ or Cl; |
| X | is $CH_3$, $OCH_3$, $OCH_2CH_3$, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and |
| Y | is H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C\equiv CH$, $CH_2OCH_2CH_3$, $OCH_2CH_2OCH_3$, $CH_2SCH_3$, $CH_2SCH_2CH_3$, |

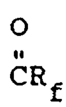 , 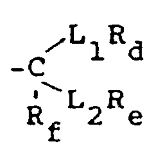 , 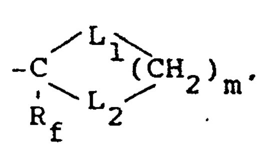

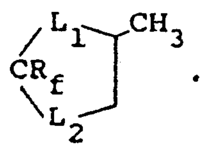

$OCF_2H$, $SCF_2H$, C≡CH or $C≡CCH_3$.

**3.** Compounds of Claim 2 where A is A-1.

**4.** Compounds of Claim 3 where the sulfonylurea bridge is attached to the 2-position of the thiophene ring.

**5.** Compounds of Claim 4 where

Q is $ER_2$,

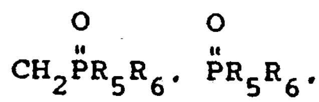

$C_1$-$C_2$ alkyl substituted with $R_8$, $C_1$-$C_2$ haloalkyl, CN, $SO_2NHC_1$-$C_2$ alkyl, $SO_2NH$cyclopropyl or

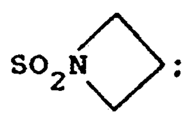

$R_2$ is $C_1$-$C_3$ alkyl substituted with $R_8$, $C_1$-$C_3$ haloalkyl, $C_2$-$C_3$ haloalkenyl or $C_3$-$C_4$ alkynyl;

$R_5$ and $R_6$ are independently $CH_3$, $OCH_3$ or $SCH_3$;

$R_8$ is $OR_9$, $C_1$-$C_2$ alkylthio, $C_1$-$C_2$ alkylsulfinyl, $C_1$-$C_2$ alkylsulfonyl, $CO_2(C_1$-$C_2$ alkyl), $SO_2N(C_1$-$C_2$ alkyl$)_2$, $C(O)CH_3$, CN or $C(O)N(CH_3)_2$;

$R_9$ is H, $C_1$-$C_2$ alkyl or $C_2$-$C_3$ cyanoalkyl.

**6.** Compounds of claim 5 where

X is $CH_3$, $OCH_3$, $OCH_2CH_3$, Cl, $OCF_2H$ or $OCH_2CF_3$;

Y is $CH_3$, $OCH_3$, $C_2H_5$, $CH_2OCH_3$, $NHCH_3$ or $CH(OCH_3)_2$;

Q is $ER_2$, $C_1$-$C_2$ alkyl substituted with $R_8$ or $C_1$-$C_2$ haloalkyl, CN or $SO_2NHC_1$-$C_2$ alkyl;

E is S or $SO_2$;

$R_2$ is $C_1$-$C_2$ alkyl substituted with $R_8$ or $C_1$-$C_2$ haloalkyl;

$R_8$ is $CH_3O$, $CH_3CH_2O$, $CH_3S$, $CH_3SO_2$, $SO_2N(CH_3)_2$ or $CO_2CH_3$.

**7.** Compounds of Claim 3 where the sulfonylurea bridge is attached to the 3-position of the thiophene ring.

**8.** Compounds of Claim 7 where

Q is $ER_2$,

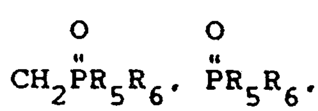

$C_1$-$C_2$ alkyl substituted with $R_8$, $C_1$-$C_2$ haloalkyl, CN, $SO_2NHC_1$-$C_2$ alkyl, $SO_2NH$cyclopropyl or

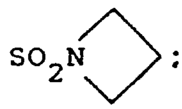

| | |
|---|---|
| $R_2$ | is $C_1$-$C_3$ alkyl substituted with $R_8$, $C_1$-$C_3$ haloalkyl, $C_2$-$C_3$ haloalkenyl or $C_3$-$C_4$ alkynyl; |
| $R_5$ and $R_6$ | are independently $CH_3$, $OCH_3$ or $SCH_3$; |
| $R_8$ | is $OR_9$, $C_1$-$C_2$ alkylthio, $C_1$-$C_2$ alkylsulfinyl, $C_1$-$C_2$ alkylsulfonyl, $CO_2(C_1$-$C_2$ alkyl), $SO_2N(C_1$-$C_2$ alkyl$)_2$, $C(O)CH_3$, CN or $C(O)N(CH_3)_2$; |
| $R_9$ | is H, $C_1$-$C_2$ alkyl or $C_2$-$C_3$ cyanoalkyl. |

**9.** Compounds of Claim 8 where

| | |
|---|---|
| X | is $CH_3$, $OCH_3$, $OCH_2CH_3$, Cl, $OCF_2H$ or $OCH_2CF_3$; |
| Y | is $CH_3$, $OCH_3$, $C_2H_5$, $CH_2OCH_3$, $NHCH_3$ or $CH(OCH_3)_2$; |
| Q | is $ER_2$, $C_1$-$C_2$ alkyl substituted with $R_8$ or $C_1$-$C_2$ haloalkyl, CN or $SO_2NHC_1$-$C_2$ alkyl; |
| E | is S or $SO_2$; |
| $R_2$ | is $C_1$-$C_2$ alkyl substituted with $R_8$ or $C_1$-$C_2$ haloalkyl; |
| $R_8$ | is $CH_3O$, $CH_3CH_2O$, $CH_3S$, $CH_3SO_2$, $SO_2N(CH_3)_2$ or $CO_2CH_3$. |

**10.** The compound of claim 1 which is N-[(4,6-dimethoxypyrimidin-2-yl)aminocarbonyl]-2-(methoxymethyl)-3-thiophenesulfonamide.

**11.** The compound of Claim 1 which is N-[(4-methoxy-6-methyl-1,3,5-triazin-2-yl)aminocarbonyl]-2-(methoxymethyl)-3-thiophenesulfonamide.

**12.** The compound of Claim 1 which is N-[(4-methoxy-6-methylpyrimidin-2-yl)aminocarbonyl]-4-cyano-3-thiophenesulfonamide.

**13.** The compound of Claim 1 which is N-[(4,6-dimethoxypyrimidin-2-yl)aminocarbonyl]-3-(2-methoxyethyl)-2-thiophenesulfonamide.

**14.** A composition suitable for controlling the growth of undesired vegetation which comprises an effective amount of a compound of any of claims 1 to 13 and at least one of the following: surfactant, solid or liquid diluent.

**15.** A method for controlling the growth of undesired vegetation which comprises applying to the locus to be protected an effective amount of a compound of any of claims 1 to 13.

**16.** A method for regulating the growth of plants which comprises applying to the locus of such plants an effective but substantially non-phytotoxic amount of a plant growth regulant selected from compounds of any of claims 1 to 13.

**17.** A process for the preparation of a compound of claim 1, which comprises:
(a) reacting a sulfonamide of formula

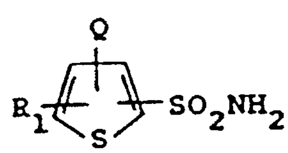

(2)

with an appropriate methylcarbamate of formula

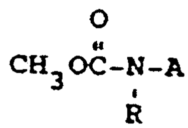

(3)

(b) reacting a sulfonylcarbamate of formula

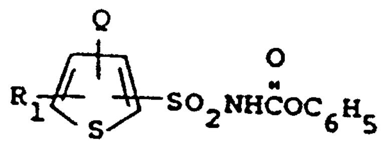

(4)

with an appropriate heterocyclic amine of formula

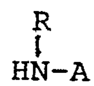

(5)

(c) in a compound of general formula

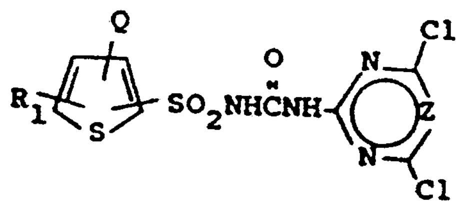

(7)

replacing one chlorine by $OCH_3$ and/or replacing one chlorine by OY', where Y' is $CH_3$, $C_2H_5$ or $CH_2CF_3$, by methods known per se;

(d) reacting a thienylsulfonylisocyanate of formula

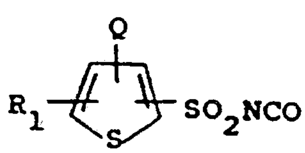

(8)

with said heterocyclic amine (5); provided that $R_a$, $R_b$, $R_4$, $R_9$, $R_{11}$, $R_{12}$, $R_{19}$ and $R_{20}$ are not H; J is not NH and $R_5$, $R_6$ and $R_7$ are not alkylamino; or

(e) reacting said sulfonamide (2) with a heterocyclic carbamate of formula

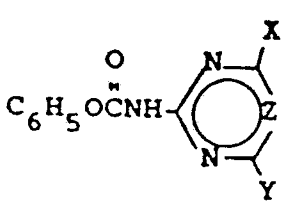

(10)

## Revendications

## Revendications pour les Etats contractants suivants : BE, CH, DE, FR, GB, IT, LI, LU, NL, SE

**1.** Un composé de la formule

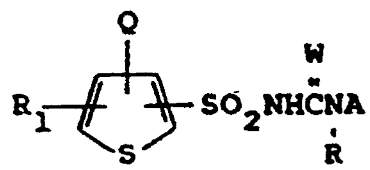

I

dans laquelle

- R — est H ou $CH_3$ ;
- $R_1$ — est H, un groupe alkyle en $C_1$-$C_3$, halogénalkyle en $C_1$-$C_3$, halogéno, nitro, alcoxy en $C_1$-$C_3$, $SO_2NR_aR_b$, alkylthio en $C_1$-$C_3$, alkylsulfinyle en $C_1$-$C_3$, alkylsulfonyle en $C_1$-$C_3$, CN, $CO_2R_c$, halogénalcoxy en $C_1$-$C_3$ ou halogénalkylthio en $C_1$-$C_3$ ;
- $R_a$ — est H, un groupe alkyle en $C_1$-$C_4$, cyanalkyle en $C_2$-$C_3$, méthoxy, éthoxy ou alcényle en $C_3$-$C_4$ ;
- $R_b$ — est H ou un groupe alkyle en $C_1$-$C_3$ ; ou bien
- $R_a$ et $R_b$ — peuvent être pris ensemble pour former $(CH_2)_3$, $(CH_2)_4$, $(CH_2)_5$ ou $CH_2CH_2OCH_2CH_2$ ;
- $R_c$ — est un groupe alkyle en $C_1$-$C_4$, alcényle en $C_3$-$C_4$, alcynyle en $C_3$-$C_4$, halogénalkyle en $C_2$-$C_4$, cyanalkyle en $C_2$-$C_3$, cyclopropylméthyle ou alcoxyalkyle en $C_2$-$C_4$ ;
- Q — est $ER_2$, $NR_3R_4$,

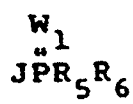 ,

$OSO_2R_7$, un groupe halogénalkyle en $C_1$-$C_4$, CN, $SO_2NHR_{21}$,

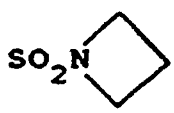 . 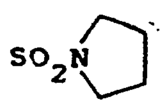 .

$SO_2NR_{22}NR_{23}R_{24}$ ou un groupe alkyle en $C_1$-$C_4$ substitué par $R_8$ ;

- E — est O, S, SO ou $SO_2$ ;
- W et $W_1$ — sont indépendamment O ou S ;
- J — est O, S, NH, $NCH_3$, $CH_2$ ou une liaison simple ;
- $R_2$ — est un groupe alkyle en $C_1$-$C_6$ substitué par $R_8$, alcényle en $C_2$-$C_6$ substitué par $R_8$, alcynyle en $C_3$-$C_6$, alcynyle en $C_3$-$C_6$ substitué par $R_8$, halogénalkyle en $C_1$-$C_6$, halogénalcényle en $C_2$-$C_6$ ou halogénalcynyle en $C_3$-$C_6$ ;
- $R_3$ — est un groupe alkyle en $C_1$-$C_4$ ;
- $R_4$ — est H ou un groupe alkyle en $C_1$-$C_4$ ; ou bien
- $R_3$ et $R_4$ — peuvent être pris ensemble pour former $(CH_2)_4$, $(CH_2)_5$ ou $CH_2CH_2OCH_2CH_2$ ;
- $R_5$ et $R_6$ — sont indépendamment un groupe alkyle en $C_1$-$C_2$, alcoxy en $C_1$-$C_2$, alkylthio en $C_1$-$C_2$, alkylamino en $C_1$-$C_2$ ou di(alkyle en $C_1$-$C_2$)amino ;
- $R_7$ — est un groupe alkyle en $C_1$-$C_4$, halogénalkyle en $C_1$-$C_4$, alcényle en $C_3$-$C_4$, halogénalcényle en $C_3$-$C_4$, alcynyle en $C_3$-$C_4$, halogénalcynyle en $C_3$-$C_4$ ou $NR_{19}R_{20}$ ;
- $R_8$ — est $OR_9$, $S(O)_nR_{10}$, $CO_2R_{10}$, $SO_2NR_{11}R_{12}$, $NR_{11}R_{12}$,$CONR_{11}R_{12}$, $C(O)R_{13}$,

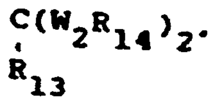, 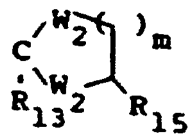,

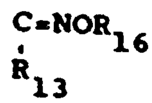, 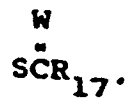,

CN, SCN, SH, $NO_2$ ou $N_3$ ;

$R_9$ est H, un groupe alkyle en $C_1$-$C_4$, alcényle en $C_3$-$C_4$, alcynyle en $C_3$-$C_4$, halogénalkyle en $C_1$-$C_4$, halogénalcényle en $C_3$-$C_4$, halogénalcynyle en $C_3$-$C_4$, alkylcarbonyle en $C_2$-$C_4$, alkylsulfonyle en $C_1$-$C_4$, alcoxyalkyle en $C_2$-$C_4$, alkylthioalkyle en $C_2$-$C_4$ ou cyanalkyle en $C_2$-$C_4$ ;

$R_{10}$ est H, un groupe alkyle en $C_1$-$C_4$, alcényle en $C_3$-$C_4$, alcynyle en $C_3$-$C_4$, halogénalkyle en $C_1$-$C_4$, halogénalcényle en $C_3$-$C_4$, halogénalcynyle en $C_3$-$C_4$, alcoxyalkyle en $C_2$-$C_4$, alkylthioalkyle en $C_2$-$C_4$ ou cyanalkyle en $C_2$-$C_4$ ;

$R_{11}$ est H ou un groupe alkyle en $C_1$-$C_3$ ;

$R_{12}$ est H, un groupe alkyle en $C_1$-$C_4$, halogénalkyle en $C_1$-$C_4$, cycloalkyle en $C_3$-$C_6$, alcoxyalkyle en $C_2$-$C_4$, alkylthioalkyle en $C_2$-$C_4$, cyanalkyle en $C_2$-$C_4$, alcoxy en $C_1$-$C_3$, alcényle en $C_3$-$C_4$ ou alcynyle en $C_3$-$C_4$ ; ou bien

$R_{11}$ et $R_{12}$ peuvent être pris ensemble pour former $(CH_2)_3$, $(CH_2)_4$, $(CH_2)_5$ ou $CH_2CH_2OCH_2CH_2$ ;

$R_{13}$ est H, un groupe alkyle en $C_1$-$C_4$ ou halogénalkyle en $C_1$-$C_4$ ;

$R_{14}$ est un groupe alkyle en $C_1$-$C_2$ ;

$R_{15}$ est H ou $CH_3$ ;

$R_{16}$ est H, un groupe alkyle en $C_1$-$C_4$, alcényle en $C_3$-$C_4$ ou alcynyle en $C_3$-$C_4$ ;

$R_{17}$ est un groupe alkyle en $C_1$-$C_4$, alcoxy en $C_1$-$C_4$, alkylthio en $C_1$-$C_4$, alkylamino en $C_1$-$C_4$ ou di(alkyle en $C_1$-$C_4$)amino ;

$R_{18}$ est F, Cl, Br, CN, $CH_3$, $OCH_3$, $SCH_3$ ou $NO_2$ ;

$R_{19}$ est H, un groupe alkyle en $C_1$-$C_3$, alcényle en $C_3$-$C_4$ ou alcynyle en $C_3$-$C_4$ ;

$R_{20}$ est H ou un groupe alkyle en $C_1$-$C_3$ ; ou bien

$R_{19}$ et $R_{20}$ peuvent être pris ensemble pour former $(CH_2)_4$, $(CH_2)_5$, ou $CH_2CH_2OCH_2CH_2$ ;

$R_{21}$ est un groupe alkyle en $C_1$-$C_4$, halogénalkyle en $C_1$-$C_3$, cyanalkyle en $C_2$-$C_3$, cyclopropyle, alcényle en $C_3$-$C_4$, alcynyle en $C_3$-$C_4$, alcoxyalkyle en $C_2$-$C_4$ ou alcoxy en $C_1$-$C_2$ ;

$R_{22}$ est H ou un groupe alkyle en $C_1$-$C_4$ ;

$R_{23}$ est H ou un groupe alkyle en $C_1$-$C_4$ ;

$R_{24}$ est H, un groupe alkyle en $C_1$-$C_4$, alcényle en $C_3$-$C_4$, alcynyle en $C_3$-$C_4$, halogénalkyle en $C_1$-$C_4$ ou phényle qui peut être facultativement substitué par $R_{25}$ ; ou bien

$R_{23}$ et $R_{24}$ peuvent être pris ensemble pour former $(CH_2)_4$, $(CH_2)_5$ ou $CH_2CH_2OCH_2CH_2$ ;

$R_{25}$ est H, $CH_3$, Cl, F, Br, $NO_2$, $CF_3$, CN ou $OCH_3$ ;

m est 1 ou 2 ;

n est 0, 1 ou 2 ;

$W_2$ et $W_3$ sont indépendamment O ou S ;

A est

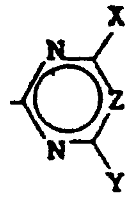 , 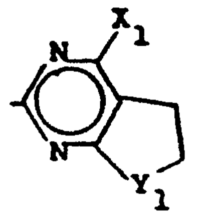 , 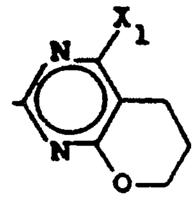 ,

A-1 A-2 A-3

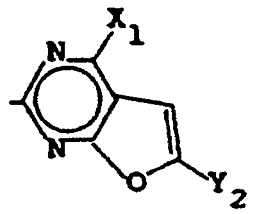 , 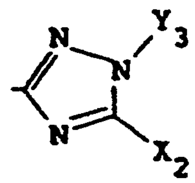 , 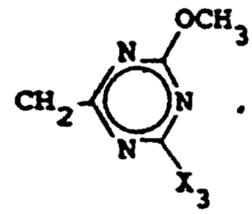 ,

A-4 A-5 A-6

ou 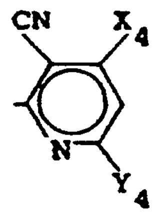 ;

A-7

X est H, un groupe alkyle en $C_1$-$C_4$, alcoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$ ou di-(alkyle en $C_1$-$C_3$)amino ;

Y est H, un groupe alkyle en $C_1$-$C_4$, alcoxy en $C_1$-$C_4$, halogénalcoxy en $C_1$-$C_4$, halogénalkylthio en $C_1$-$C_4$, alkylthio en $C_1$-$C_4$, alcoxyalkyle en $C_2$-$C_5$, alcoxyalcoxy en $C_2$-$C_5$, amino, alkylamino en $C_1$-$C_3$, di(alkyle en $C_1$-$C_3$)amino, alcényloxy en $C_3$-$C_4$, alcynyloxy en $C_3$-$C_4$, alkylthioalkyle en $C_2$-$C_5$, alkylsulfinylalkyle en $C_2$-$C_5$, alkylsulfonylalkyle en $C_2$-$C_5$, halogénalkyle en $C_1$-$C_4$, cycloalkyle en $C_4$-$C_5$, alcynyle en $C_2$-$C_4$, cyano,

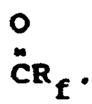 , 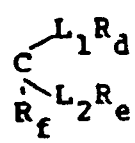 , 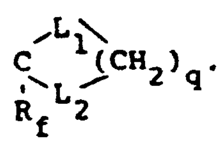 ,

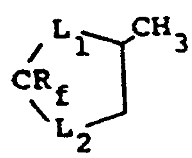

ou $N(OCH_3)CH_3$ ;

| | |
|---|---|
| q | est 2 ou 3 ; |
| $L_1$ et $L_2$ | sont indépendamment O ou S ; |
| $R_d$ et $R_e$ | sont indépendamment un groupe alkyle en $C_1$-$C_2$ ; |
| $R_f$ | est H ou $CH_3$ ; |
| Z | est CH ou N ; |
| $Y_1$ | est O ou $CH_2$ ; |
| $X_1$ | est $CH_3$, $OCH_3$, $OC_2H_5$ ou $OCF_2H$ ; |
| $Y_2$ | est H ou $CH_3$ ; |
| $X_2$ | est $CH_3$, $OCH_3$ ou $SCH_3$ ; |
| $Y_3$ | est $CH_3$, $CH_2CH_3$ ou $CH_2CF_3$ ; |
| $X_3$ | est $CH_3$ ou $OCH_3$ ; |
| $X_4$ | est $CH_3$, $OCH_3$, $OC_2H_5$, $CH_2OCH_3$ ou Cl ; et |
| $Y_4$ | est $CH_3$, $OCH_3$, $OC_2H_5$ ou Cl ; |

et ses sels utilisables en agriculture,
avec les conditions suivantes

a) si X est Cl, F, Br ou I, alors Z est CH et Y est $OCH_3$, $OC_2H_5$, $N(OCH_3)CH_3$, $NHCH_3$, $N(CH_3)_2$ ou $OCF_2H$ ;
b) si X ou Y est un groupe halogénalcoxy en $C_1$, alors Z est CH ;
c) si Q est $CF_3$, alors A est A-4, A-5, A-6 ou A-7 ;
d) si E est O ou S et $R_9$ est H, alors $R_2$ est autre chose que $CH_2OR_9$ ;
e) si W est S, alors A est A-1, R est H et Y est $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$ ou

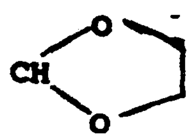 ;

f) le nombre total de carbone dans $R_1$ et Q est inférieur ou égal à 10 ;
g) si Q est un groupe halogénalkyle en $C_1$-$C_4$ ou

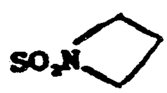 ,

alors X et Y sont autre chose que $OCF_2H$ ou $SCF_2H$ ;
h) $X_4$ et $Y_4$ ne peuvent pas être simultanément Cl ;
i) le substituant Q et le pont de sulfonylurée se trouvent sur des atomes de carbone adjacents ;
j) le nombre total d'atomes de carbone dans $R_{22}$, $R_{23}$ et $R_{24}$ est inférieur ou égal à 10 ;
k) si $R_{21}$ est un groupe alkyle en $C_1$-$C_4$, cyanalkyle en $C_2$-$C_3$, alcoxy en $C_1$-$C_2$ ou alcényle en $C_3$-$C_4$, alors X et Y sont autre chose que $OCF_2H$ ou $SCF_2H$ ;
l) si Y est CN et $R_1$ est H, F, Cl ou $CH_3$, alors $R_{21}$ est autre chose qu'un groupe alkyle en $C_1$-$C_3$ ;
m) si $R_{21}$ est un groupe alkyle en $C_1$-$C_4$, alors Y est autre chose qu'un groupe alkylamino en $C_1$-$C_3$, di(alkyle en $C_1$-$C_3$)amino, $N(OCH_3)CH_3$ ou alcynyle en $C_2$-$C_4$ et X est autre chose qu'un halogène, un groupe alkylamino en $C_1$-$C_3$ ou di(alkyle en $C_1$-$C_3$)amino.

**2.** Composés de la revendication 1, dans lesquels

| | |
|---|---|
| R | est H ; |
| W | est O ; |
| $R_1$ | est H, $CH_3$ ou Cl ; |
| X | est $CH_3$, $OCH_3$, $OCH_2CH_3$, Cl, F, Br, I, $OCF_2H$, $CH_2F$, $CF_3$, $OCH_2CH_2F$, $OCH_2CHF_2$, $OCH_2CF_3$, $CH_2Cl$ ou $CH_2Br$ ; et |
| Y | est H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C\equiv CH$, $CH_2OCH_2CH_3$, $OCH_2CH_2OCH_3$, $CH_2SCH_3$, $CH_2SCH_2CH_3$, |

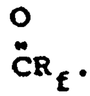, 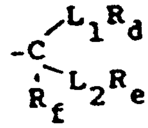, 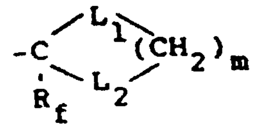,

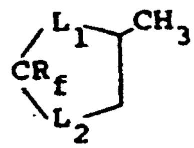.

$OCF_2H$, $SCF_2H$, $C{\equiv}CH$ ou $C{\equiv}CCH_3$.

3. Composés de la revendication 2, dans lesquels A est A-1.

4. Composés de la revendication 3, dans lesquels le pont de sulfonylurée est fixé à la position 2 du noyau de thiophène.

5. Composés de la revendication 4, dans lesquels

Q est $ER_2$,

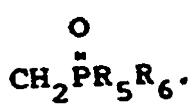, 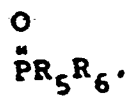,

un groupe alkyle en $C_1$-$C_2$ substitué par $R_8$, halogénalkyle en $C_1$-$C_2$, CN, $SO_2NH$-(alkyle en $C_1$-$C_2$), $SO_2$ NH-cyclopropyle ou

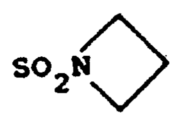 ;

$R_2$ est un groupe alkyle en $C_1$-$C_3$ substitué par $R_8$, halogénalkyle en $C_1$-$C_3$, halogénalcényle en $C_2$-$C_3$ ou alcynyle en $C_3$-$C_4$ ;

$R_5$ et $R_6$ sont indépendamment $CH_3$, $OCH_3$ ou $SCH_3$ ;

$R_8$ est $OR_9$, un groupe alkylthio en $C_1$-$C_2$, alkylsulfinyle en $C_1$-$C_2$, alkylsulfonyle en $C_1$-$C_2$, $CO_2$(alkyle en $C_1$-$C_2$), $SO_2N$(alkyle en $C_1$-$C_2$)$_2$, $C(O)CH_3$, CN ou $C(O)N(CH_3)_2$ ;

$R_9$ est H, un groupe alkyle en $C_1$-$C_2$ ou cyanalkyle en $C_2$-$C_3$.

6. Composés de la revendication 5, dans lesquels

X est $CH_3$, $OCH_3$, $OCH_2CH_3$, Cl, $OCF_2H$ ou $OCH_2CF_3$ ;

Y est $CH_3$, $OCH_3$, $C_2H_5$, $CH_2OCH_3$, $NHCH_3$ ou $CH(OCH_3)_2$ ;

O est $ER_2$, un groupe alkyle en $C_1$-$C_2$ substitué par $R_8$ ou halogénalkyle en $C_1$-$C_2$, CN ou $SO_2NH$(alkyle en $C_1$-$C_2$) ;

E est S ou $SO_2$ ;

$R_2$ est un groupe alkyle en $C_1$-$C_2$ substitué par $R_8$ ou halogénalkyle en $C_1$-$C_2$ ;

$R_8$ est $CH_3O$, $CH_3CH_2O$, $CH_3S$, $CH_3SO_2$, $SO_2N(CH_3)_2$ ou $CO_2CH_3$.

7. Composés de la revendication 3, dans lesquels le pont de sulfonylurée est fixé à la position 3 du noyau de thiophène.

**8.** Composés de la revendication 7, dans lesquels Qest $ER_2$,

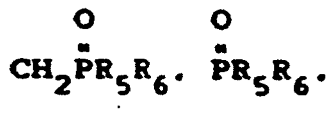
,

un groupe alkyle en $C_1$-$C_2$ substitué par $R_8$, halogénalkyle en $C_1$-$C_2$, CN, $SO_2NH$(alkyle en $C_1$-$C_2$), $SO_2NH$-cyclopropyle ou ;

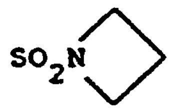
:

| | |
|---|---|
| $R_2$ | est un groupe alkyle en $C_1$-$C_3$ substitué par $R_8$, halogénalkyle en $C_1$-$C_3$, halogénalcényle en $C_2$-$C_3$ ou alcynyle en $C_3$-$C_4$ ; |
| $R_5$ et $R_6$ | sont indépendamment $CH_3$, $OCH_3$ ou $SCH_3$ ; |
| $R_8$ | est $OR_9$, un groupe alkylthio en $C_1$-$C_2$, alkylsulfinyle en $C_1$-$C_2$, alkylsulfonyle en $C_1$-$C_2$, $CO_2$(alkyle en $C_1$-$C_2$), $SO_2N$(alkyle en $C_1$-$C_2$)$_2$, $C(O)CH_3$, CN ou $C(O)N(CH_3)_2$ ; |
| $R_9$ | est H, un groupe alkyle en $C_1$-$C_2$ ou cyanalkyle en $C_2$-$C_3$. |

**9.** Composés de la revendication 8, dans lesquels

| | |
|---|---|
| X | est $CH_3$, $OCH_3$, $OCH_2CH_3$, Cl, $OCF_2H$ ou $OCH_2CF_3$ ; |
| Y | est $CH_3$, $OCH_3$, $C_2H_5$, $CH_2OCH_3$, $NHCH_3$ ou $CH(OCH_3)_2$ ; |
| Q | est $ER_2$, un groupe alkyle en $C_1$-$C_2$ substitué par $R_8$ ou halogénalkyle en $C_1$-$C_2$, CN ou $SO_2NH$(alkyle en $C_1$-$C_2$) ; |
| E | est S ou $SO_2$ ; |
| $R_2$ | est un groupe alkyle en $C_1$-$C_2$ substitué par $R_8$ ou halogénalkyle en $C_1$-$C_2$ ; |
| $R_8$ | est $CH_3O$, $CH_3CH_2O$, $CH_3S$, $CH_3SO_2$, $SO_2N(CH_3)_2$ ou $CO_2CH_3$. |

**10.** Le composé de la revendication 1 qui est le N-[(4,6-diméthoxypyrimidine-2-yl)aminocarbonyl]-2-(méthoxyméthyl)-3-thiophène-sulfonamide.

**11.** Le composé de la revendication 1 qui est le N-[(4-méthoxy-6-méthyl-1,3,5-triazine-2-yl)aminocarbonyl]-2-(méthoxyméthyl)-3-thiophène-sulfonamide.

**12.** Le composé de la revendication 1 qui est le N-[(4-méthoxy-6-méthylpyrimidine-2-yl)aminocarbonyl]-4-cyano-3-thiophène-sulfonamide.

**13.** Le composé de la revendication 1 qui est le N-[(4,6-diméthoxypyrimidine-2-yl)aminocarbonyl]-3-(2-méthoxyéthyl)-2-thiophène-sulfonamide.

**14.** Une composition convenant pour lutter contre la croissance de végétation indésirable, qui comprend une quantité efficace d'un composé de l'une quelconque des revendications 1 à 13 et au moins l'un des ingrédients suivants : agent tensio-actif, diluant solide ou liquide.

**15.** Un procédé pour lutter contre la croissance de végétation indésirable, qui consiste à appliquer au lieu à protéger une quantité efficace d'un composé de l'une quelconque des revendications 1 à 13.

**16.** Un procédé pour réguler la croissance de plantes, qui consiste à appliquer à l'emplacement de ces plantes une quantité efficace, mais sensiblement non phytotoxique, d'un régulateur de croissance de plantes choisi parmi les composés de l'une quelconque des revendications 1 à 13.

**17.** Un procédé pour la préparation d'un composé de la revendication 1, qui consiste à :

(a) faire réagir un sulfonamide de formule

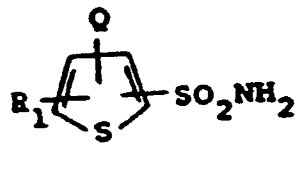

(2)

avec un carbamate de méthyle approprié de formule

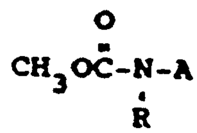

(3)

(b) faire réagir un sulfonylcarbamate de formule

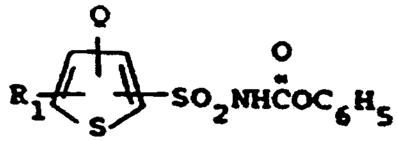

(4)

avec une amine hétérocyclique appropriée de formule

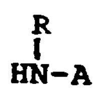

(5)

(c) dans un composé de formule générale

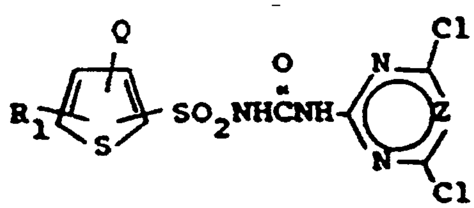

(7)

remplacer un atome de chlore par $OCH_3$ et/ou remplacer un atome de chlore par OY', où Y' est $CH_3$, $C_2H_5$ ou $CH_2CF_3$, par des procédés connus en soi ;

(d) faire réagir un isocyanate de thiénylsulfonyle de formule

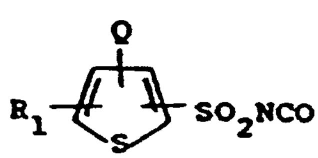

(8)

avec ladite amine hétérocyclique (5) ; à condition que $R_a$, $R_b$, $R_4$, $R_9$, $R_{11}$, $R_{12}$, $R_{19}$ et $R_{20}$ ne soient pas H, J ne soit pas NH et $R_5$, $R_6$ et $R_7$ ne soient pas un groupe alkylamino ; ou

(e) faire réagir ledit sulfonamide (2) avec un carbamate hétérocyclique de formule

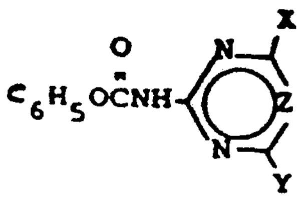

(10)

## Patentansprüche

### Patentansprüche für folgende Vertragsstaaten : BE, CH, DE, FR, GB, IT, LI, LU, NL, SE

1. Verbindung der Formel

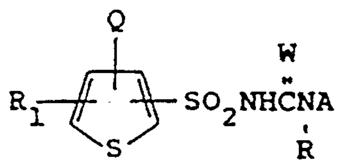

I

,

worin

R H oder $CH_3$ ist;

$R_1$ H, $C_1$-$C_3$-Alkyl, $C_1$-$C_3$-Halogenalkyl, Halogen, Nitro, $C_1$-$C_3$-Alkoxy, $SO_2NR_aR_b$, $C_1$-$C_3$-Alkylthio, $C_1$-$C_3$-Alkylsulfinyl, $C_1$-$C_3$-Alkylsulfonyl, CN, $CO_2R_c$,$C_1$-$C_3$-Halogenalkoxy oder $C_1$-$C_3$-Halogenalkylthio ist;

$R_a$ H, $C_1$-$C_4$-Alkyl, $C_2$-$C_3$-Cyanalkyl, Methoxy, Ethoxy oder $C_3$-$C_4$-Alkenyl ist;

$R_b$ H oder $C_1$-$C_3$-Alkyl ist; oder

$R_a$ und $R_b$ zur Bildung von $(CH_2)_3$, $(CH_2)_4$, $(CH_2)_5$ oder $CH_2CH_2OCH_2CH_2$ zusammengenommen werden können;

$R_c$ $C_1$-$C_4$-Alkyl, $C_3$-$C_4$-Alkenyl, $C_3$-$C_4$-Alkinyl $C_2$-$C_4$-Halogenalkyl, $C_2$-$C_3$-Cyanalkyl, Cyclopropylmethyl oder $C_2$-$C_4$-Alkoxyalkyl ist;

Q $ER_2$, $NR_3R_4$,

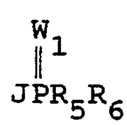

$OSO_2R_7$, $C_1$-$C_4$-Halogenalkyl, CN, $SO_2NHR_{21}$,

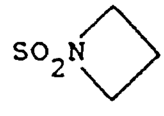, 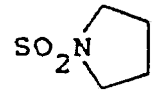,

$SO_2NR_{22}NR_{23}R_{24}$ oder mit $R_8$ substiutiertes $C_1$-$C_4$-Alkyl ist;

E O, S, SO oder $SO_2$ ist;

W und $W_1$ unabhängig voneinander O oder S sind;

J O, S, NH, $NCH_3$, $CH_2$ oder eine Einfachbindung ist;

$R_2$ mit $R_8$ substituiertes $C_1$-$C_6$-Alkyl, mit $R_8$ substituiertes $C_2$-$C_6$-Alkenyl, $C_3$-$C_6$-Alkinyl, mit $R_8$ substituiertes $C_3$-$C_6$-Alkinyl, $C_1$-$C_6$-Halogenalkyl, $C_2$-$C_6$-Halogenalkenyl oder $C_3$-$C_6$-Halogenalkinyl ist;

$R_3$ $C_1$-$C_4$-Alkyl ist;

| | |
|---|---|
| $R_4$ | H oder $C_1$-$C_4$-Alkyl ist; oder |
| $R_3$ und $R_4$ | zur Bildung von $(CH_2)_4$, $(CH_2)_5$ oder $CH_2CH_2OCH_2CH_2$ zusammengenommen werden können; |
| $R_5$ und $R_6$ | unabhängig voneinander $C_1$-$C_2$-Alkyl, $C_1$-$C_2$-Alkoxy, $C_1$-$C_2$-Alkylthio, $C_1$-$C_2$-Alkylamino oder Di($C_1$-$C_2$-alkyl)amino sind; |
| $R_7$ | $C_1$-$C_4$-Alkyl, $C_1$-$C_4$-Halogenalkyl, $C_3$-$C_4$-Alkenyl, $C_3$-$C_4$-Halogenalkenyl, $C_3$-$C_4$-Alkinyl $C_3$-$C_4$-Halogenalkinyl oder $NR_{19}R_{20}$ ist; |
| $R_8$ | $OR_9$, $S(O)_nR_{10}$, $CO_2R_{10}$, $SO_2NR_{11}R_{12}$, $NR_{11}R_{12}$,$CONR_{11}R_{12}$, $C(O)R_{13}$, 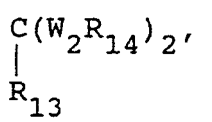, 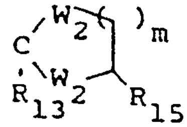, 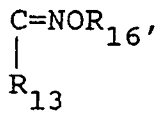, 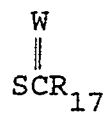, CN, SCN, SH, $NO_2$ oder $N_3$ ist: |
| $R_9$ | H, $C_1$-$C_4$-Alkyl, $C_3$-$C_4$-Alkenyl, $C_3$-$C_4$-Alkinyl, $C_1$-$C_4$-Halogenalkyl, $C_3$-$C_4$-Halogenalkenyl, $C_3$-$C_4$-Halogenalkinyl, $C_2$-$C_4$-Alkylcarbonyl, $C_1$-$C_4$-Alkylsulfonyl, $C_2$-$C_4$-Alkoxyalkyl, $C_2$-$C_4$-Alkylthioalkyl oder $C_2$-$C_4$-Cyanalkyl ist; |
| $R_{10}$ | H, $C_1$-$C_4$-Alkyl, $C_3$-$C_4$-Alkenyl, $C_3$-$C_4$-Alkinyl, $C_1$-$C_4$-Halogenalkyl, $C_3$-$C_4$-Halogenalkenyl, $C_3$-$C_4$-Halogenalkinyl, $C_2$-$C_4$-Alkoxyalkyl, $C_2$-$C_4$-Alkylthioalkyl oder $C_2$-$C_4$-Cyanalkyl ist; |
| $R_{11}$ | H oder $C_1$-$C_3$-Alkyl ist; |
| $R_{12}$ | H, $C_1$-$C_4$-Alkyl, $C_1$-$C_4$-Halogenalkyl, $C_3$-$C_6$-Cycloalkyl, $C_2$-$C_4$-Alkoxyalkyl, $C_2$-$C_4$-Alkylthioalkyl, $C_2$-$C_4$-Cyanalkyl, $C_1$-$C_3$-Alkoy, $C_3$-$C_4$-Alkenyl oder $C_3$-$C_4$-Alkinyl ist; oder |
| $R_{11}$ und $R_{12}$ | zur Bildung von $(CH_2)_3$, $(CH_2)_4$, $(CH_2)_5$ oder $CH_2CH_2OCH_2CH_2$ zusammengenommen werden können; |
| $R_{13}$ | H, $C_1$-$C_4$-Alkyl oder $C_1$-$C_4$-Halogenalkyl ist; |
| $R_{14}$ | $C_1$-$C_2$-Alkyl ist; |
| $R_{15}$ | H oder $CH_3$ ist; |
| $R_{16}$ | H, $C_1$-$C_4$-Alkyl, $C_3$-$C_4$-Alkenyl oder $C_3$-$C_4$-Alkinyl ist; |
| $R_{17}$ | $C_1$-$C_4$-Alkyl, $C_1$-$C_4$-Alkoxy, $C_1$-$C_4$-Alkylthio, $C_1$-$C_4$-Alkylamino oder Di($C_1$-$C_4$-alkyl)-amino ist; |
| $R_{18}$ | F, Cl, Br, CN, $CH_3$, $OCH_3$, $SCH_3$ oder $NO_2$ ist; |
| $R_{19}$ | H, $C_1$-$C_3$-Alkyl, $C_3$-$C_4$-Alkenyl oder $C_3$-$C_4$-Alkinyl ist; oder |
| $R_{20}$ | H oder $C_1$-$C_3$-Alkyl ist; oder |
| $R_{19}$ und $R_{20}$ | zur Bildung von $(CH_2)_4$, $(CH_2)_5$ oder $CH_2CH_2OCH_2CH_2$ zusammengenommen werden können; |
| $R_{21}$ | $C_1$-$C_4$-Alkyl, $C_1$-$C_3$-Halogenalkyl, $C_2$-$C_3$-Cyanalkyl, Cyclopropyl, $C_3$-$C_4$-Alkenyl, $C_3$-$C_4$-Alkinyl, $C_2$-$C_4$-Alkoxyalkyl oder $C_1$-$C_2$-Alkoxy ist; |
| $R_{22}$ | H oder $C_1$-$C_4$-Alkyl ist; |
| $R_{23}$ | H oder $C_1$-$C_4$-Alkyl ist; |
| $R_{24}$ | H, $C_1$-$C_4$-Alkyl, $C_3$-$C_4$-Alkenyl, $C_3$-$C_4$-Alkinyl, $C_1$-$C_4$-Halogenoalkyl oder gegebenenfalls mit $R_{25}$ substituiertes Phenyl ist; oder |
| $R_{23}$ und $R_{24}$ | zur Bildung von $(CH_2)_4$, $(CH_2)_5$ oder $CH_2CH_2OCH_2CH_2$ zusammengenommen werden können; |
| $R_{25}$ | H, $CH_3$, Cl, F, Br, $NO_2$, $CF_3$, CN oder $OCH_3$ ist; |
| m | 1 oder 2 ist; |
| n | 0, 1 oder 2 ist; |

W2 und W3 unabhängig O oder S sind;

A

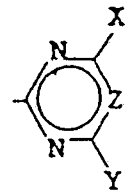,
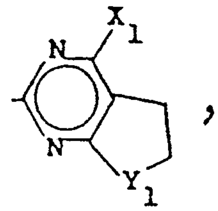,
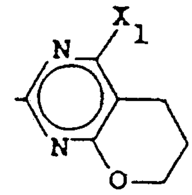,

A-1 A-2 A-3

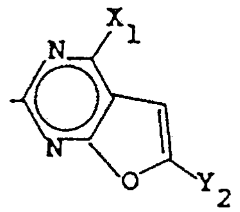,
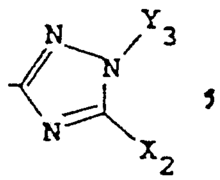,
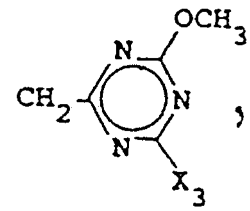,

A-4 A-5 A-6

oder 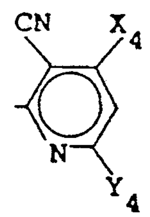 ist;

A-7

X H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino oder Di($C_1$-$C_3$-alkyl)amino ist;

Y H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_2$-$C_5$-Alkylsulfinylalkyl, $C_2$-$C_5$-Alkylsulfonylalkyl, $C_1$-$C_4$-Halogenalkyl, $C_4$-$C_5$-Cycloalkyl, $C_2$-$C_4$-Alkinyl, Cyan,

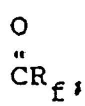,
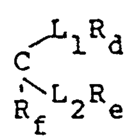,
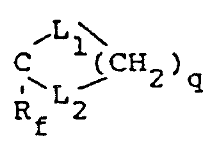,
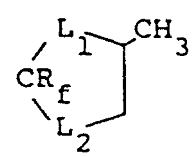

oder $N(OCH_3)CH_3$ ist;

q 2 oder 3 ist;

$L_1$ und $L_2$ unabhängig voneinander O oder S sind;

$R_d$ und $R_e$ unabhängig voneinander $C_1$-$C_2$-Alkyl sind;

$R_f$ H oder $CH_3$ ist;

Z CH oder N ist;

$Y_1$ O oder $CH_2$ ist;

$X_1$ $CH_3$, $OCH_3$, $OC_2H_5$ oder $OCF_2H$ ist;

| | |
|---|---|
| $Y_2$ | H oder $CH_3$ ist; |
| $X_2$ | $CH_3$, $OCH_3$ oder $SCH_3$ ist; |
| $Y_3$ | $CH_3$, $CH_2CH_3$ oder $CH_2CF_3$ ist; |
| $X_3$ | $CH_3$ oder $OCH_3$ ist; |
| $X_4$ | $CH_3$, $OCH_3$, $OC_2H_5$, $CH_2OCH_3$ oder Cl ist; und |
| $Y_4$ | $CH_3$, $OCH_3$, $OC_2H_5$ oder Cl ist; |

und deren landwirtschaftlich geeignete Salze; mit den Maßgaben, daß

a) wenn X Cl, F, Br oder I ist, dann Z CH ist und Y $OCH_3$, $OC_2H_5$, $N(OCH_3)CH_3$, $NHCH_3$, $N(CH_3)_2$ oder $COF_2H$ ist;

b) wenn X oder Y $C_1$-Halogenalkoxy ist, dann Z CH ist;

c) wenn Q $CF_3$ ist, dann A A-4, A-5, A-6 oder A-7 ist;

d) wenn E O oder S ist und $R_9$ H ist, dann $R_2$ von $CH_2OR_9$ verschieden ist;

e) wenn W S ist, dann A A-1 ist, R H ist und Y $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$oder

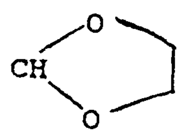

ist;

f) die Gesamtzahl der Kohlenstoff-Atome in $R_1$ und Q kleiner als oder gleich 10 ist;

g) wenn Q $C_1$-$C_4$-Halogenalkyl oder

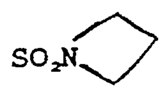

ist, dann X und Y von $OCF_2H$ oder $SCF_2H$ verschieden sind;

h) $X_4$ und $Y_4$ nicht gleichzeitig Chlor sein können;

i) der Substituent Q und die Sulfonylharnstoff-Brücke sich an benachbarten Kohlenstoff-Atomen befinden;

j) die Gesamtzahl der Kohlenstoff-Atome in $R_{22}$, $R_{23}$ und $R_{24}$ kleiner als oder gleich 10 ist;

k) wenn $R_{21}$ $C_1$-$C_4$-Alkyl, $C_2$-$C_3$-Cyanalkyl, $C_1$-$C_2$-Alkoxy oder $C_3$-$C_4$-Alkenyl ist, dann X und Y von $OCF_2H$ oder $SCF_2H$ verschieden sind;

l) wenn Y CN ist und $R_1$ H, F, Cl oder $CH_3$ ist, dann $R_{21}$ von $C_1$-$C_3$-Alkyl verschieden ist;

m) wenn $R_{21}$ $C_1$-$C_4$-Alkyl ist, dann Y von $C_1$-$C_3$-Alkylamino, Di($C_1$-$C_3$-alkyl)amino, $N(OCH_3)CH_3$ oder $C_2$-$C_4$-Alkinyl verschieden ist und X von Halogen, $C_1$-$C_3$-Alkylamino oder Di($C_1$-$C_3$-alkyl)amino verschieden ist.

**2.** Verbindungen nach Anspruch 1, worin

| | |
|---|---|
| R | H ist; |
| W | O ist; |
| $R_1$ | H, $CH_3$ oder Cl ist; |
| X | $CH_3$, $OCH_3$, $OCH_2CH_3$, Cl, F, Br, I, $OCF_2H$, $CH_2F$, $CF_3$, $OCH_2CH_2F$, $OCH_2CHF_2$, $OCH_2CF_3$, $CH_2Cl$ oder $CH_2Br$ ist; und |
| Y | H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C{\equiv}CH$, $CH_2OCH_2CH_3$, $OCH_2CH_2OCH_3$, $CH_2SCH_3$, $CH_2SCH_2CH_3$, |

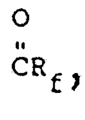, 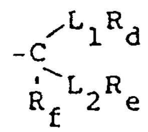, 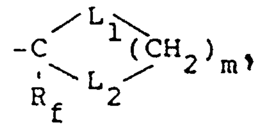, 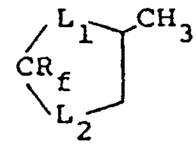,

$OCF_2H$, $SCF_2H$, C≡CH oder $C{\equiv}CCH_3$ ist.

3. Verbindungen nach Anspruch 2, worin A A-1 ist.

4. Verbindungen nach Anspruch 3, worin die Sulfonylharnstoff-Brücke an die 2-Position des Thiophen-Rings gebunden ist.

5. Verbindungen nach Anspruch 4, worin
   Q   $ER_2$,

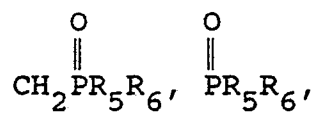

mit $R_8$ substituiertes $C_1$-$C_2$-Alkyl, $C_1$-$C_2$-Halogenalkyl, CN, $SO_2NH$-$C_1$-$C_2$-Alkyl, $SO_2NH$-Cyclopropyl oder

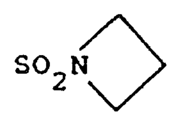

ist;
   $R_2$   mit $R_8$ substituiertes $C_1$-$C_3$-Alkyl, $C_1$-$C_3$-Halogenalkyl, $C_2$-$C_3$-Halogenalkenyl oder $C_3$-$C_4$-Alkinyl ist;
   $R_5$   und $R_6$ unabhängig voneinander $CH_3$, $OCH_3$ oder $SCH_3$ sind;
   $R_8$   $OR_9$, $C_1$-$C_2$-Alkylthio, $C_1$-$C_2$-Alkylsulfinyl, $C_1$-$C_2$-Alkylsulfonyl, $CO_2(C_1$-$C_2$-Alkyl), $SO_2N(C_1$-$C_2$-Alkyl$)_2$, $C(O)CH_3$, CN oder $C(O)N(CH_3)_2$ ist;
   $R_9$   H, $C_1$-$C_2$-Alkyl oder $C_2$-$C_3$-Cyanalkyl ist.

6. Verbindungen nach Anspruch 5, worin
   X   $CH_3$, $OCH_3$, $OCH_2CH_3$, Cl, $OCF_2H$ oder $OCH_2CF_3$ ist;
   Y   $CH_3$, $OCH_3$, $C_2H_5$, $CH_2OCH_3$, $NHCH_3$ oder $CH(OCH_3)_2$ ist;
   Q   $ER_2$, mit $R_8$ substituiertes $C_1$-$C_2$-Alkyl, oder $C_1$-$C_2$-Halogenalkyl, CN oder $SO_2NH$-$C_1$-$C_2$-Alkyl ist;
   E   S oder $SO_2$ ist;
   $R_2$   mit $R_8$ substituiertes $C_1$-$C_2$-Alkyl oder $C_1$-$C_2$-Halogenalkyl ist;
   $R_8$   $CH_3O$, $CH_3CH_2O$, $CH_3S$, $CH_3SO_2$, $SO_2N(CH_3)_2$ oder $CO_2CH_3$ ist.

7. Verbindungen nach Anspruch 3, worin die Sulfonylharnstoff-Brücke an die 3-Position des Thiophen-Rings gebunden ist.

8. Verbindungen nach Anspruch 7, worin
   Q   $ER_2$,

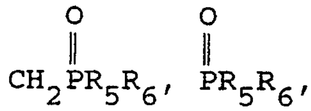

mit $R_8$ substituiertes $C_1$-$C_2$-Alkyl, $C_1$-$C_2$-Halogenalkyl, CN, $SO_2NH$-$C_1$-$C_2$-Alkyl, $SO_2$ NH-Cyclopropyl oder

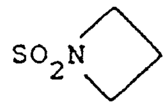

ist;

| | |
|---|---|
| $R_2$ | mit $R_8$ substituiertes $C_1$-$C_3$-Alkyl, $C_1$-$C_3$-Halogenalkyl, $C_2$-$C_3$-Halogenalkenyl oder $C_3$-$C_4$-Alkinyl ist; |
| $R_5$ und $R_6$ | unabhängig voneinander $CH_3$, $OCH_3$ oder $SCH_3$ sind; |
| $R_8$ | $OR_9$, $C_1$-$C_2$-Alkylthio, $C_1$-$C_2$-Alkylsulfinyl, $C_1$-$C_2$-Alkylsulfonyl, $CO_2(C_1$-$C_2$-Alkyl), $SO_2N(C_1$-$C_2$-Alkyl$)_2$, $C(O)CH_3$, CN oder $C(O)N(CH_3)_2$ ist; |
| $R_9$ | H, $C_1$-$C_2$-Alkyl oder $C_2$-$C_3$-Cyanalkyl ist. |

**9.** Verbindungen nach Anspruch 8, worin

| | |
|---|---|
| X | $CH_3$, $OCH_3$, $OCH_2CH_3$, Cl, $OCF_2H$ oder $OCH_2CF_3$ ist; |
| Y | $CH_3$, $OCH_3$, $C_2H_5$, $CH_2OCH_3$, $NHCH_3$ oder $CH(OCH_3)_2$ ist; |
| Q | $ER_2$, mit $R_8$ substituiertes $C_1$-$C_2$-Alkyl, oder $C_1$-$C_2$-Halogenalkyl, CN oder $SO_2NH$-$C_1$-$C_2$-Alkyl ist; |
| E | S oder $SO_2$ ist; |
| $R_2$ | mit $R_8$ substituiertes $C_1$-$C_2$-Alkyl oder $C_1$-$C_2$-Halogenalkyl ist; |
| $R_8$ | $CH_3O$, $CH_3CH_2O$, $CH_3S$, $CH_3SO_2$, $SO_2N(CH_3)_2$ oder $CO_2CH_3$ ist. |

**10.** Verbindung nach Anspruch 1, die N-[(4,6-Dimethoxypyrimidin-2-yl)aminocarbonyl]-2-(methoxy)methyl)-3-thiophensulfonamid ist.

**11.** Verbindung nach Anspruch 1, die N-[(4-Methoxy-6-methyl-1,3,5-triazin-2-yl)aminocarbonyl]-2-(methoxy)-methyl)-3-thiophensulfonamid ist.

**12.** Verbindung nach Anspruch 1, die N-[(4-Methoxy-6-methylpyrimidin-2-yl)aminocarbonyl]-4-cyano-3-thiophensulfonamid ist.

**13.** Verbindung nach Anspruch 1, die N-[(4,6-Dimethoxypyrimidin-2-yl)aminocarbonyl]-3-(2-methoxy)-methyl)-2-thiophensulfonamid ist.

**14.** Zur Bekämpfung des Wachstums unerwünschter Vegetation geeignete Zusammensetzung, umfassend eine wirksame Menge einer Verbindung nach irgendeinem der Ansprüche 1 bis 13 und wenigstens einen der folgenden Stoffe: Tensid, festes oder flüssiges Verdünnungsmittel.

**15.** Verfahren zur Bekämpfung des Wachstums unerwünschter Vegetation, umfassend das Aufbringen einer wirksamen Menge einer Verbindung nach irgendeinem der Ansprüche 1 bis 13 auf den zu schützenden Ort.

**16.** Verfahren zur Regulierung des Wachstums von Pflanzen, umfassend das Aufbringen einer wirksamen, jedoch im wesentlichen nicht-phytotoxischen Menge eines aus Verbindungen nach irgendeinem der Ansprüche 1 bis 13 ausgewählten Regulierungsmittels für das Pflanzenwachstum auf den Ort solcher Pflanzen.

**17.** Verfahren zur Herstellung einer Verbindung nach Anspruch 1, umfassend

(a) die Umsetzung eines Sulfonamids der Formel

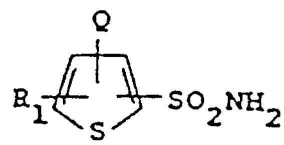

(2)

mit einem geeigneten Methylcarbamat der Formel

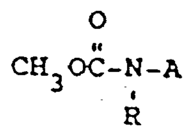

(3)

,

(b) die Umsetzung eines Sulfonylcarbamats der Formel

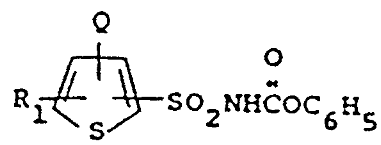

(4)

mit einem geeigneten heterocyclischen Amin der Formel

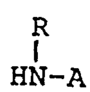

(5)

,

(c) in einer Verbindung der allgemeinen Formel

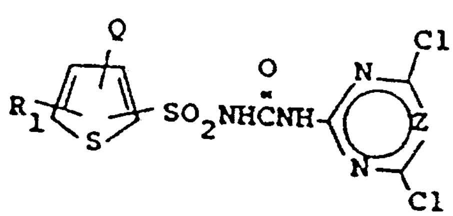

(7)

das Ersetzen eines Chlors durch $OCH_3$ und/oder das Ersetzen eines Chlors durch OY', worin Y' $CH_3$, $C_2H_5$ oder $CH_2CF_3$ ist, mittels an sich bekannter Methoden;

(d) die Umsetzung eines Thienylsulfonylisocyanats der Formel

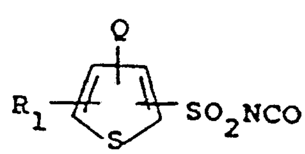

(8)

mit dem bezeichneten heterocyclischen Amin (5), mit der Maßgabe, daß $R_a$, $R_b$, $R_4$, $R_9$, $R_{11}$, $R_{12}$, $R_{19}$ und $R_{20}$ nicht H sind, J nicht NH ist und $R_5$, $R_6$ und $R_7$ nicht Alkylamino sind; oder
(e) die Umsetzung des bezeichneten Sulfonamids (2) mit einem heterocyclischen Carbamat der Formel

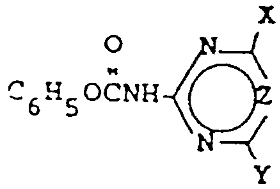

(10)

.